# EUROPEAN PATENT SPECIFICATION

(11) **EP 2 257 647 B1**
(45) Date of publication and mention of the grant of the patent: **14.09.2016**
(21) Application number: 09713926.5
(22) Date of filing: 27.02.2009
(51) Int. Cl.: C12Q 1/68

(54) **MICRO RNA-BASED METHODS AND COMPOSITIONS FOR THE DIAGNOSIS OF GASTRIC CANCER**
AUF MICRO-RNA BERUHENDE VERFAHREN UND ZUSAMMENSETZUNGEN ZUR DIAGNOSE VON MAGENKREBS
PROCÉDÉS ET COMPOSITIONS FONDÉS SUR MICRO ARN POUR LE DIAGNOSTIC DU CANCER DE L'ESTOMAC

(30) Priority: 28.02.2008 US 67445 P
(43) Date of publication of application: 08.12.2010
(60) Divisional application: 16165519.6
(73) Proprietor: The Ohio State University Research Foundation, Columbus, OH 43201 (US)
(72) Inventor: CROCE, Carlo, M., Columbus, OH 43221 (US); PETROCCA, Fabio, Boston, MA 02128 (US); VECCHIONE, Andrea, Rome 00162 (IT)
(74) Representative: Turner, Craig Robert
(86) International application number: PCT/US2009/035458
(87) International publication number: WO 2009/108853

(56) References cited:
- WO-A2-2007/081740
- US-A1- 2005 069 918
- VOLINIA STEFANO ET AL: "A microRNA expression signature of human solid tumors defines cancer gene targets", PROCEEDINGS OF THE NATIONAL ACADEMY OF SCIENCES, NATIONAL ACADEMY OF SCIENCES, vol. 103, no. 7, 14 February 2006 (2006-02-14), pages 2257-2261, XP002608380, ISSN: 0027-8424, DOI: DOI:10.1073/PNAS.0510565103 [retrieved on 2006-02-03]
- I. IVANOVSKA ET AL: "MicroRNAs in the miR-106b Family Regulate p21/CDKN1A and Promote Cell Cycle Progression", MOLECULAR AND CELLULAR BIOLOGY, vol. 28, no. 7, 22 January 2008 (2008-01-22), pages 2167-2174, XP55000128, ISSN: 0270-7306, DOI: 10.1128/MCB.01977-07
- THOMSON J MICHAEL ET AL: "A custom microarray platform for analysis of microRNA gene expression", NATURE METHODS, NATURE PUBLISHING GROUP, GB, vol. 1, no. 1, 1 October 2004 (2004-10-01) , pages 47-53, XP002532712, ISSN: 1548-7091, DOI: DOI:10.1038/NMETH704
- MICHAEL THOMSON: "Supplementary data for XP002532712", NATURE METHODS, vol. 1, no. 1, 1 October 2004 (2004-10-01) , XP55000149,
- LI Z ET AL: "Inhibition of PRL-3 gene expression in gastric cancer cell line SGC7901 via microRNA suppressed reduces peritoneal metastasis", BIOCHEMICAL AND BIOPHYSICAL RESEARCH COMMUNICATIONS, ACADEMIC PRESS, vol. 348, no. 1, 15 September 2006 (2006-09-15), pages 229-237, XP024924217, ISSN: 0006-291X, DOI: DOI:10.1016/J.BBRC.2006.07.043 [retrieved on 2006-09-15]
- CARLO M CROCE ET AL: "miRNAs, cancer, and stem cell division", CELL, CELL PRESS, US, vol. 122, no. 1, 15 July 2005 (2005-07-15) , pages 6-7, XP008129199, ISSN: 0092-8674, DOI: DOI:10.1016/J.CELL.2005.06.036
- PETROCCA FABIO ET AL: "MicroRNAs deregulation in gastric cancer", PROCEEDINGS OF THE ANNUAL MEETING OF THE AMERICAN ASSOCIATION FOR CANCER RESEARCH, AMERICAN ASSOCIATION FOR CANCER RESEARCH, US, vol. 47, 1 April 2006 (2006-04-01), page 1338, XP009148993, ISSN: 0197-016X
- PETROCCA FABIO ET AL: "E2F1-regulated microRNAs impair TGFbeta-dependent cell-cycle arrest and apoptosis in gastric cancer", CANCER CELL, CELL PRESS, US, vol. 13, no. 3, 1 March 2008 (2008-03-01), pages 272-286, XP009149018, ISSN: 1535-6108
- SCHETTER ET AL.: 'MicroRNA expression profiles associated with prognosis and therapeutic outcome in colon ademocarcinoma.' JAMA vol. 299, January 2008, pages 425 - 436
- AKAO ET AL.: 'let-7 microRNA functions as a potential growth suppressor in human colon cancer cells.' BIOL PHARM BULL vol. 29, 2006, pages 903 - 906
- LIU ET AL.: 'An oligonucleotide microchip for genome-wide microRNA profiling in human and mouse tissues.' PNAS vol. 101, 2004, pages 9740 - 9744
- Young-Kook Kim ET AL: "Processing of intronic microRNAs", The EMBO Journal, vol. 26, no. 3, 7 February 2007 (2007-02-07), pages 775-783, XP055116805, ISSN: 0261-4189, DOI: 10.1038/sj.emboj.7601512

## Description

### STATEMENT REGARDING FEDERALLY SPONSORED RESEARCH

This invention was made with government support under the NCI Grant Number(s) CA76259 and CA8134. The government has certain rights in this invention.

### TECHNICAL FIELD AND

### INDUSTRIAL APPLICABILITY OF THE INVENTION

This invention relates generally to the field of molecular biology. Certain aspects of the invention include application in diagnostics, therapeutics, and prognostics of gastric cancer related disorders.

### BACKGROUND OF THE INVENTION

Microarray technology has enabled the investigation of microRNA expression (Thomson et al., 2004). Research has identified several microRNAs involved in cancer pathogenesis (Volinia et al., 2006 and Ivanovska et al., 2007), thus allowing potential therapeutic targets to be identified (Li et al., 2006). Patent application WO 2007/081740 discloses methods and compositions for the diagnosis and treatment of solid cancers.

Although the incidence of gastric cancer declined in Western countries from the 1940s to the 1980s, it remains a major public health problem throughout the world, being the second most widely diagnosed malignancy worldwide and cause of 12% of all cancer-related deaths each year (Uemura et al., 2001). Over 95% of gastric tumors are adenocarcinomas histologically classified either as intestinal or diffuse type (Lauren P, 1965). The evolution of intestinal tumors has been characterized as progressing through a number of sequential steps. Among the others, two events are characteristic of gastric tumorigenesis: upregulation of E2F1 (Suzuki et al., 1999) and development of TGF-beta resistance (Ju et al., 2003; Park et al., 1994).

E2F1 is a master regulator of cell cycle that promotes the G1/S transition transactivating a variety of genes involved in chromosomal DNA replication, including its own promoter (DeGregori, 2002). While overexpression of E2F1 is an oncogenic event per se that predisposes cells to transformation (Pierce et al., 1999) it also represents a potent apoptotic signal when occurring over a critical threshold (Lazzerini Denchi et al., 2005).

On the other hand, Transforming Growth Factor-beta (TGFβ - is a cytokine playing a major role within the so-called morphogenetic program, a complex system of crosstalk between the epithelial and the stromal compartments that guides gastrointestinal cells towards proliferation, differentiation or apoptosis (van den Brink and Offerhaus, 2007).

In spite of considerable research into therapies to treat these diseases, they remain difficult to diagnose and treat effectively, and the mortality observed in patients indicates that improvements are needed in the diagnosis, treatment and prevention of the disease.

### SUMMARY OF THE INVENTION

In the present disclosure , there is provided a method of diagnosing whether a subject has, or is at risk for developing a gastric-related disorder, determining a prognosis of a subject with gastric cancer and/or related disorder, and/or treating the subject who has such disorder, comprising: measuring the level of at least one biomarker in a test sample from the subject, wherein an alteration in the level of the biomarker in the test sample, relative to the level of a corresponding biomarker in a control sample, is indicative of the subject either having, or being at risk for developing, the disorder.

According to the present invention, there is provided a method of diagnosing whether a subject has gastric adenocarcinoma, comprising: measuring the level of a biomarker in a test sample from the subject, wherein the biomarker is the miR-106b-25 cluster: miR-106b, miR-93 and miR-25 that are upregulated in the test sample, relative to the level of a corresponding biomarker in a control sample, being indicative of the subject having gastric adenocarcinoma.

In another aspect, the invention also provides the use of a biomarker of gastric adenocarcinoma, consisting of the miR-106b-25 cluster: miR-106b, miR-93 and miR-25, for diagnosing gastric adenocarcinoma.

In certain embodiments, the level of the at least one biomarker in the test sample is less than the level of the corresponding biomarker in the control sample.

In certain embodiments, the level of the at least one biomarker in the test sample is greater than the level of the corresponding biomarker in the control sample.

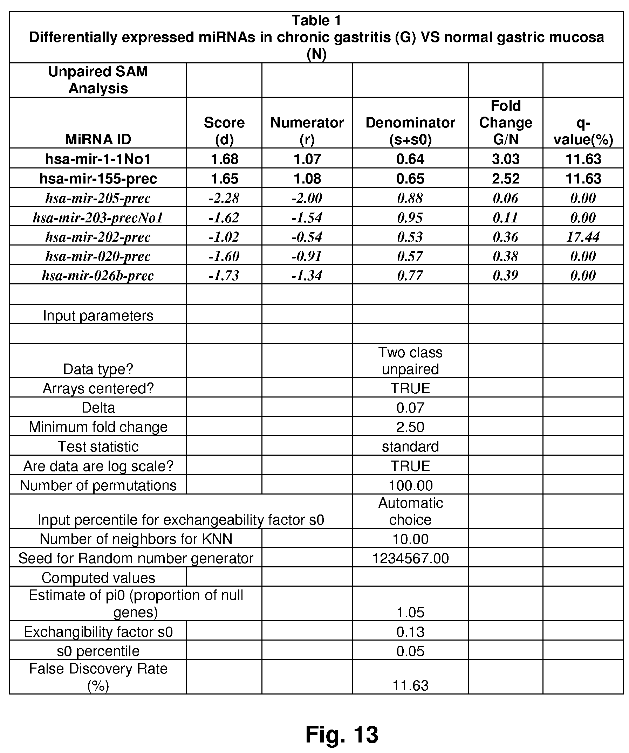

In certain embodiments, at least one biomarker differentially expressed is selected from the group listed in Figure 13 - Table 1.

In the present disclosure, the disorder comprises chronic gastritis and at least one biomarker is selected from the group consisting of: miR-1 and miR-155 that are up-regulated.

In the present disclosure, the disorder comprises chronic gastritis and at least one biomarker is selected from the group consisting of: miR-205, miR-203, miR-202, miR-20 and miR-26b that are down-regulated.

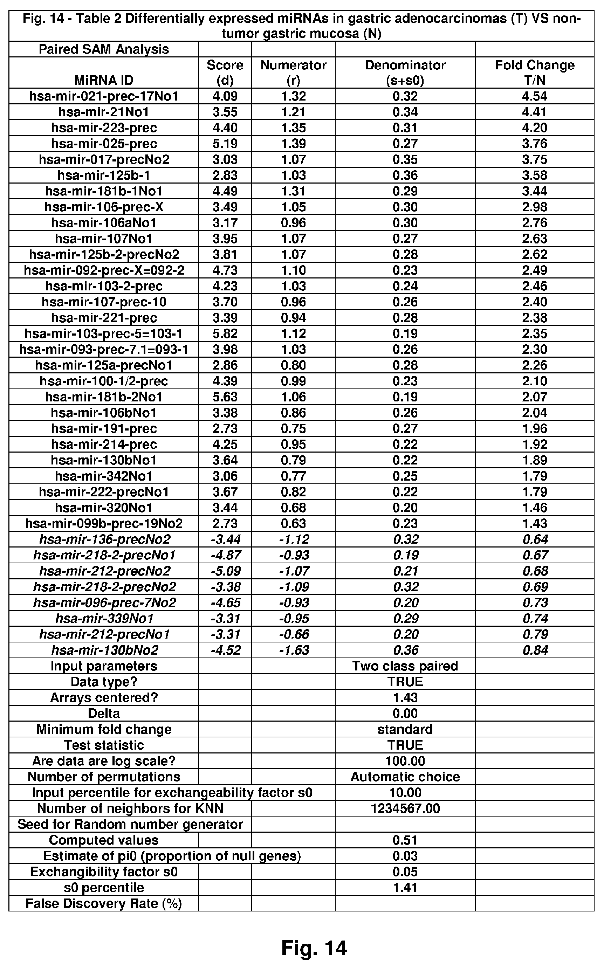

In the present disclosure, the at least one biomarker differentially expressed is selected from the group listed in Figure 14 - Table 2.

In the present disclosure, the disorder comprises gastric adenocarcinoma and at least one biomarker is selected from the group consisting of miR-21, miR-223, miR-25, miR-17-5-p, miR-125b, miR-181b, miR-106a, miR-107, miR-92, miR-103, miR-221, miR-93, miR-100, miR-181, miR-106b, miR-191, miR-214, miR-130, miR-342, miR-222, miR-320 and miR-99b that are up-regulated.

In the present disclosure, the disorder comprises gastric adenocarcinoma and at least one biomarker is selected from the group consisting of: miR-136, miR-218, miR-212, miR-96, miR-339 and miR-130b that are down-regulated.

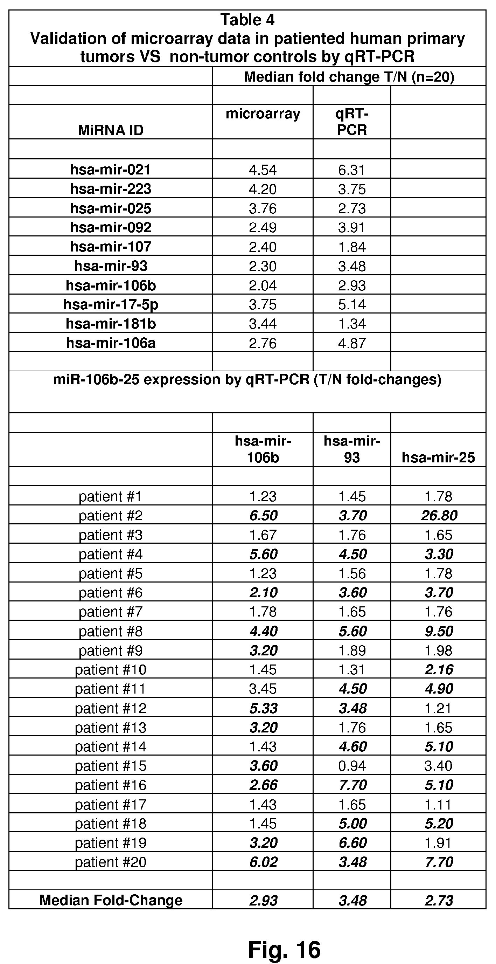

In the present disclosure, at least one biomarker differentially expressed is selected from the group listed in Figure 16 - Table 3: miR-21, miR-223, miR-25, miR-92, miR-107, miR-93, miR-106b, miR-17-5p, miR-181b and miR-106a.

In certain embodiments, at least one biomarker comprises the miR-106b-25 cluster: miR-106b, miR-93 and miR-25.

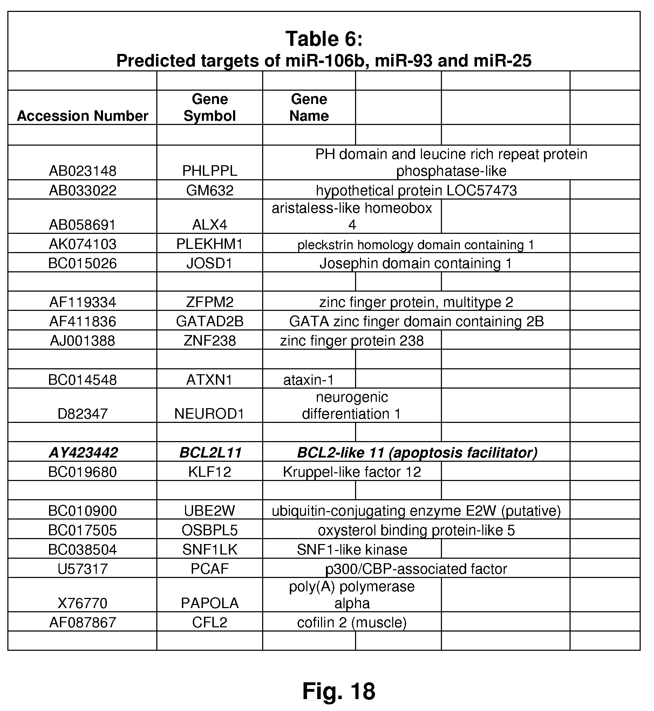

The present disclosure relates to at least one biomarker comprising the miR-106b-25 cluster: miR-106b, miR-93 and miR-25, in the modulation of expression of one or more of the genes listed in Figure 18 - Table 6: PHLPPL, GM632, ALX4, PLEKHM1, JOSD1, ZFPM2, GATAD2B, ZNF238, ATXN1, NEUROD1, BCL2L11, KLF12, UBE2W, OSBPL5, SNF1LK, PCAF, PAPOLA, and CFL2.

The present disclosure relates to a method for regulating E2F1 expression in a subject in need thereof, comprising administering an effective amount of miR-106b and/or miR-93, or a functional variant thereof, sufficient to modulate expression of E2F1.

The present disclosure relates to the use of miR-106b and miR-93 to regulate E2F1 expression in a subject in need thereof.

The present disclosure relates to a method modulating a TGF-beta tumor suppressor pathway that interferes with expression of CDKN1A (p21Waf1/Cip) and/or BCL2L11 (Bim), comprising up-regulating one or more of miR-106b, miR-93 and miR-25.

The present disclosure relates to use of the miR-106b-25 cluster in E2F1 post-transcriptional regulation and modulation of development of TGF-beta resistance in gastric cancer.

The present disclosure relates to a method for controlling E2F1 expression in a subject in need thereof, comprising modulating levels of miR-106b and miR-93 in the subject.

The present disclosure relates to the use of E2F1 to regulates miR-106b-25 expression in parallel with Mcm7, in a subject in need thereof.

The present disclosure relates to a method for controlling the rate of E2F1 protein synthesis, preventing its excessive accumulation in a subject in need thereof, comprising modulating levels of the miR-106b-25 cluster in the subject.

The present disclosure relates to the use of miR-106b and miR-93 to impair TGF-beta-induced cell cycle arrest in a subject in need thereof.

The present disclosure relates to the use of miR-106b and miR-93 to interfere with TGF-beta-induced cell cycle arrest by inhibiting expression of p21 at a post-transcriptional level in a subject in need thereof.

The present disclosure relates to the use of miR-25 in cooperation with miR-106b and miR-93 in preventing the onset of TGF-beta-induced apoptosis, in a subject in need thereof.

The present disclosure relates to a method for modulating expression of the miR-106b-25 cluster to prevent protection of gastric cancer cells from apoptosis in a subject in need thereof.

The present disclosure relates to a distinct microRNA expression signature in gastric cancer comprising alterations in the expression of one or more biomarkers that regulate tumor microRNA processing.

The present disclosure relates to a method for influencing transcript abundance and/or protein expression of target mRNAs in gastric cancer, comprising deregulating one or more microRNAs in a subject in need thereof.

In the present disclosure, the method further comprises inhibiting the protein expression of cancer-related genes.

In the present disclosure, the method further comprises altering expression of one or more of miR-106b, miR-93 and miR-25 to inhibit the protein expression of cancer-related genes.

The present disclosure relates to the use of a large-scale gene expression profiling of both microRNAs and protein-encoding RNAs to identify alterations in microRNA function that occur in human gastric cancer.

The present disclosure relates to a method comprising determining the prognosis of a subject with gastric cancer, comprising measuring the level of at least one biomarker in a test sample from the subject, wherein: i) the biomarker is associated with an adverse prognosis in such cancer; and ii) an alteration in the level of the at least one biomarker in the test sample, relative to the level of a corresponding biomarker in a control sample, is indicative of an adverse prognosis.

In present disclosure, the method further comprises diagnosing whether a subject has, or is at risk for developing, gastric cancer, comprising: 1) reverse transcribing RNA from a test sample obtained from the subject to provide a set of target oligodeoxynucleotides; 2) hybridizing the target oligodeoxynucleotides to a microarray comprising miRNA-specific probe oligonucleotides to provide a hybridization profile for the test sample; and 3) comparing the test sample hybridization profile to a hybridization profile generated from a control sample, wherein an alteration in the signal of at least one miRNA is indicative of the subject either having, or being at risk for developing, such cancer.

In certain embodiments, the signal of at least one miRNA, relative to the signal generated from the control sample, is down-regulated, and/or wherein the signal of at least one miRNA, relative to the signal generated from the control sample, is up-regulated.

In the present disclosure certain embodiments, an alteration in the signal of at least one biomarker selected from the group listed in: Table 13, Table 14 and Table 16, are indicative of the subject either having, or being at risk for developing, such cancer with an adverse prognosis.

In another aspect, there is provided herein a biomarker of a gastric disorder or disease, comprising one or more of: miR-106b, miR-93 and mir-25.

The present disclosure relates to a method for regulating protein expression in gastric cancer cells, comprising modulating the expression of one or more of: miR-106b, miR-93 and mir-25 in the gastric cancer cells.

The present disclosure relates to a composition for modulating expression of one or more of E2F1, CDKN1A (p21Waf1Cip1) and BCL2L11 (Bim) in gastric cancer cells, the composition comprising one or more of: miR-106b, miR-93 and mir-25, or functional variants thereof.

The present disclosure relates to a method for regulating one or more of E2F1 and p21/WAF1 protein levels in a subject in need thereof, comprising using one or more of: miR-106b, miR-93 and mir-25, or functional variants thereof.

The present disclosure relates to a composition comprising antisense miR-106b useful to increase p21/WAF1 and/or E2F1 protein levels in gastric cancer cells in a subject in need thereof.

The present disclosure relates to a method of treating gastric cancer in a subject who has a gastric cancer in which at least one biomarker is down-regulated or up-regulated in the cancer cells of the subject relative to control cells, comprising: 1) when the at least one biomarker is down-regulated in the cancer cells, administering to the subject an effective amount of at least one isolated biomarker, or an isolated variant or biologically-active fragment thereof, such that proliferation of cancer cells in the subject is inhibited; or 2) when the at least one biomarker is up-regulated in the cancer cells, administering to the subject an effective amount of at least one compound for inhibiting expression of the at least one biomarker, such that proliferation of cancer cells in the subject is inhibited.

The present disclosure relates to a method of treating gastric cancer in a subject, comprising: 1) determining the amount of at least one biomarker in gastric cancer cells, relative to control cells; and 2) altering the amount of biomarker expressed in the gastric cancer cells by: i) administering to the subject an effective amount of at least one isolated biomarker, if the amount of the biomarker expressed in the cancer cells is less than the amount of the biomarker expressed in control cells; or ii) administering to the subject an effective amount of at least one compound for inhibiting expression of the at least one biomarker, if the amount of the biomarker expressed in the cancer cells is greater than the amount of the biomarker expressed in control cells.

The present disclosure relates to a pharmaceutical composition for treating gastric cancer, comprising at least one isolated biomarker, and a pharmaceutically-acceptable carrier.

In the present disclosure at least one isolated biomarker corresponds to a biomarker that is down-regulated in gastric cancer cells relative to control cells.

In the present disclosure , the pharmaceutical composition comprises at least one miR expression-inhibitor compound and a pharmaceutically-acceptable carrier.

The present disclosure relates to a method of identifying an anti-gastric cancer agent, comprising providing a test agent to a cell and measuring the level of at least one biomarker associated with decreased expression levels in gastric cancer cells, wherein an increase in the level of the biomarker in the cell, relative to a control cell, is indicative of the test agent being an anti-gastric cancer agent.

The present disclosure relates to a method of identifying an anti-gastric cancer agent, comprising providing a test agent to a cell and measuring the level of at least one biomarker associated with increased expression levels in gastric cancer cells, wherein a decrease in the level of the biomarker in the cell, relative to a control cell, is indicative of the test agent being an anti- cancer agent.

The present disclosure relates to a method of assessing the effectiveness of a therapy to prevent, diagnose and/or treat a gastric cancer associated disease, comprising: i) subjecting an animal to a therapy whose effectiveness is being assessed, and ii) determining the level of effectiveness of the treatment being tested in treating or preventing the disease, by evaluating at least one biomarker listed in one or more of Tables 13, 14 and 16.

The present disclosure relates to, wherein the candidate therapeutic agent comprises one or more of: pharmaceutical compositions, nutraceutical compositions, and homeopathic compositions.

In the present disclosure, the therapy being assessed is for use in a human subject.

The present disclosure relates to an article of manufacture comprising: at least one capture reagent that binds to a marker for a gastric cancer associated disease comprising at least one biomarker listed in one or more of Tables 13, 14 and 16.

The present disclosure relates to a kit for screening for a candidate compound for a therapeutic agent to treat a gastric cancer associated disease, wherein the kit comprises: one or more reagents of at least one biomarker listed in one or more of Tables 13, 14 and 16, and a cell expressing at least one biomarker.

In the present disclosure, the presence of the biomarker is detected using a reagent comprising an antibody or an antibody fragment which specifically binds with at least one biomarker.

The present disclosure relates to an agent that interferes with a gastric cancer associated disease response signaling pathway, for the manufacture of a medicament for treating, preventing, reversing or limiting the severity of the disease complication in an individual, wherein the agent comprises at least one biomarker listed in one or more of Tables 13, 14 and 16.

The present disclosure relates to a method of treating, preventing, reversing or limiting the severity of a gastric cancer associated disease complication in an individual in need thereof, comprising: administering to the individual an agent that interferes with at least a gastric cancer associated disease response cascade, wherein the agent comprises at least one biomarker listed in one or more of Tables 13, 14 and 16.

The present disclosure relates to use of an agent that interferes with at least a gastric cancer associated disease response cascade, for the manufacture of a medicament for treating, preventing, reversing or limiting the severity of a gastric cancer-related disease complication in an individual, wherein the agent comprises at least one biomarker listed in one or more of Tables 13, 14 and 16.

The present disclosure relates to a composition comprising an antisense inhibitor of one or more of miR-1o6b, miR-93 and miR-25.

The present disclosure relates to a method of treating a gastric disorder in a subject in need thereof, comprising administering to a subject a therapeutically effective amount of the composition.

In the present disclosure, the composition is administered prophylactically.

In the present disclosure, administration of the composition delays the onset of one or more symptoms of gastric cancer.

In the present disclosure, administration of the composition inhibits development of gastric cancer.

In the present disclosure, administration of the composition inhibits tumor growth.

In another aspect, there is provided herein a method for detecting the presence of gastric cancer in a biological sample, comprising: i) exposing the biological sample suspected of containing gastric cancer to a marker therefor; and ii) detecting the presence or absence of the marker, if any, in the sample.

In certain embodiments, the marker includes a detectable label.

In certain embodiments, the method further comprises comparing the amount of the marker in the biological sample from the subject to an amount of the marker in a corresponding biological sample from a normal subject.

In certain embodiments, the method further comprises collecting a plurality of biological samples from a subject at different time points and comparing the amount of the marker in each biological sample to determine if the amount of the marker is increasing or decreasing in the subject over time.

The present disclosure relates to a method for treating a gastric cancer in a subject, the method comprising: gastric cancer receptor agonist.

In the present disclosure, the receptor agonist is an antisense inhibitor of one or more of: miR-106b, miR-93 and miR-25.

The present disclosure relates to a use, to manufacture a drug for the treatment of gastric cancer, comprised of a nucleic acid molecule chosen from among the miR shown in Tables 13, 14 and 16, a sequence derived therefrom, a complementary sequence from such miR and a sequence derived from such a complementary sequence.

In the present disclosure, the drug comprises a nucleic acid molecule presenting a sequence chosen from among: miRs listed in Tables 13, 14 and 16, a sequence derived from such miRs, the complementary sequence of such miRs, and a sequence derived from such a complementary sequence.

The present disclosure relates to an in vitro method to identify effective therapeutic agents or combinations of therapeutic agents to induce the differentiation of gastric cancer cells, the method comprising the stages of: i) culturing of cells derived from a gastric tumor, ii) adding at least one compound to the culture medium of the cell line, iii) analyzing the evolution of the level of expression of at least one miR between stages (i) and (ii), and iv) identifying compounds or combinations of compounds inducing a change in the level of expression of the miR between stages (i) and (ii).

In the present disclosure, stage (iii) includes the analysis of the level of expression of at least one miR.

In the present disclosure, stage (iv) includes the identification of the compounds or combinations of compounds modulating the level of expression of at least one miR.

In the present disclosure, stage (iv) includes the identification of compounds or combinations of compounds reducing the level of expression of at least one miR.

In the present disclosure, the compound is a therapeutic agent for the treatment of cancer.

Various objects and advantages of this invention will become apparent to those skilled in the art from the following detailed description of the preferred embodiment, when read in light of the accompanying drawings.

### BRIEF DESCRIPTION OF THE DRAWINGS

The patent or application file may contain one or more drawings executed in color and/or one or more photographs. Copies of this patent or patent application publication with color drawing(s) and/or photograph(s) will be provided by the Patent Office upon request and payment of the necessary fee.

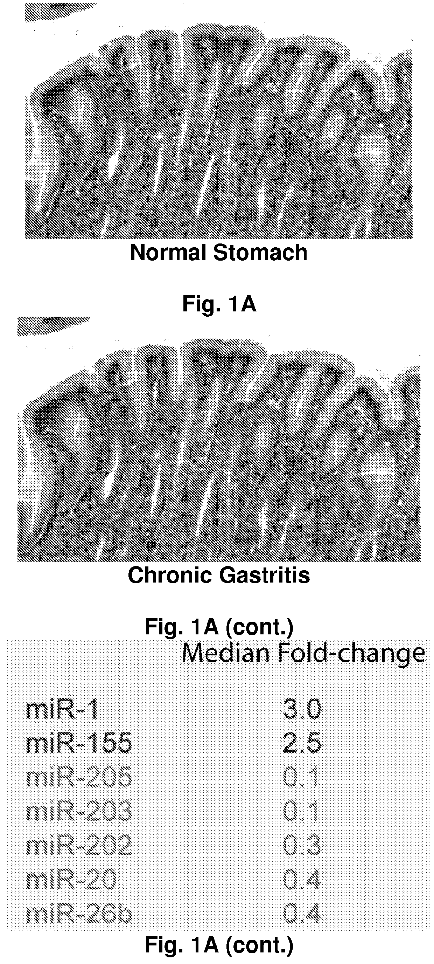

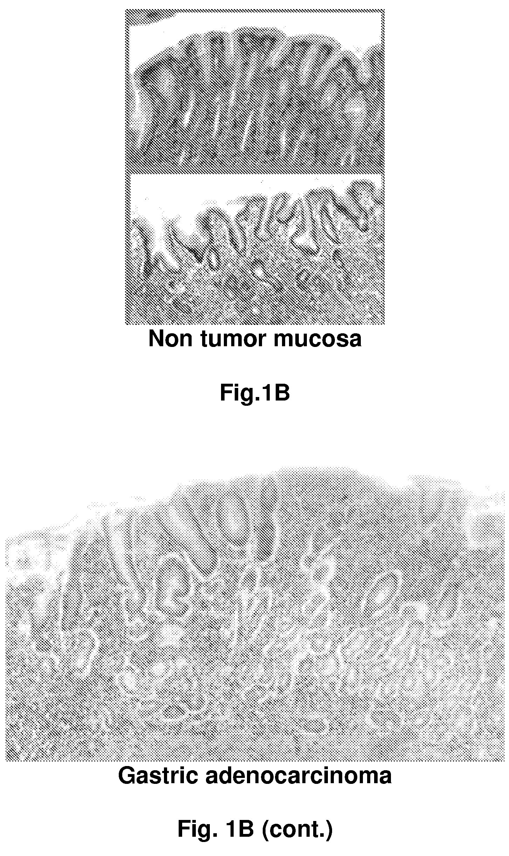

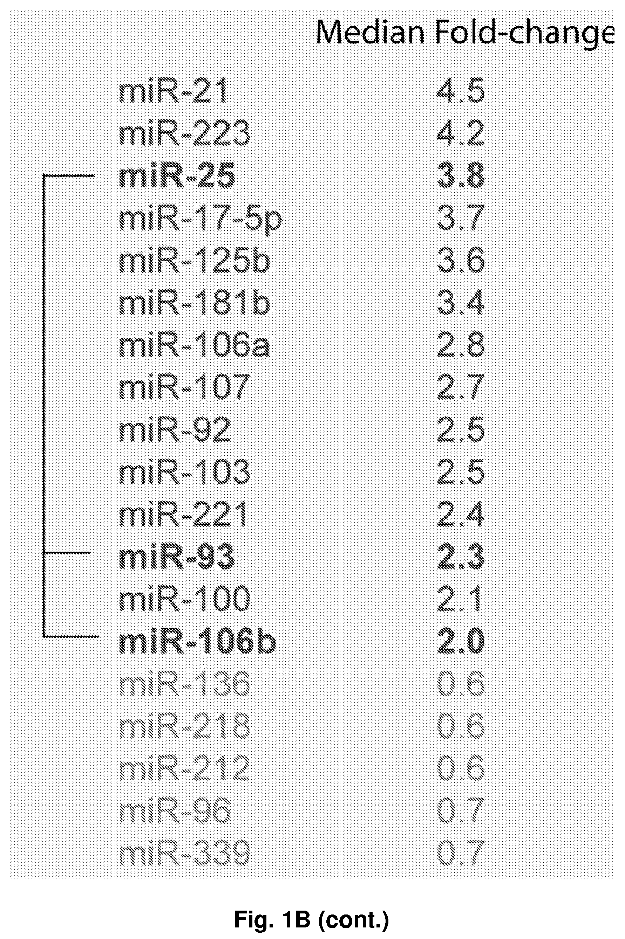

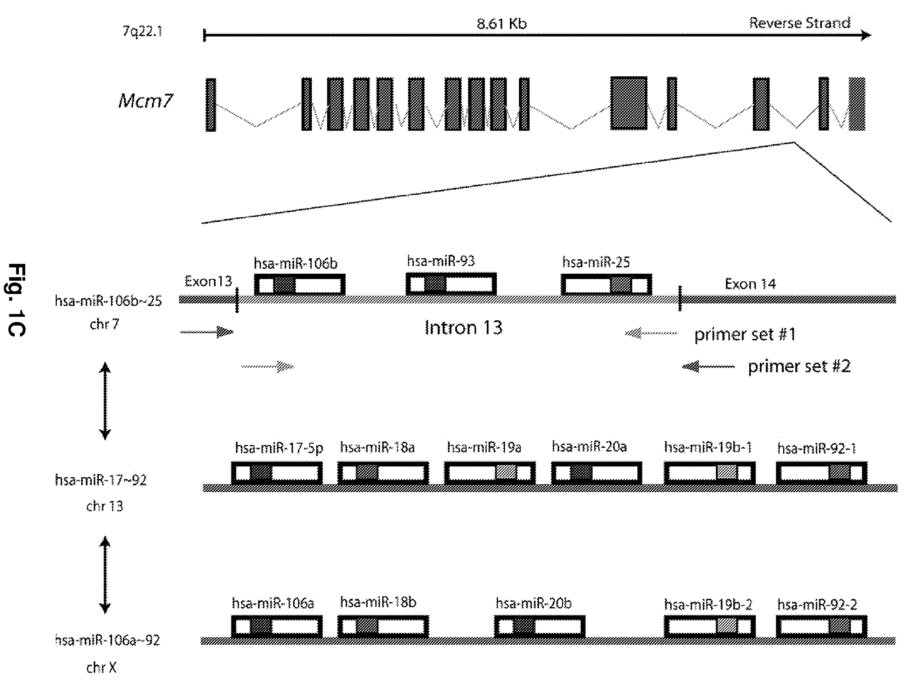

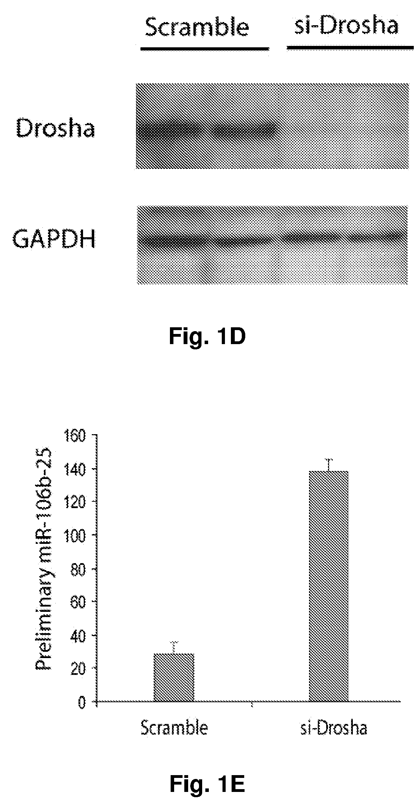

**Figures 1A-1E****:** Alteration of miRNA expression in chronic gastritis and gastric adenocarcinoma. MiRNAs significantly associated with either chronic gastritis **(****Fig. 1A****)** or gastric adenocarcinoma **(****Fig. 1B****)** by SAM analysis (FDR=0%, q=0). Red and Green colors indicate upregulation and downregulation, respectively. Representative histological features of normal gastric mucosa, chronic gastritis and gastric adenocarcinoma are shown, Hematoxylin & Eosin (H&E) staining. **Fig. 1C****:** Schematic representation of the miR-106b-25 cluster genomic locus hosted in the intron 13 of Mcm7. The primary transcript of this gene contains all the three miRNAs fused into a unique molecule that we retrotranscribed, amplified and sequenced from Snu-16 cells using two different sets of primers (#1 and #2). This molecule is not just a by-product of Mcm7 transcription as downregulation of Drosha by RNAi **(****Fig. 1D****)** induced a dramatic accumulation of this transcript **(****Fig. 1E****)** confirming the presence of an active preliminary miRNA. Bars indicate RNA expression normalized to U6 +/- SD. This cluster shares a high degree of homology with miR-17-92 and miR-106a-92 clusters, located on chromosomes 13 and X, respectively. Colors identify miRNAs of the same family.

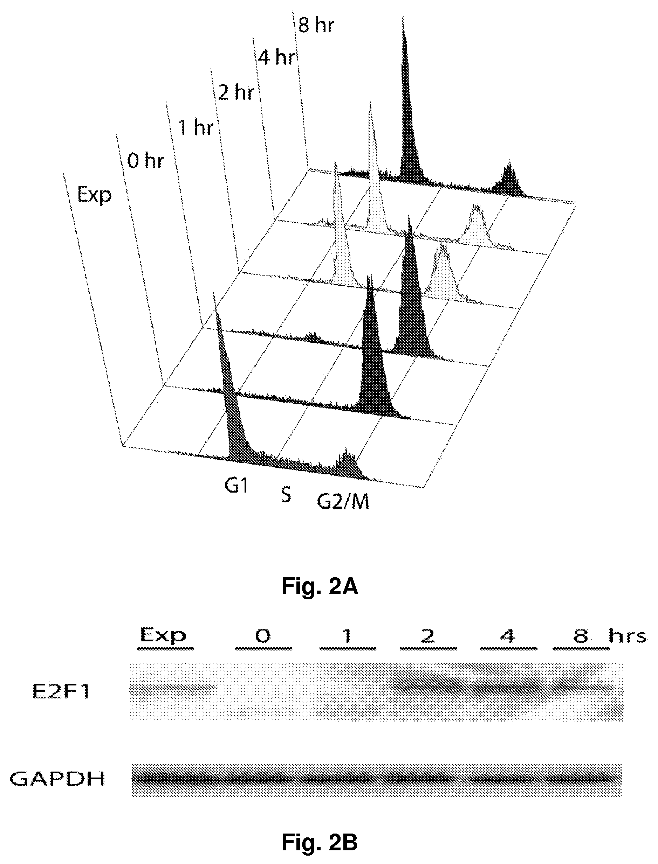

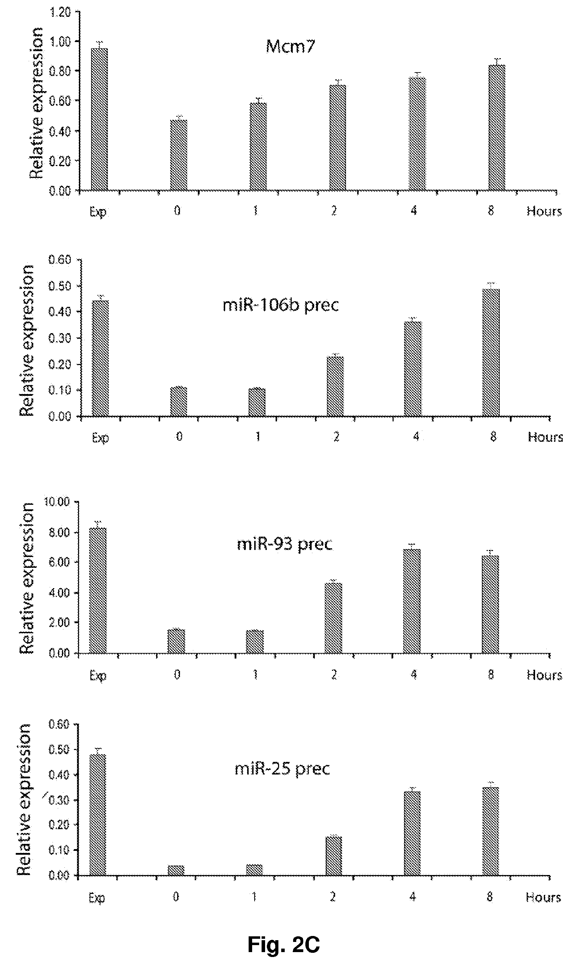

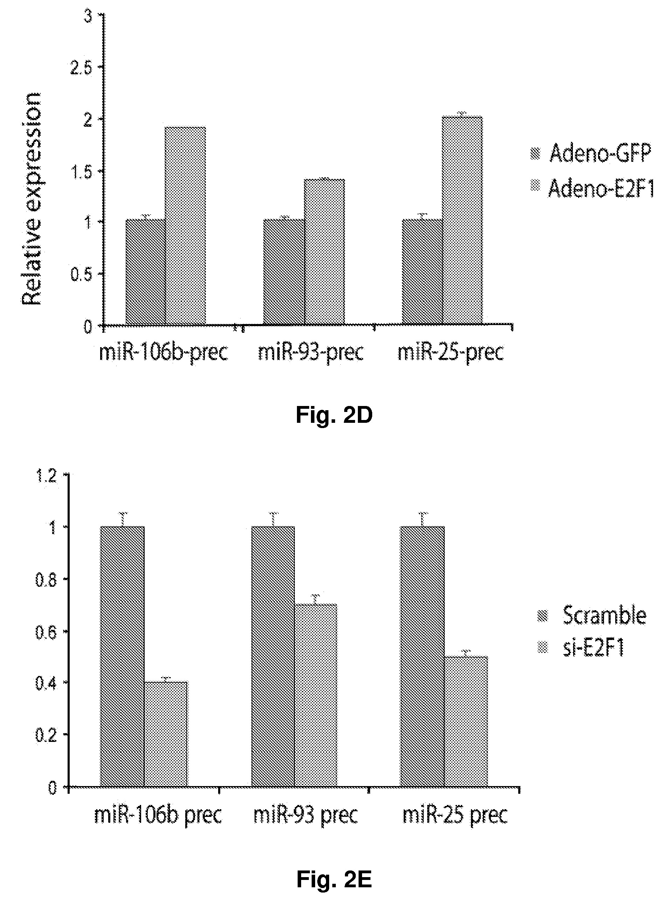

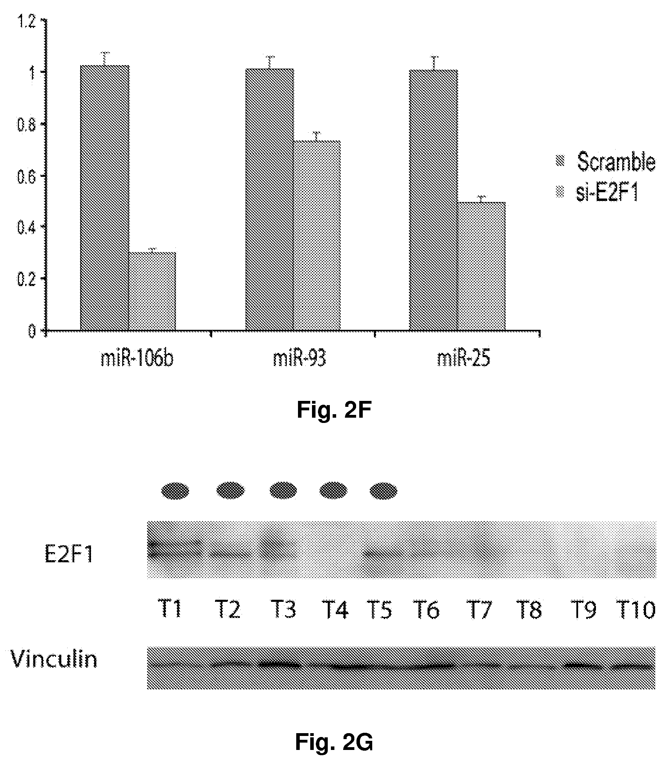

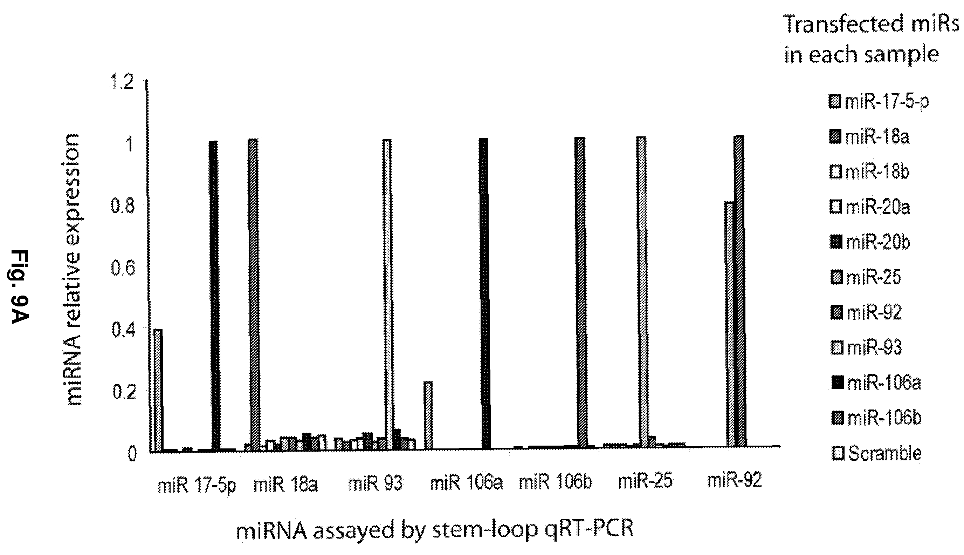

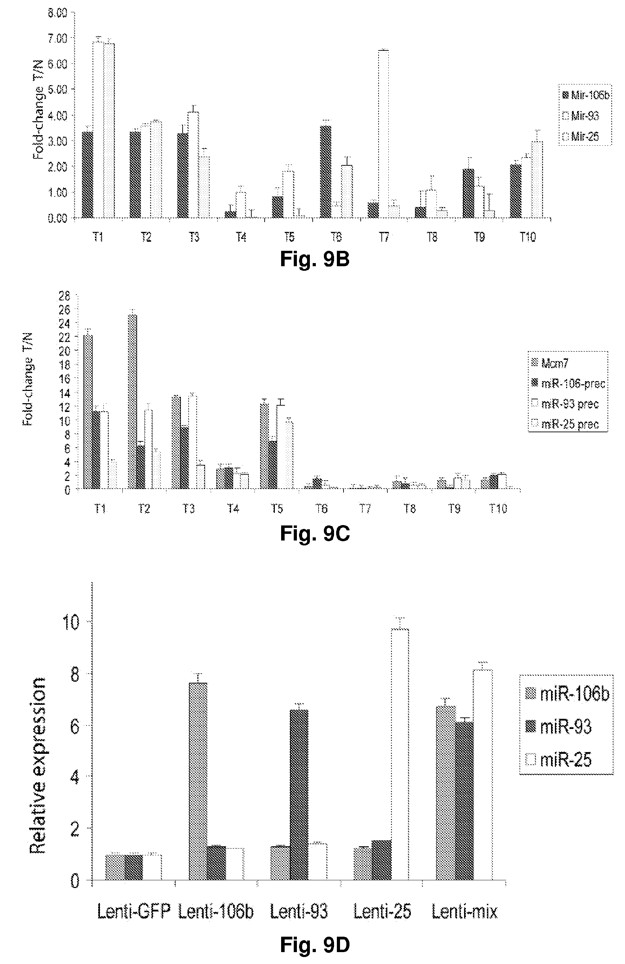

**Figures 2A**-**2G****:** E2F1 regulates miR-106b-25 expression. Fig. 2A: FACS analysis of AGS cells synchronized in mitosis by nocodazole treatment for 12 hours and subsequently released in fresh medium. Cells were harvested at different time points and analyzed for E2F1 protein content by Western Blot **(****Fig. 2B****)** and Mcm7, miR-106b, miR-93 and miR-25 precursors RNA levels by qRT-PCR **(****Fig. 2C****).** Each analysis was performed in triplicate. Bars indicate RNA expression normalized to U6 +/- SD. AGS cells were plated at 90% confluence and starved in 0.5% FBS RPMI 1640 medium for 36 hours. Cells were then infected with either adeno-GFP or adeno-E2F1 viruses at a M.O.I, of 25 and incubated for additional 21 hours: at this time, cells displayed no signs of apoptosis, as determined by morphology, trypan-blue staining and analysis of subdiploid DNA content (data not shown). MiR-106b, miR-93 and miR-25 precursors were measured by qRT-PCR as above. Snu-16 cells were transfected with a siRNA against E2F1 (100 nM) and expression of miR-106b-25 precursor **(****Fig. 2E****)** and mature **(****Fig. 2F****)** species was determined after 72 hours by qRT-PCR, as above. Bars indicate RNA expression normalized to U6 +/- SD. **Fig. 2G****:** Expression of E2F1 protein in the same gastric primary tumors presented in **Figure 9****.** Red circles indicate overexpression of Mcm7 and miR-106b-25 precursor RNA in the corresponding tumors, as determined by qRT-PCR.

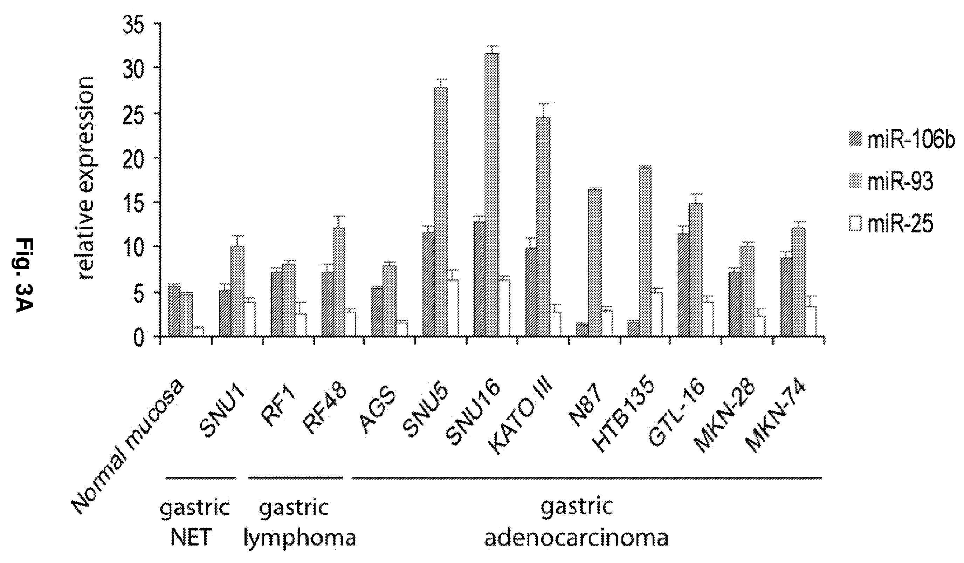

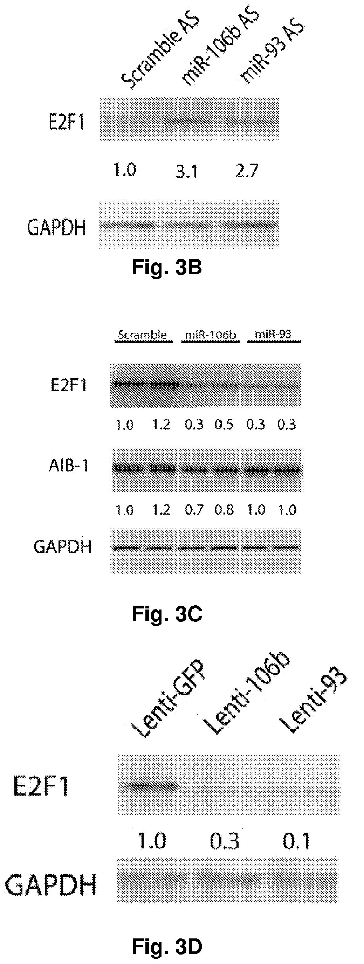

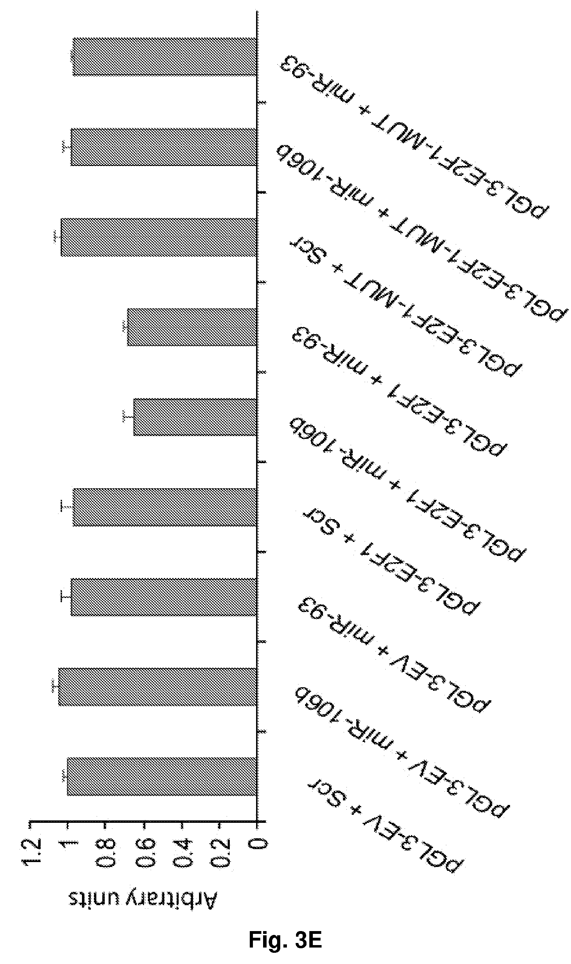

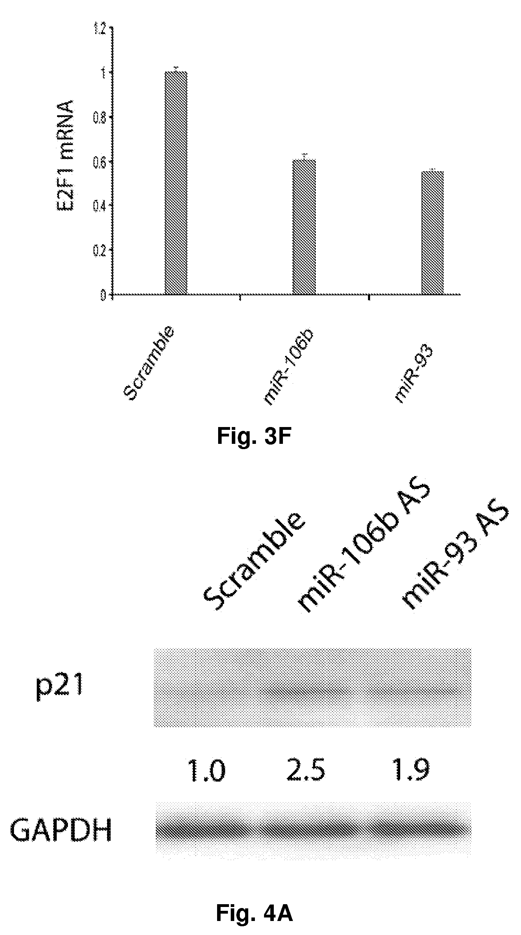

**Figures 3A**-**3F****:** E2F1 is a target of miR-106b and miR-93. **Fig. 3A****:** Endogenous expression of mature miR-106b, miR-93 and miR-25 in human gastric cancer cell lines and normal mucosa determined by stem-loop qRT-PCR; bars indicate RNA expression normalized to U6 +/- SD. Snu-1 cells are thought to derive from a gastric neuroendocrine tumor (NET) while RF1 and RF48 cells are from a B-cell lymphoma of the stomach. All the other cell lines are from gastric adenocarcinoma. **Fig. 3B****:** Western Blot of Snu-16 cells 48 hours after inhibition of miR-106b and miR-93 by ASO transfection or **(****Fig. 3C****)** overexpression of the same miRNAs by oligonucleotide transfection or **(****Fig. 3D****)** lentiviral transduction. Scramble RNA or LNA oligonucleotides were used as negative control. Protein expression was quantified and normalized to GAPDH. Similar results were obtained in AGS and MKN-74 cells (data not shown). **(****Fig. 3E****)** Luciferase assay showing decreased luciferase activity in cells cotransfected with pGL3-E2F1-3'UTR and miR-106b or miR-93 oligonucleotides. Deletion of the first three bases in three putative miR-106b/miR-93 binding sites, complementary to miRNA seed regions, abrogates this effect (MUT). Bars indicate Firefly luciferase activity normalized to Renilla luciferase activity +/- SD. Each reporter plasmid was transfected at least twice (on different days) and each sample was assayed in triplicate. **Fig. 3F****:** qRT-PCR analysis showing E2F1 mRNA downregulation in the same cells presented in **Fig. 3C****.** Bars indicate RNA expression normalized to U6 +/- SD.

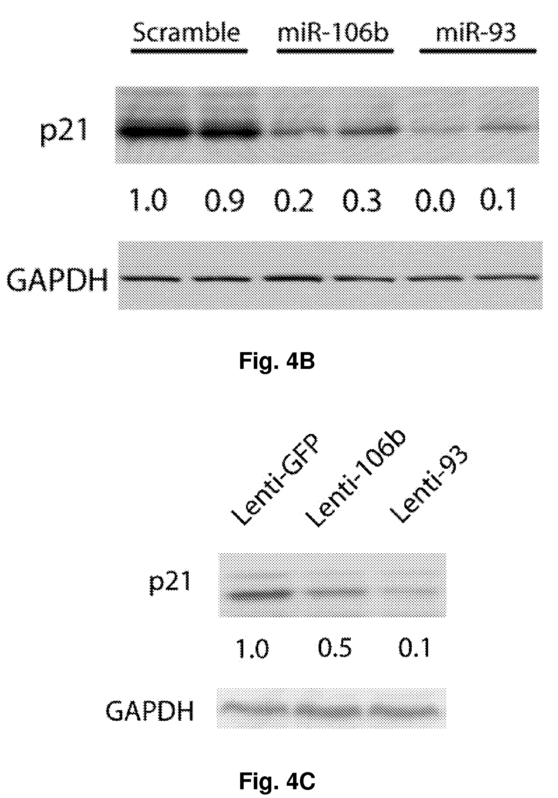

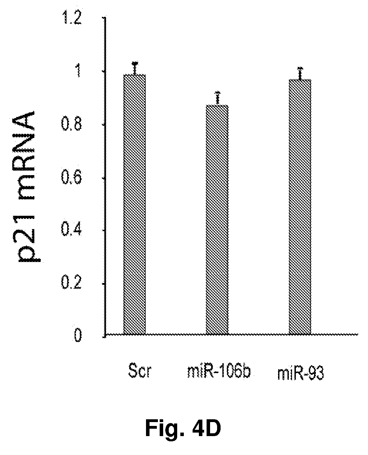

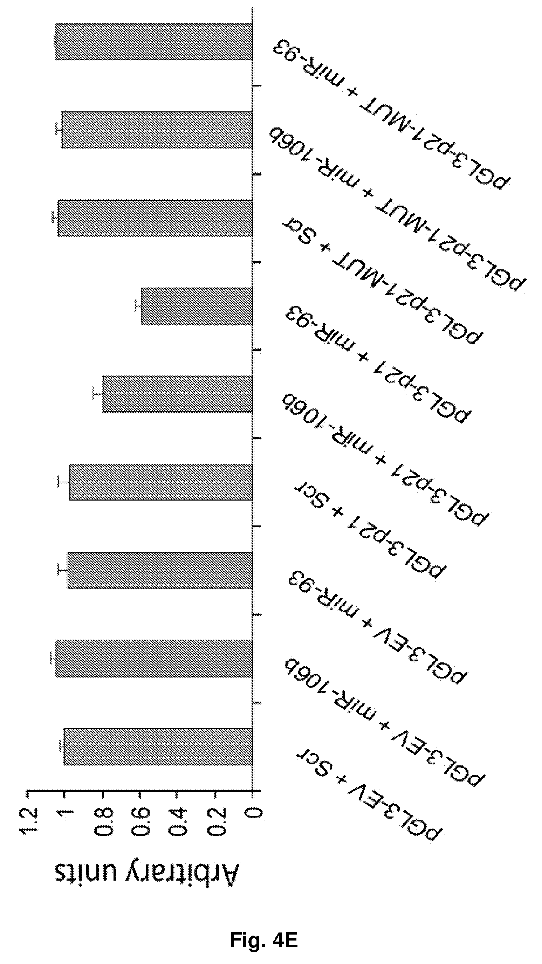

**Figures 4A-4E****:** miR-106b and miR-93 repress p21 protein expression. **Fig**. **4A****:** P21 expression in Snu-16 cells grown in 0.5% FBS RPMI 1640 after transfection with either miR-106b and miR-93 ASOs **(****Fig. 4A****)** or mimics **(Fig. 4BA)** or upon lentiviral transduction of the same miRNAs **(****Fig. 4C****).** **Fig. 4D****:** qRT-PCR results showing no significant difference in p21 mRNA levels in Snu-16 cells transfected with either miR-106b or miR-93 oligonucleotides. Bars indicate RNA expression normalized to U6 +/- SD. **Fig. 4E****:** Reporter assay showing decreased luciferase activity in cells cotransfected with pGL3-p21-3'UTR and miR-106b or miR-93 oligonucleotides. Deletion of the first 3 bases of miR-106b/miR-93 predicted binding site, complementary to miRNA seed regions, abrogates this effect (MUT). Bars indicate Firefly luciferase activity normalized to Renilla luciferase activity +/- SD. Each reporter plasmid was transfected at least twice (on different days) and each sample was assayed in triplicate.

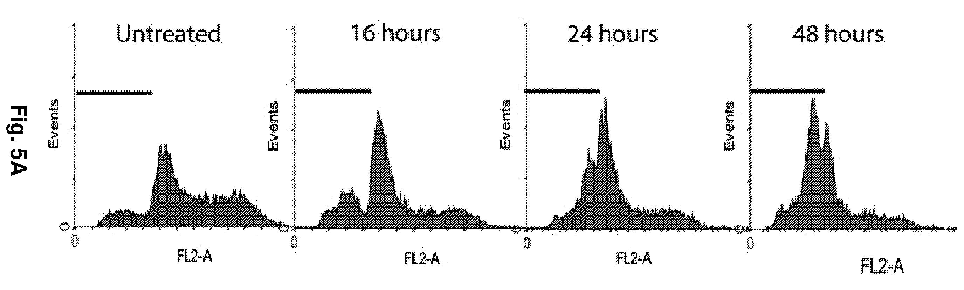

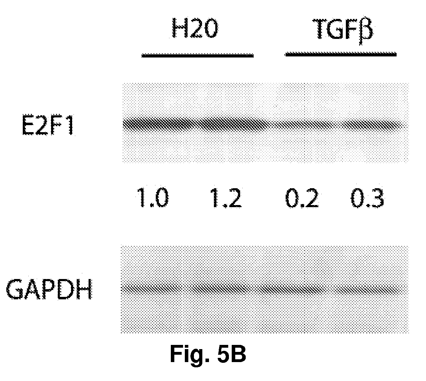

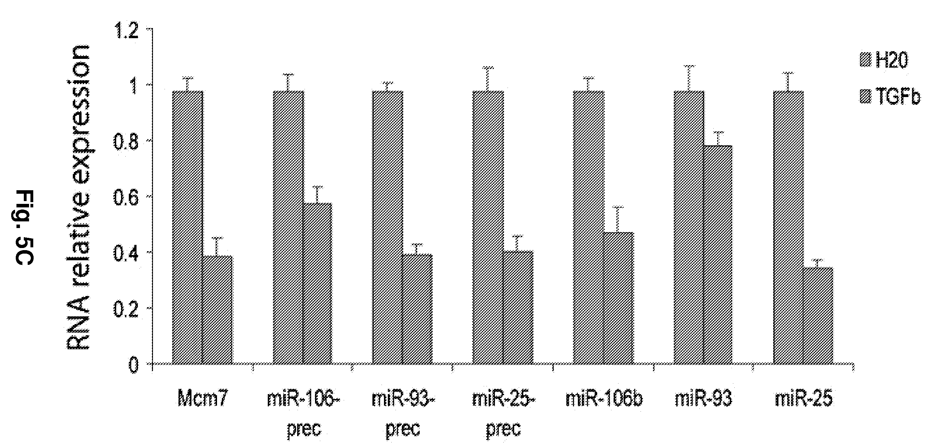

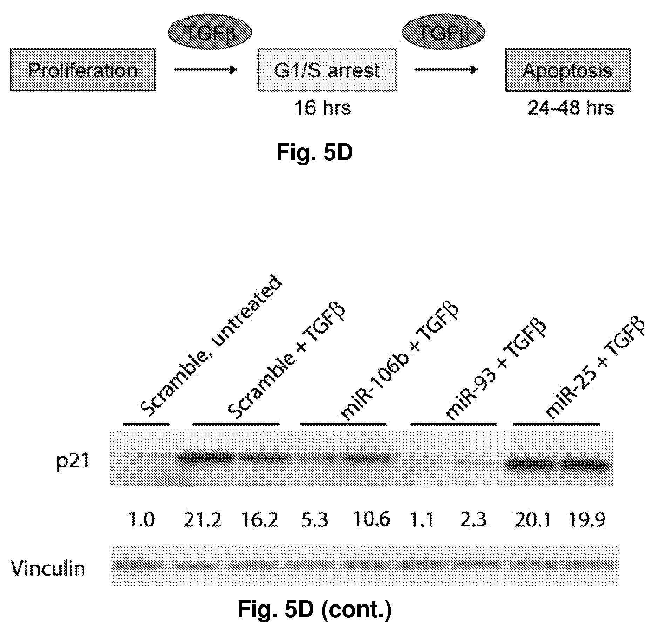

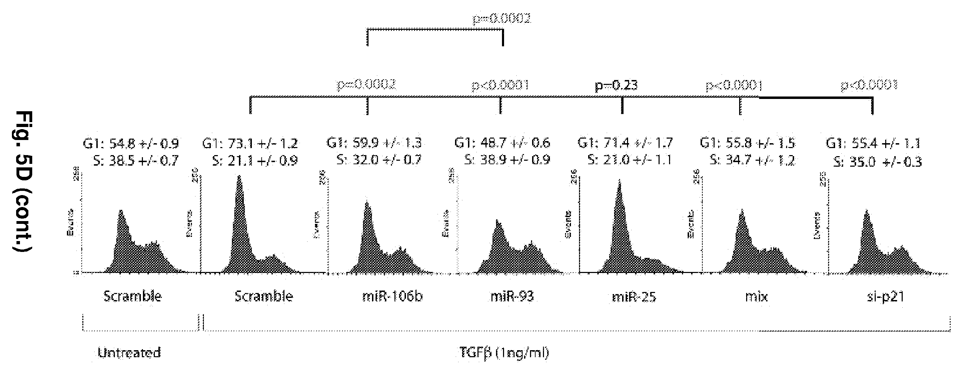

**Figure 5A-5D****:** Overexpression of miR-106b and miR-93 interfere with TGF-beta-dependent G1/S cell cycle arrest. **Fig. 5A****:** Physiological response of Snu-16 cells to 1 ng/ml TGF-beta: in the early phases of stimulation (16 hours) cells undergo a G1/S cell cycle arrest while apoptosis is still limited, as determined by sub-diploid DNA content. The number of cells undergoing apoptosis progressively increases in the following hours. **Fig. 5B****:** Downregulation of E2F1 protein and **(****Fig. 5C****)** Mcm7, miR-106b, miR-93 and miR25 precursors 16 hours after TGF-beta stimulation. Bars indicate RNA expression normalized to U6 +/- SD. **Fig. 5D****:** Snu-16 cells were transfected with the indicated oligonucleotides and treated with 1 ng/ml TGF-beta after 12 hours. Upper panel: p21 protein expression. Bottom panel: FACS analysis, comparison of G1/S fractions between mock and miRNA transfected cells using unpaired t-test.

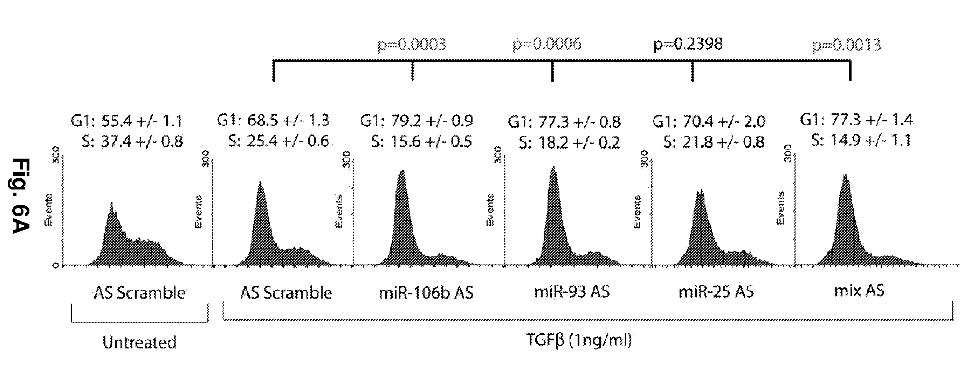

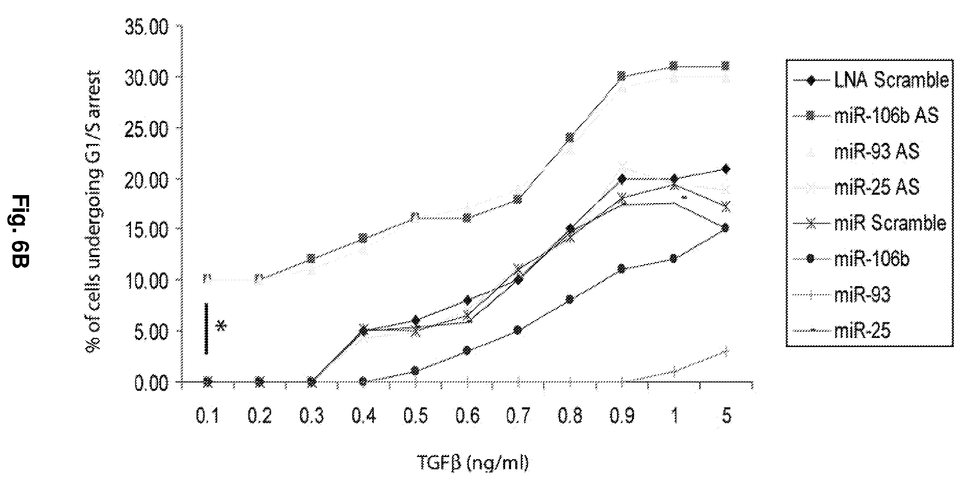

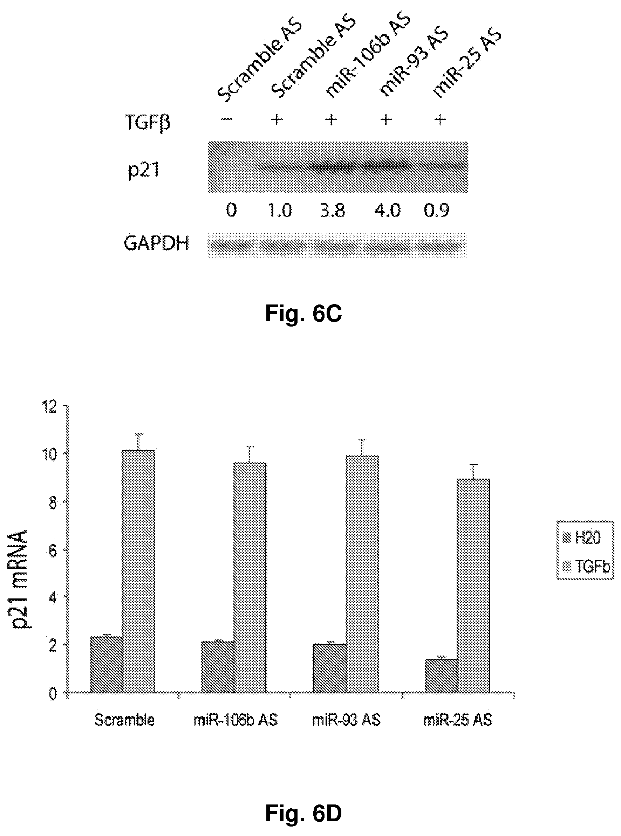

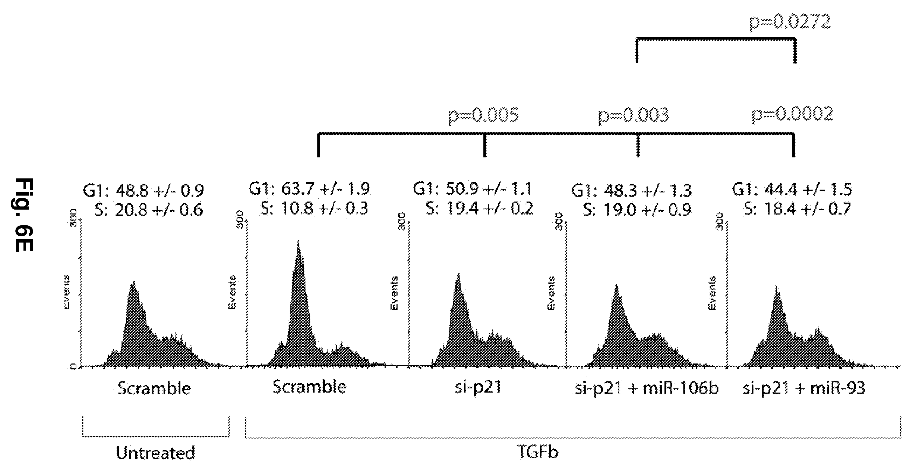

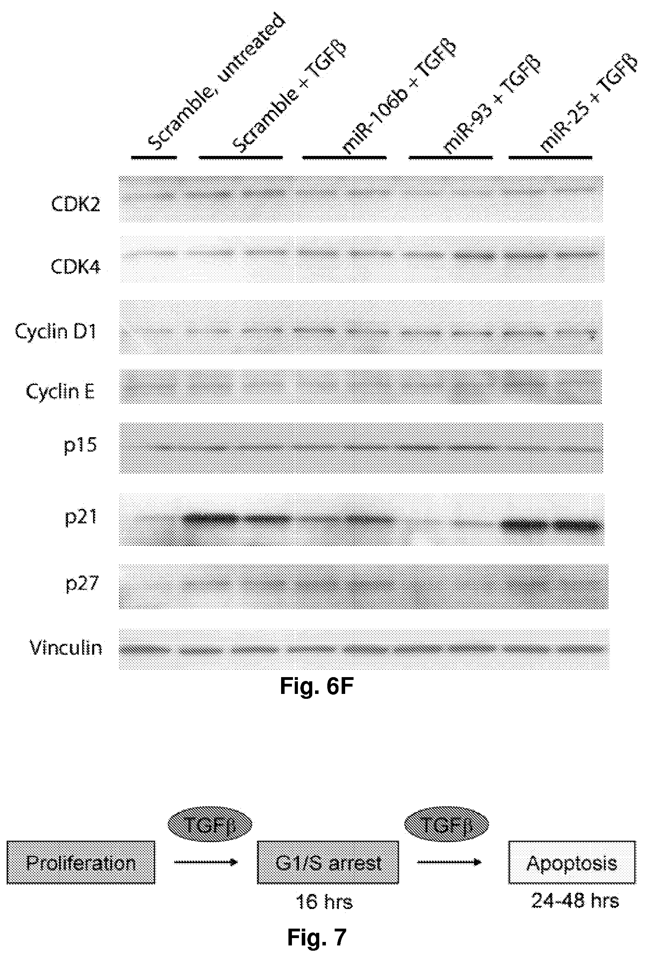

**Figures 6A-6F****:** Inhibition of endogenous miR-106b and miR-93 expression enhances TGF-beta-dependent G1/S cell cycle arrest. **Fig. 6A****:** Analysis of cell cycle in Snu-16 cells treated with TGF-beta upon inhibition of endogenous miRNAs by ASO transfection. p-value was calculated comparing the G1 fraction in ASOs transfected cells vs mock-transfected cells (unpaired t-test) **(****Fig. 6B****)** Dose-response curve of Snu-16 treated with graded doses of TGF-beta ranging from 0.1 to 5.0 ng/ml. Inhibition of endogenous miR-106b or miR-93 by ASO transfection restores sensitivity of Snu-16 cells to TGF-beta doses to which they are otherwise resistant (0.1 - 0.3 ng/ml), as determined by FACS analysis. * indicates p<0.0001 **(****Fig. 6C****).** Analysis of p21 protein and **(****Fig. 6D****)** p21 mRNA expression by Western Blot and qRT-PCR, respectively. Bars indicate RNA expression normalized to U6 +/- SD. The degree of p21 protein upregulation induced by inhibition of endogenous miR-106b and miR-93 is greatly enhanced by the presence of TGF-beta, possibly supported by the increased transcription of p21 mRNA. **(****Fig. 6E****)** Snu-16 cells were transfected with a siRNA against p21 alone or in combination with either miR-106b or miR-93 mimics and treated with 1 ng/ml TGF-beta for 16 hours. While miR-106b lost all of its effect on cell cycle, miR-93 still maintained a residual effect after p21 silencing. This differential response between miR-106b and miR-93 is statistically significant (p=0.0272) **(****Fig. 6F****)** Analysis of expression by Western Blot of various proteins involved in the G1/S checkpoint upon TGF-beta stimulation.

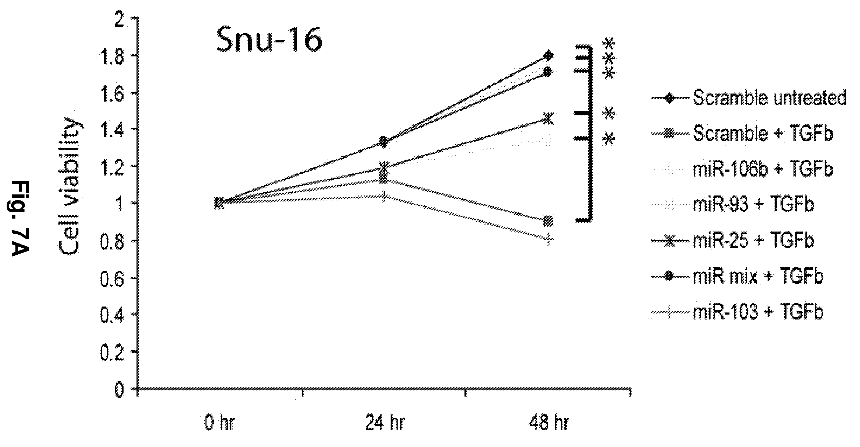

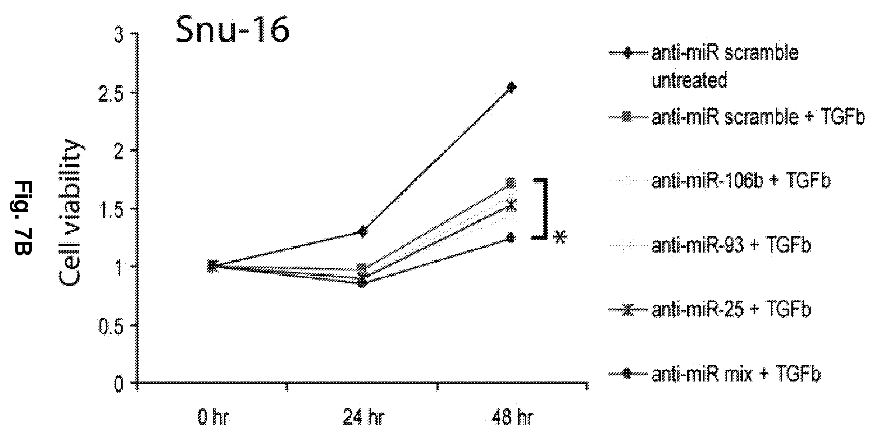

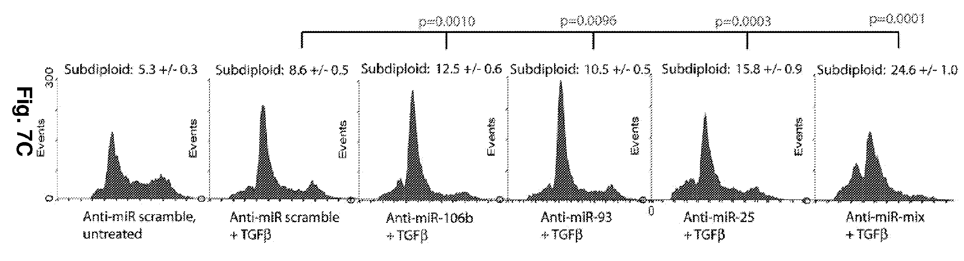

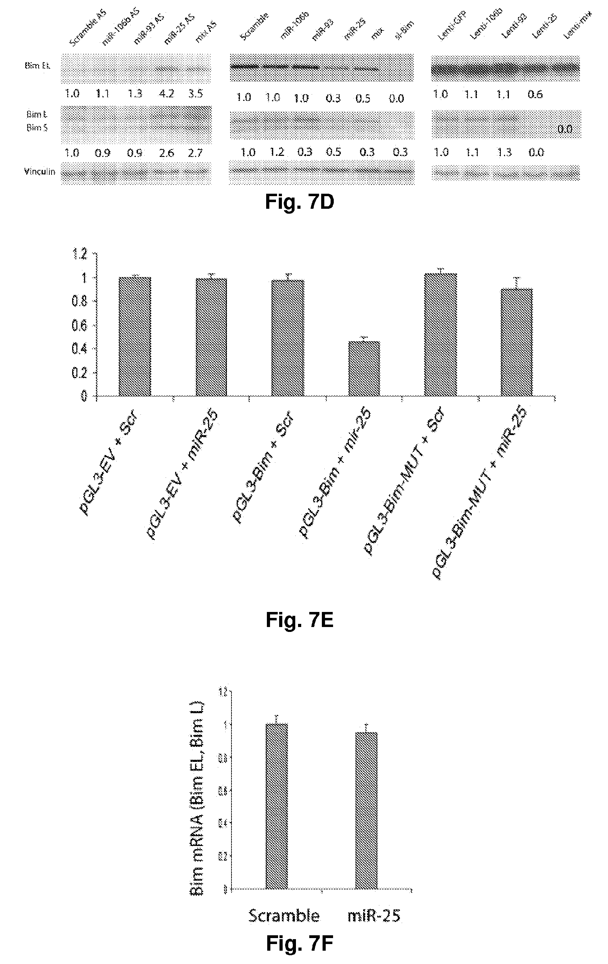

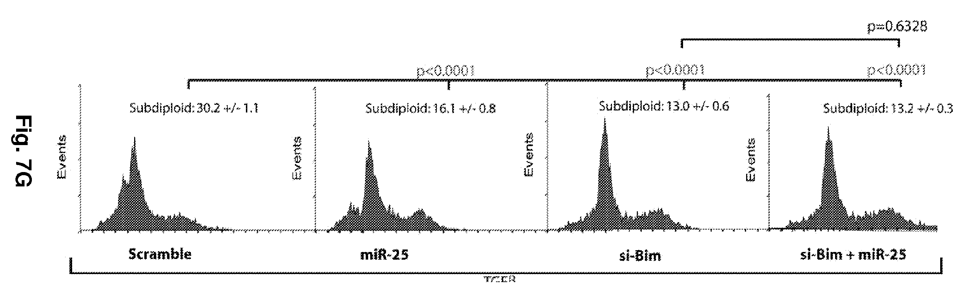

**Figures 7A-7G****:** miR-25 cooperates with miR-106b and miR-93 in preventing the onset of TGF-beta-induced apoptosis CCK-8 viability assay of Snu-16 cells transfected with miRNA mimics. * indicates significant difference (p<0.001) in the number of viable cells upon transfection of miR-106b, miR-93, miR-25 and/or miR-106b-25 and subsequently treated with 1 ng/ml TGF-beta for 48 hours. **(****Fig. 7B****)** Conversely, inhibition of miR-106b, miR-93 and miR-25 cooperatively augments the response to TGF-beta: statistical significance (p<0.001) was reached upon transfection of a mixture of the three ASOs. **(****Fig. 7C****)** Significant loss of viability was confirmed by analysis of subdiploid DNA content. **(****Fig. 7D****)** Bim protein expression in Snu-16 cells at 48 hours post-transfection with either miRNA mimics or ASOs or after lentiviral transduction of the same miRNAs. Same effects on Bim expression were obtained in AGS and MKN-74 cells (data not shown). **(****Fig. 7E****)** Luciferase assay showing decreased luciferase activity in cells cotransfected with pGL3-Bim-3'UTR and miR-25. Deletion of the first 3 bases of miR-25 predicted binding sites, complementary to miRNA seed regions, abrogates this effect (MUT). Bars indicate Firefly luciferase activity normalized to Renilla luciferase activity +/SD. Each reporter plasmid was transfected at least twice (on different days) and each sample was assayed in triplicate. **(****Fig. 7F****)** qRT-PCR analysis showing no difference in Bim mRNA (Taqman probe recognizing the two major isoforms Bim EL and Bim L) in Snu-16 cells transfected with miR-25 oligonucleotide. Bars indicate RNA expression normalized to U6 +/- SD. **(****Fig. 7G****)** FACS analysis of subdiploid DNA content in Snu-16 cells transfected with miR-25 oligonucleotide, si-Bim, both or a scramble oligonucleotide and subsequently treated with 1 ng/ml TGF-betaO. for 24 hours. Statistical analysis as above.
**Figure 8****:** The E2F1/miR-106b-25/p21 pathway. A model summarizing the mechanism of action of miR-106, miR-93 and miR-25 described herein.
**Figure 9A-9D****:** Expression of the miR-106b-25 cluster in gastric cancer. **Fig**. **9A****:** 293T/17 cells were transfected with 100 nM miRNA oligonucleotides (Ambion), as indicated, and assayed for miRNA expression by stem-loop qRT-PCR. MiR-106b, miR-93, miR-25 primers showed high specificity while miR-17-5p and miR-92 primers cross-hybridized with miR-106a and miR-25, respectively. Results were normalized to U6 and converted to the same scale. Expression of mature **(****Fig. 9B****)** and precursor **(****Fig. 9C****)** miRNAs in a set of 10 gastric primary tumors and 10 non-tumor controls, as determined by qRT-PCR. Bars represent relative fold-changes between tumor and non-tumor tissues from the same patient +/- SD. Each sample was analyzed in triplicate and normalized to either RNU49 (mature miRNAs) or CAPN2 (precursor miRNAs and Mcm7): these genes showed the least variability (<0.4 Ct values) among 12 different normalizers tested in these samples. **(****Fig. 9****)** Snu-16 cells were transduced with a lentiviral vector carrying miR-106b, miR-93, miR-25 or the miR-106b-25 cluster and mature miRNA levels were measured after 72 hours by qRT-PCR. Bars indicate RNA expression normalized to U6 +/- SD and converted to the same scale. Transduction efficiency >90% was confirmed by fluorescent microscopy. Similar results were obtained in AGS cells (data not shown).

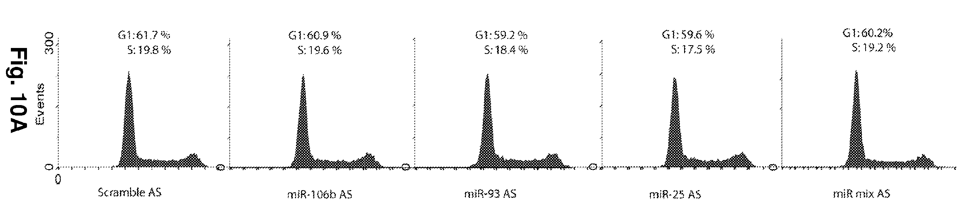

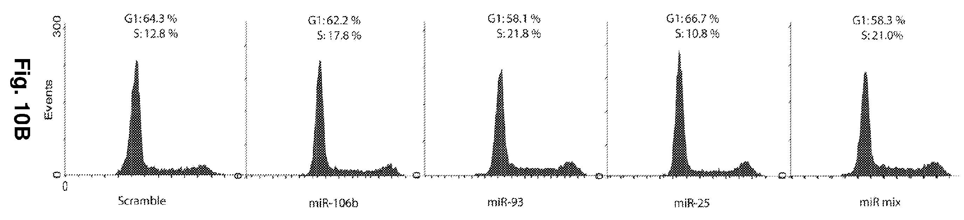

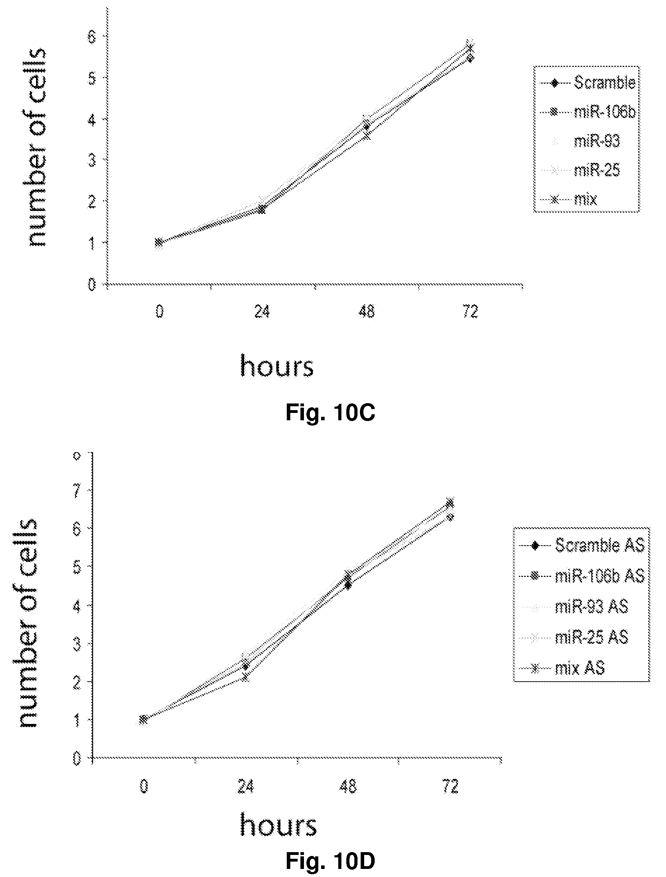

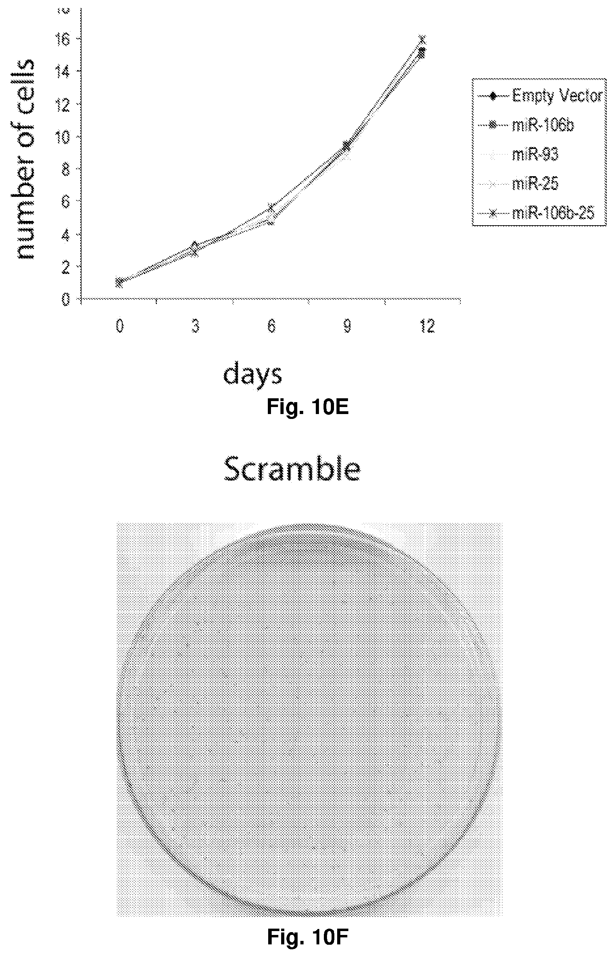

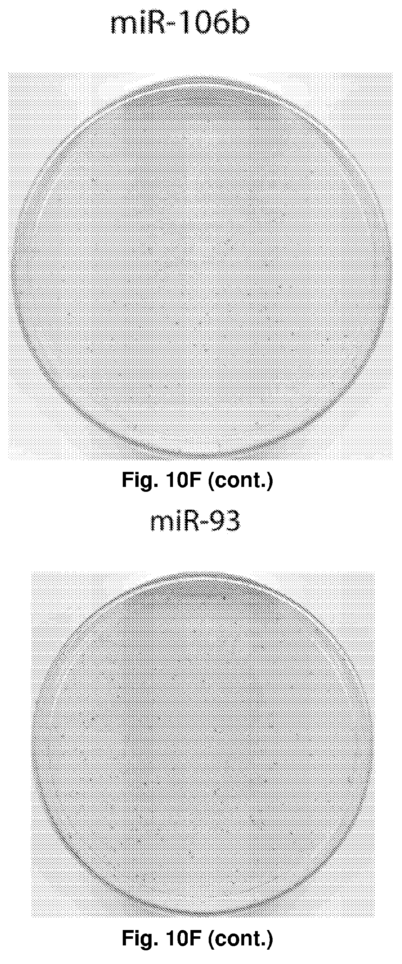

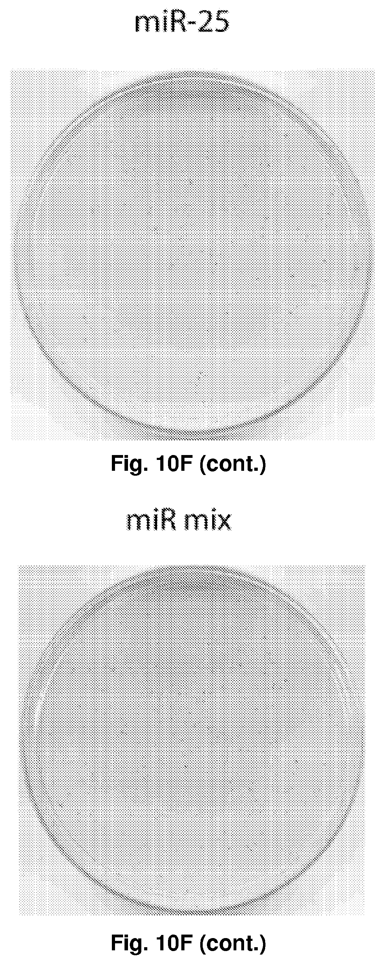

**Figures 10A-10G****:** Proliferation studies in cells with high/low miR-106b-25 (basal conditions). FACS analysis and proliferation curves of Snu-16 **(****Fig. 10A****,** **Fig. 10C**) and AGS **(****Fig. 10B****,** **Fig. 10D****)** cells transfected with miRNA ASOs or mimics, respectively. **(****Fig. 10E****)** Proliferation curves of AGS cells stably transduced with a fluorescent lentiviral vector carrying miR-106b, miR-93, miR-25 or miR-106b-25 precursors under control of a CMV promoter. Infection efficiency > 95% was determined by fluorescent microscopy. **(****Fig. 10F****)** Colony formation assay: AGS cells were transfected with pRetroSuper-Puro constructs encoding miR-106b, miR-93, miR-25 or miR-106b-25 precursors or a scramble sequence and grown in 2 ug/ml puromycin for 14 days. Efficient miRNA expression and processing by all these constructs were assayed by Northern Blot and stem-loop qRT-PCR (data not shown).
**(****Fig. 10G****)** Proliferation curve and (H) FACS analysis of Snu-16 cells in which either p21 or E2F1 were selectively silenced by RNAi. Inhibition of p21 expression produced an effect on cell cycle that was undistinguishable from miR-93.
**Figure 11****:** MKN-74 cell viability assay in the presence of TGF-beta. MKN-74 cells were transfected with the indicated LNA oligonucleotides to silence endogenous miRNA expression and subsequently treated with TGF-beta for 96 hours. Cell viability was determined by CCK-8 assay.
**Figure 12****:** Annexin V assay of miR-25 overexpressing cells. Results shown in **Figure 7G** were confirmed by Annexin V staining.
**Figure 13****:** Table 1: Differentially expressed miRNAs in chronic gastritis VS normal gastric mucosa.
**Figure 14****:** Table 2: Differentially expressed miRNAs in gastric adenocarcinomas VS non-tumor gastric mucosa.
**Figure 15****:** Table 3: MicroRNA expression in human gastric cancer cell lines.
**Figure 16****:** Table 4: Validation of microarray data in paired human primary tumors VS non-tumor controls by qRT-PCR.
**Figure 17****:** Table 5: Mcm7 mRNA and miR-106b-25 expression in 10 paired gastric primary tumors and non-tumor controls.
**Figure 18****:** Table 6: Human genes harboring putative miR-106b, miR-93 and miR-25 binding sites on the same 3' UTR.

### DETAILED DESCRIPTION OF THE PREFERRED EMBODIMENT

Deregulation of E2F1 activity and resistance to TGF-beta are hallmarks of gastric cancer. MicroRNAs (miRNAs) are small non-coding RNAs frequently misregulated in human malignancies.

Here we show that miR-106b-25 cluster, upregulated in a subset of human gastric tumors, is activated by E2F1 in parallel with its host gene Mcm7. In turn, miR-106b and miR-93 regulate E2F1 expression, establishing a miRNA-directed negative feedback loop. Furthermore, upregulation of these miRNAs impairs the TGF-beta tumor suppressor pathway interfering with the expression of CDKN1A (p21Waf1/Cip1) and BCL2L11 (Bim). Together, these results show that miR-106b-25 cluster is involved in E2F1 post-transcriptional regulation and can play a key role in the development of TGF-beta resistance in gastric cancer.

MicroRNAs (miRNAs) are small non-coding RNAs that may regulate the expression of approximately 30% of all human genes, either inhibiting target mRNA translation or inducing its degradation. These genes are abnormally expressed in human malignancies, making their biological importance increasingly apparent. Gastric cancer causes 12% of all cancer-related deaths each year calling for better treatments based on a deeper understanding of the molecular mechanisms underlying the onset of this disease.

Here, we show that overexpression of the miR-106b-25 cluster leads to deregulation of important cancer-related genes, such as the TGF-beta effectors p21Waf1/Cip1 and Bim, disrupting the G1/S checkpoint and conferring resistance to TGF-beta-dependent apoptosis.

We also show that microRNAs (miRNAs) may be involved in gastric tumorigenesis. MiRNAs are non-protein coding genes thought to regulate the expression of up to 30% of human genes, either inhibiting mRNA translation or inducing its degradation (Lewis et al., 2005). Besides a crucial role in cellular differentiation and organism development (Kloosterman and Plasterk, 2006), miRNAs are frequently misregulated in human cancer (Lu et al., 2005; Volinia et al., 2006) and they can act as either potent oncogenes or tumor suppressor genes (Esquela-Kersher et al. 2006).

Here we show that E2F1 regulates miR-106b, miR-93 and miR-25, a cluster of intronic miRNAs hosted in the Mcm7 gene, inducing their accumulation in gastric primary tumors. Conversely, miR-106b and miR-93 control E2F1 expression, establishing a negative feedback loop that may be important in preventing E2F1 self activation and, possibly, apoptosis.

On the other hand, we found that miR-106b, miR-93 and miR-25 overexpression causes a decreased response of gastric cancer cells to TGF-beta interfering with the synthesis of p21 and Bim, the two most downstream effectors of TGF-beta-dependent cell cycle arrest and apoptosis, respectively.

These miRNAs contribute to the onset of TGF-beta resistance in cancer cells and now believed by the inventors herein to represent novel therapeutic targets for the treatment of gastric cancer.

The present invention is further explained in certain of the following Examples, in which all parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the scope thereof, can make various changes and modifications of the invention to adapt it to various usages and conditions.

### EXAMPLE I

### Deregulation of miRNA expression in human gastric cancer

Most gastric adenocarcinomas arise in the context of a chronic inflammatory background, frequently associated with Helicobacter Pylori (HP) infection (Uemura et al., 2001). Nevertheless, the molecular mechanisms responsible for HP oncogenicity are poorly understood, although Th1 immune response seems to be critical in the development of preneoplastic lesions such as gastric atrophy and intestinal metaplasia (Houghton et al., 2002; Fox et al., 2000).

In the search of miRNAs potentially involved in gastric tumorigenesis, we analyzed global miRNA expression in 20 gastric primary tumors of the intestinal-type, each one paired with adjacent nontumor gastric tissue from the same patient, and 6 gastric cancer cell lines using a custom miRNA microarray. To identify specific alterations associated with inflammation and/or preneoplastic lesions, we first compared non-tumor tissues with histological signs of chronic gastritis (n=13) versus otherwise normal mucosa (n=7). Seven miRNAs were associated with chronic inflammation by unpaired Significance Analysis of Microarrays (SAM), including miR-155 that is known to predispose to cancer (Costinean et al., 2006) and to play a major role in the regulation of immune response (Rodriguez et al., 2007; Thai et al., 2007) **(****Figure 1A****,** **Figure 13** **- Table 1).**

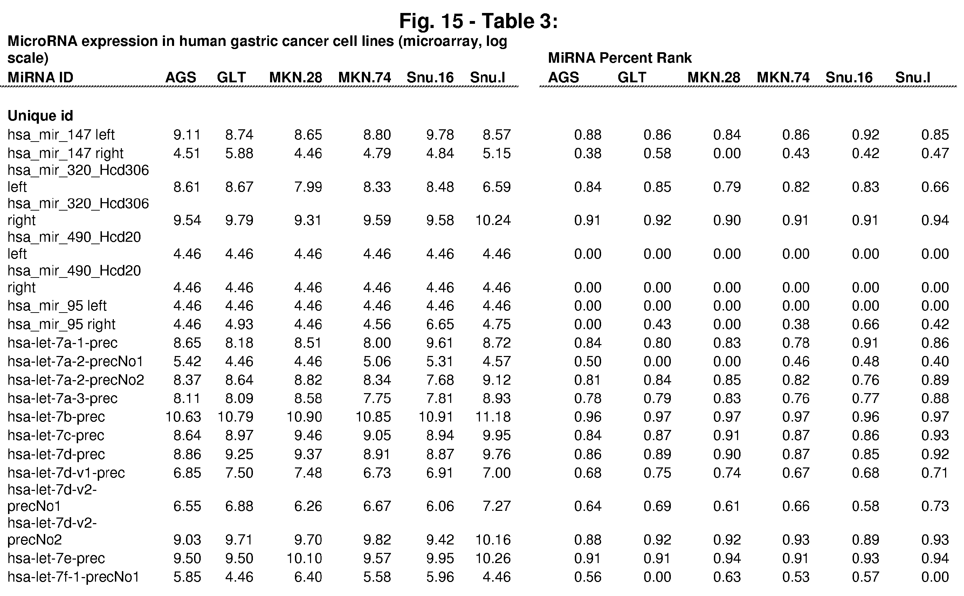

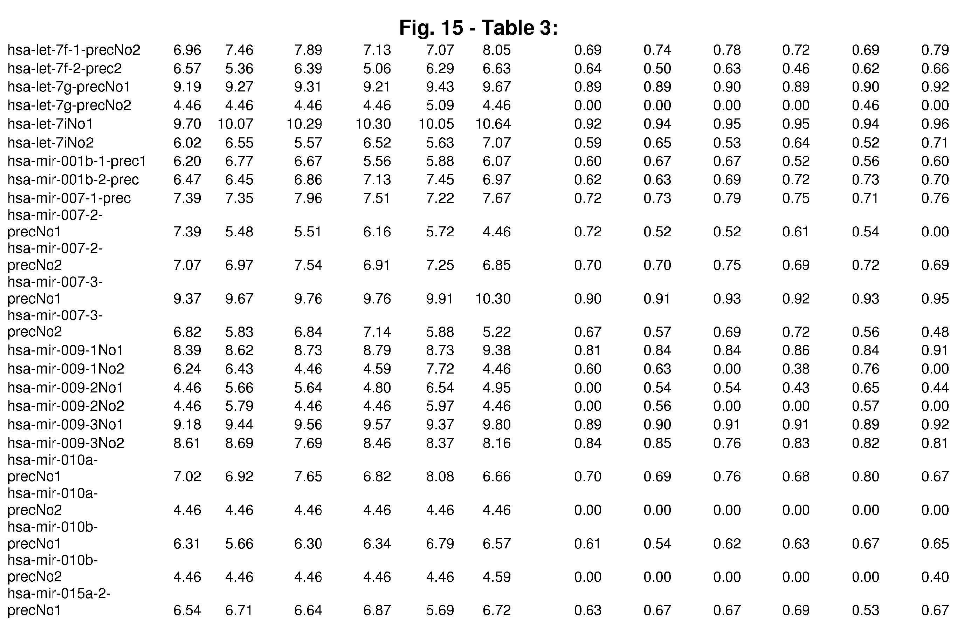

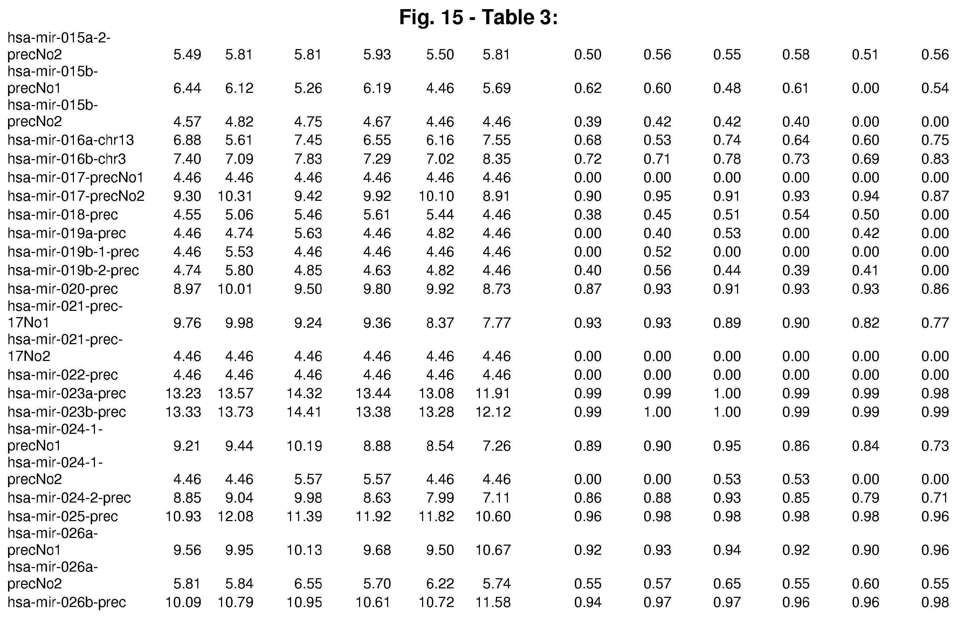

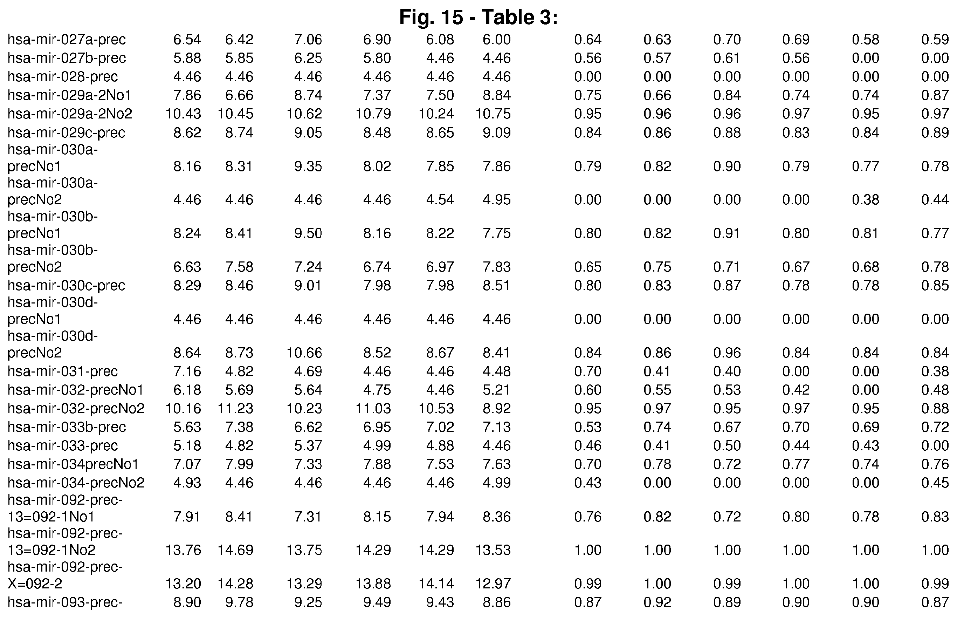

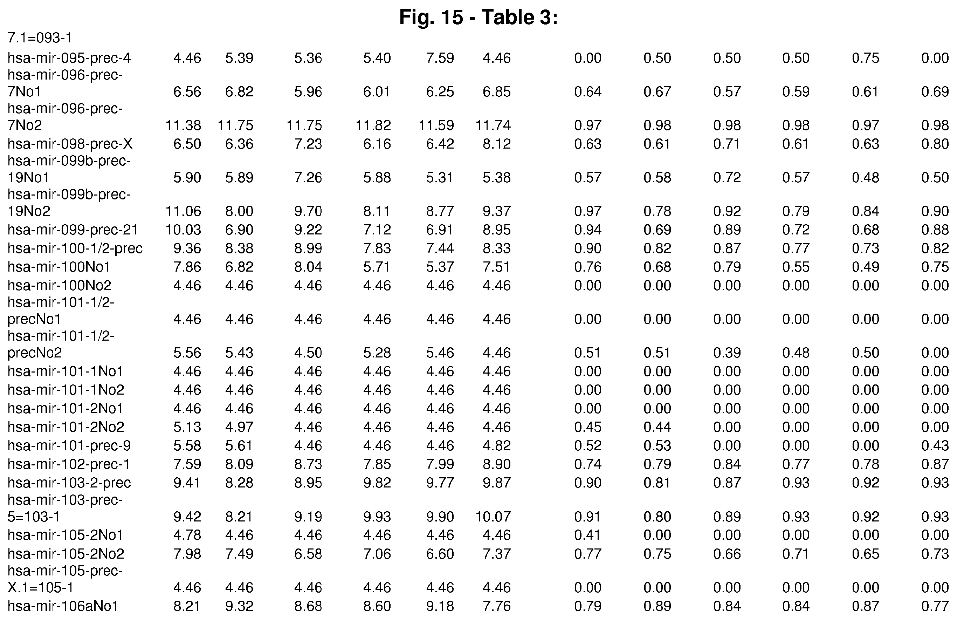

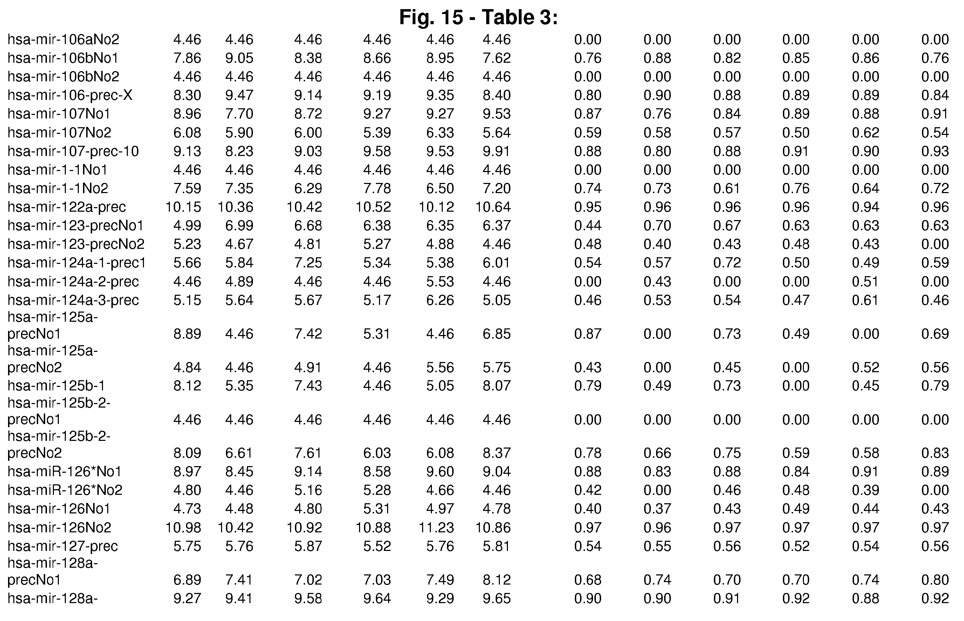

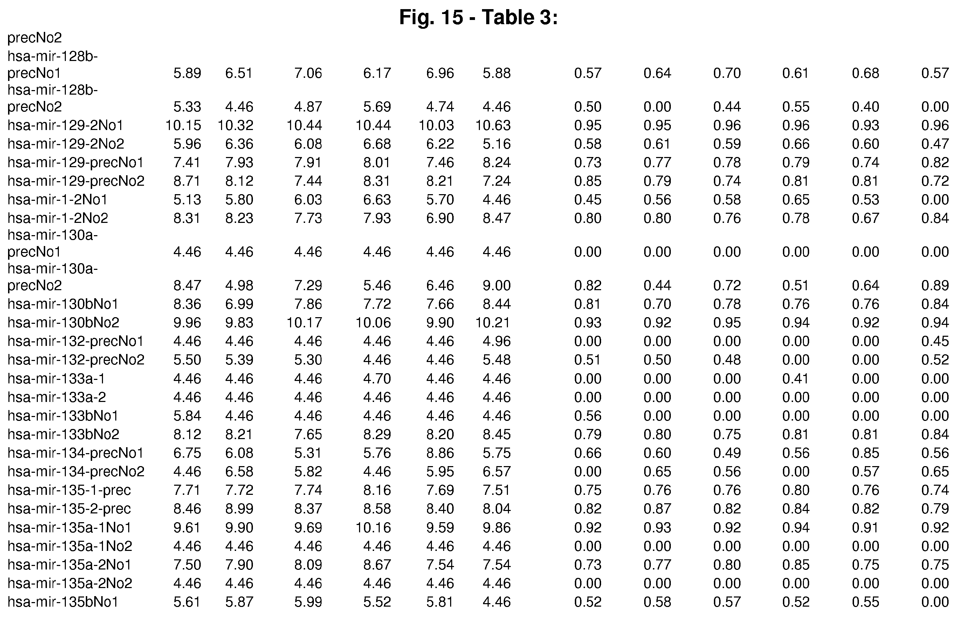

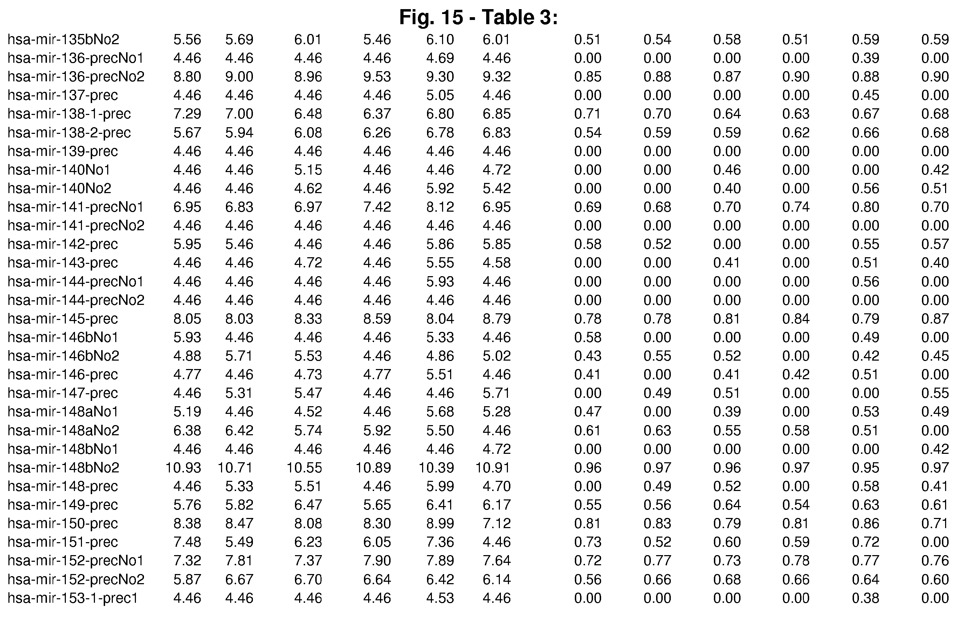

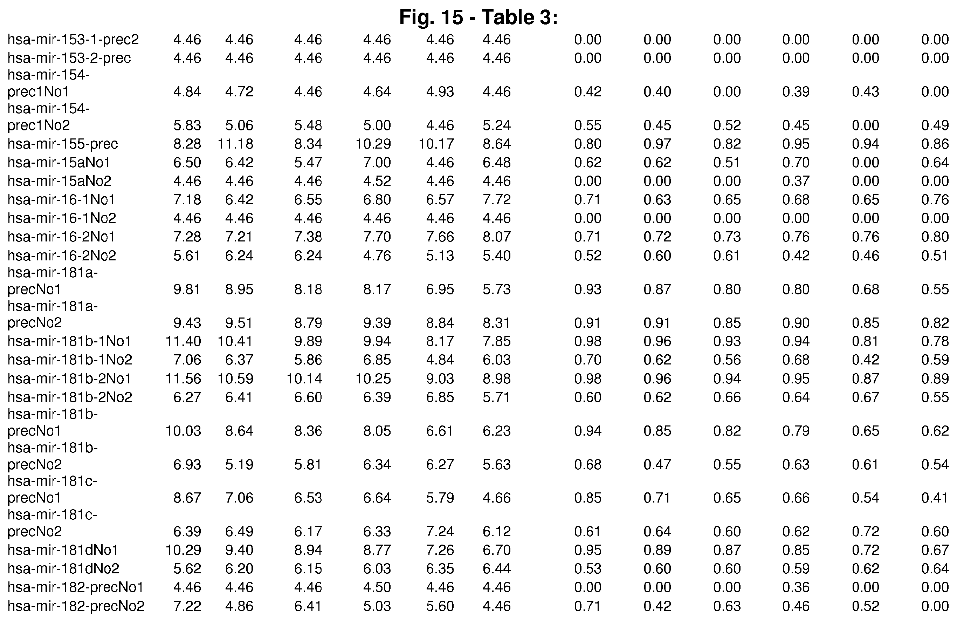

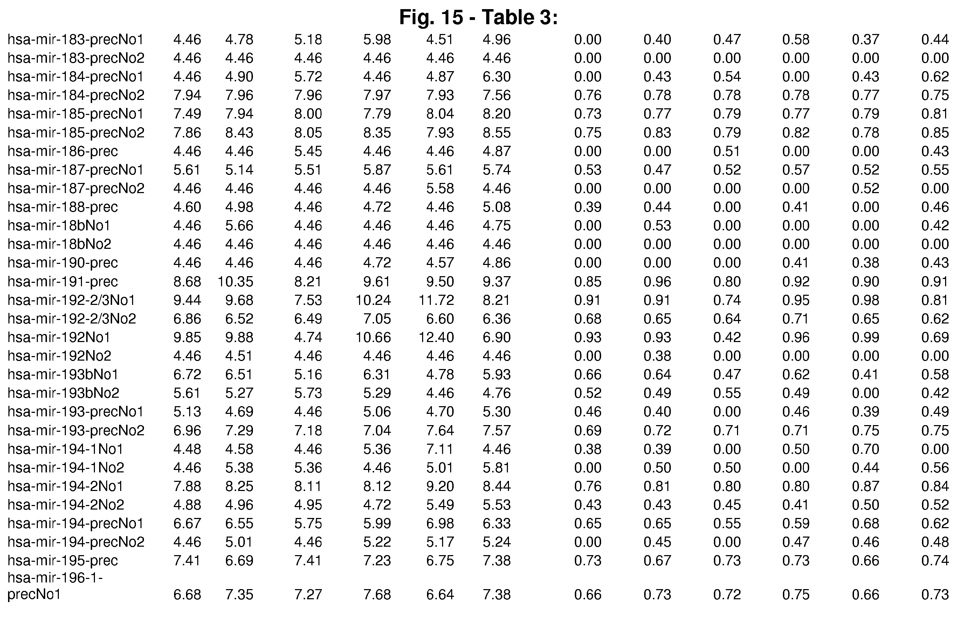

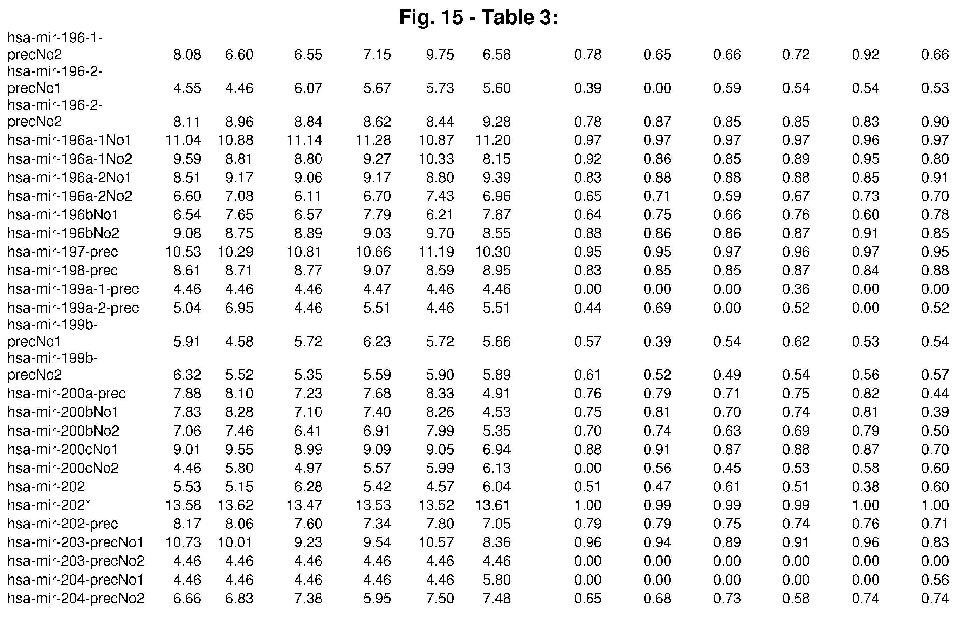

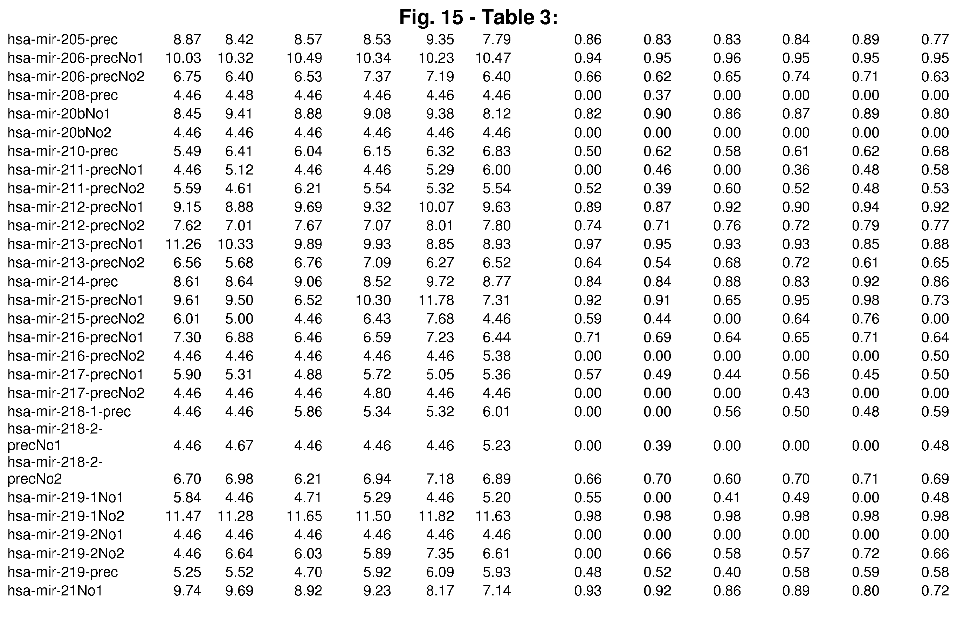

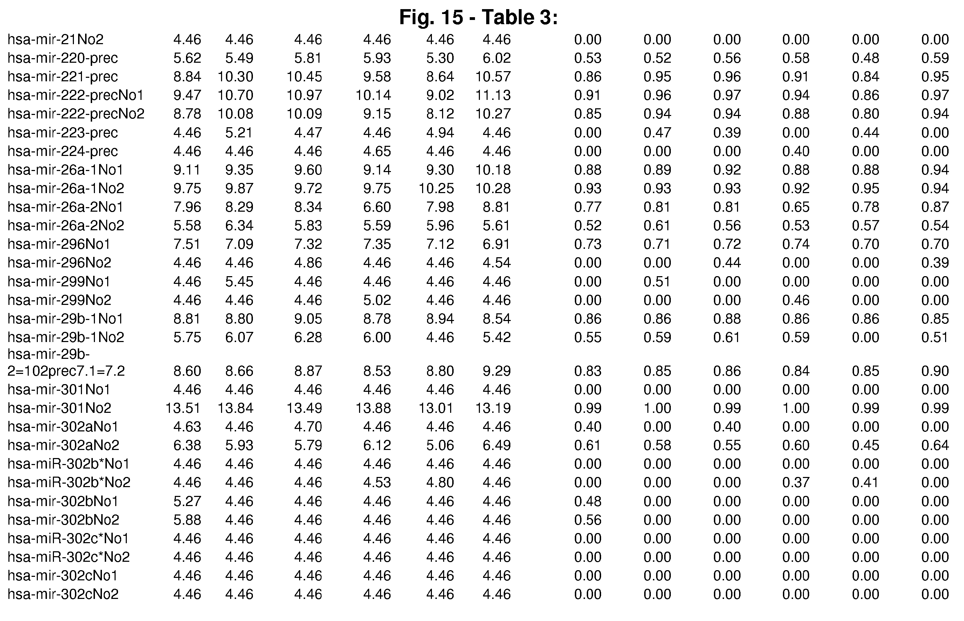

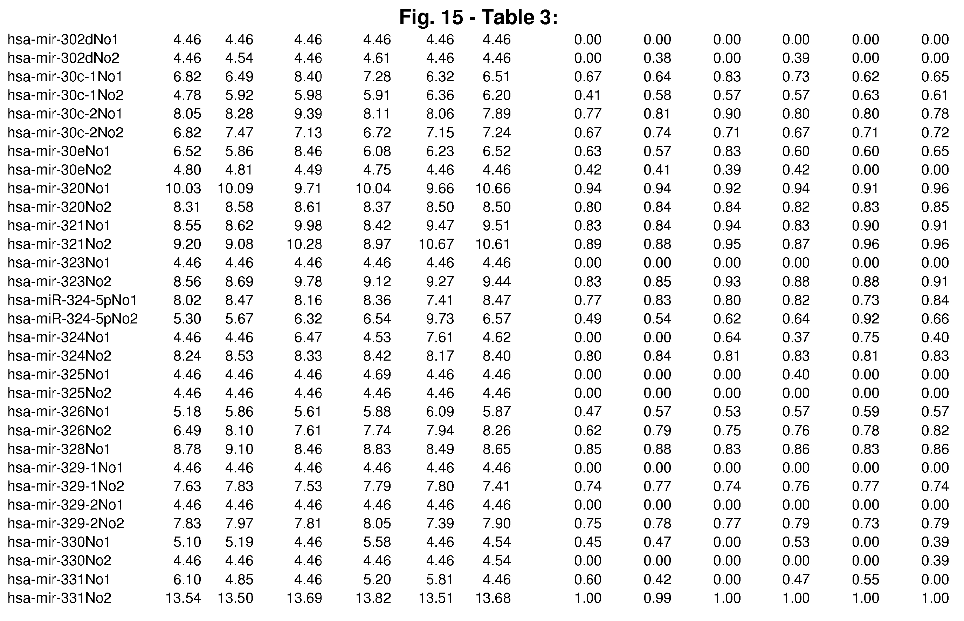

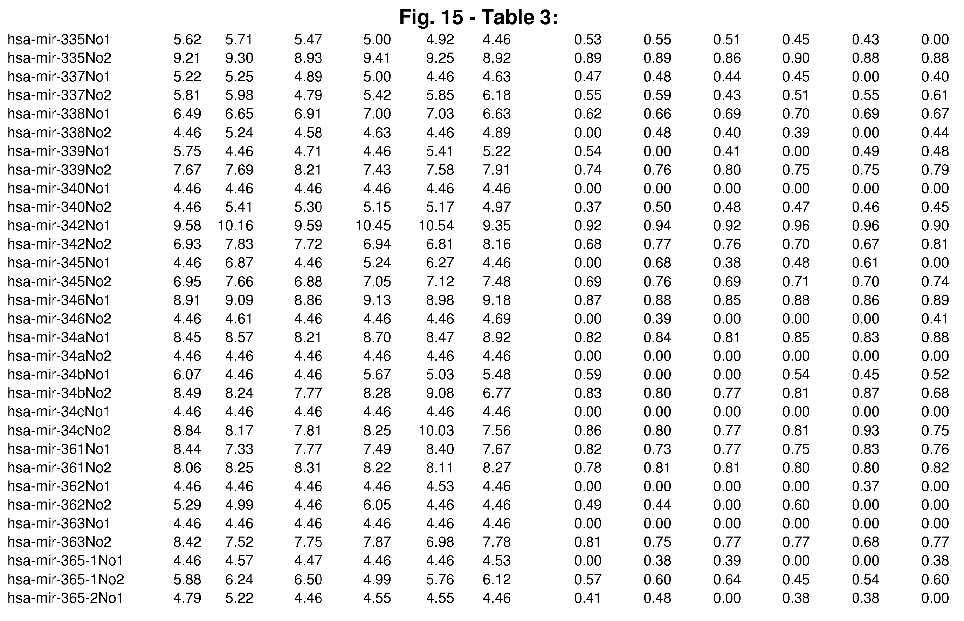

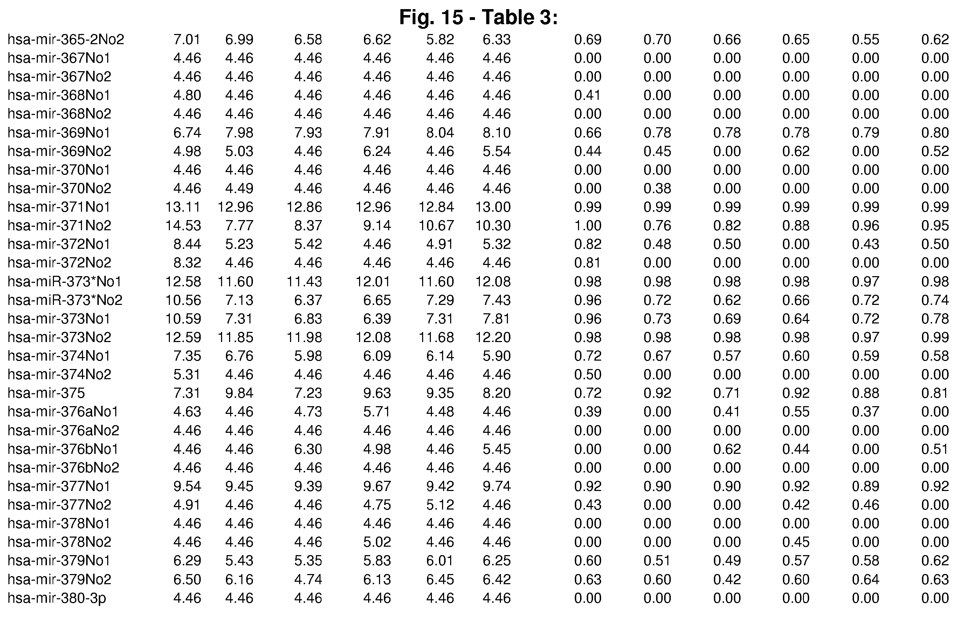

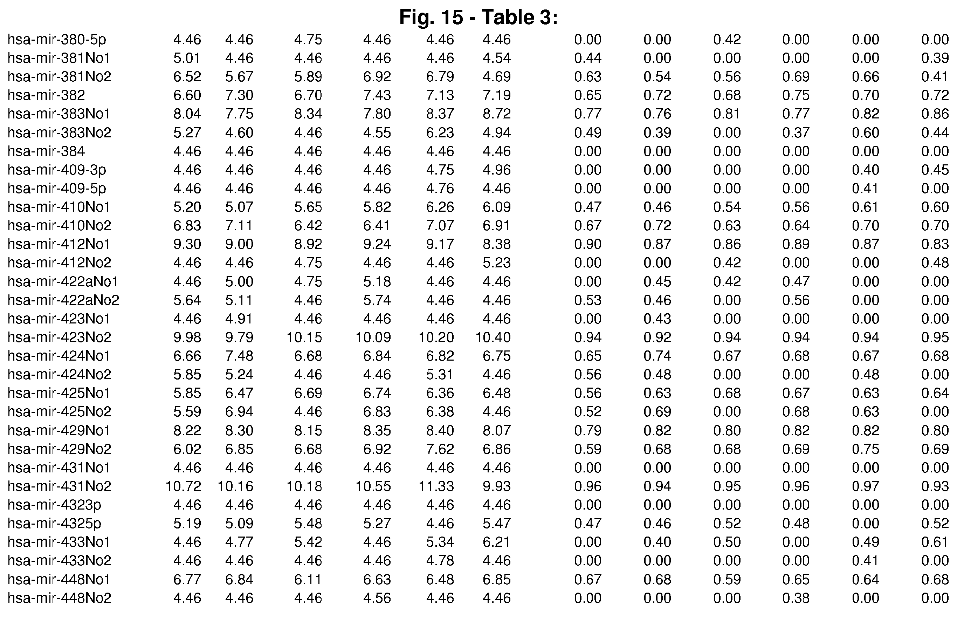

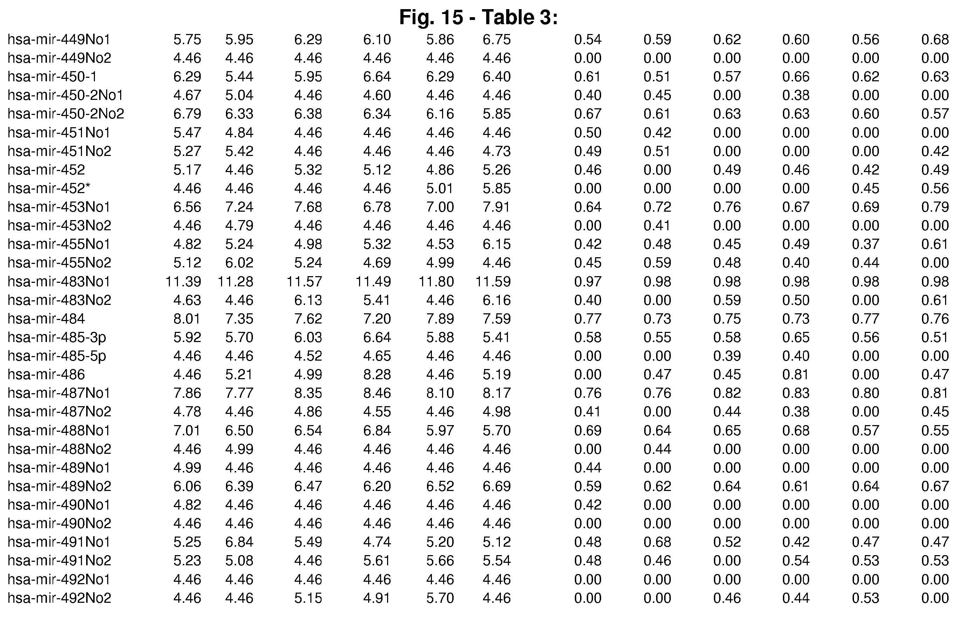

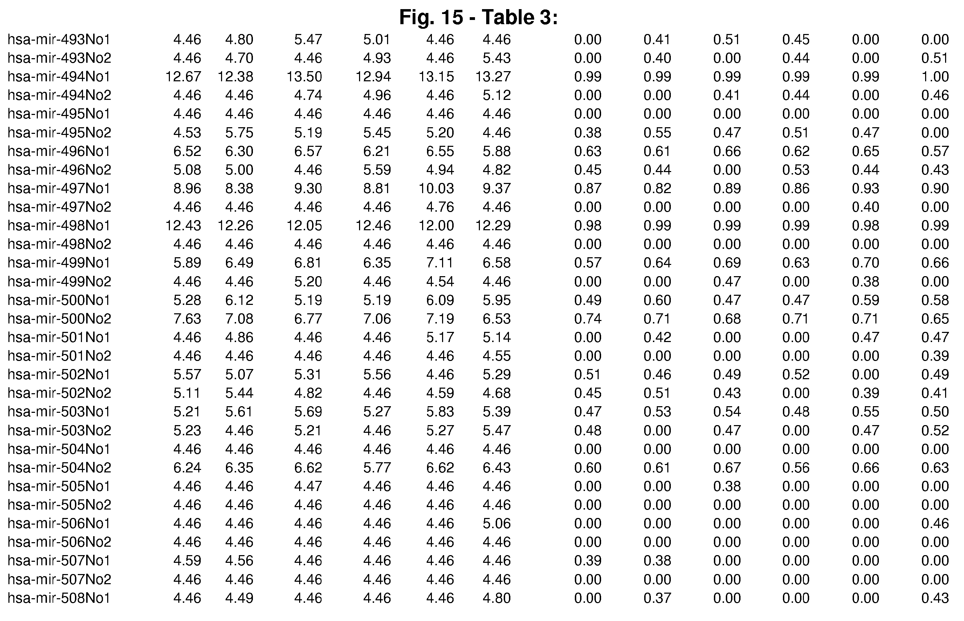

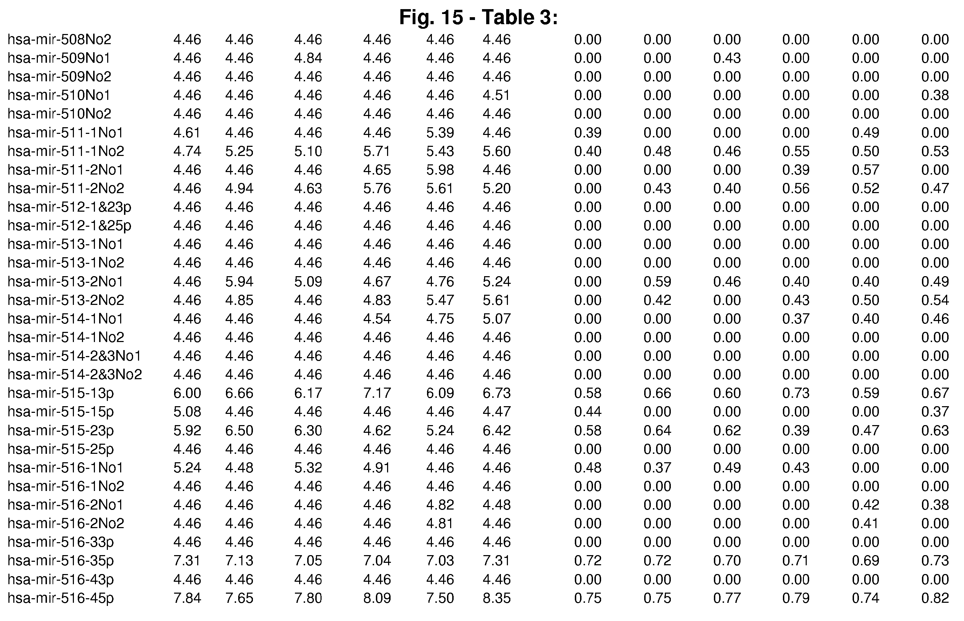

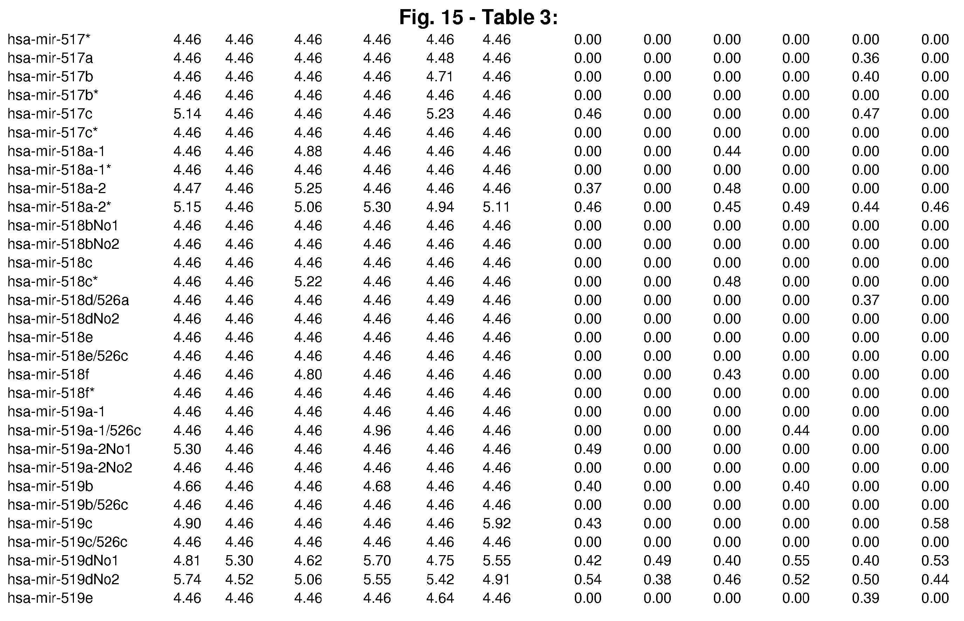

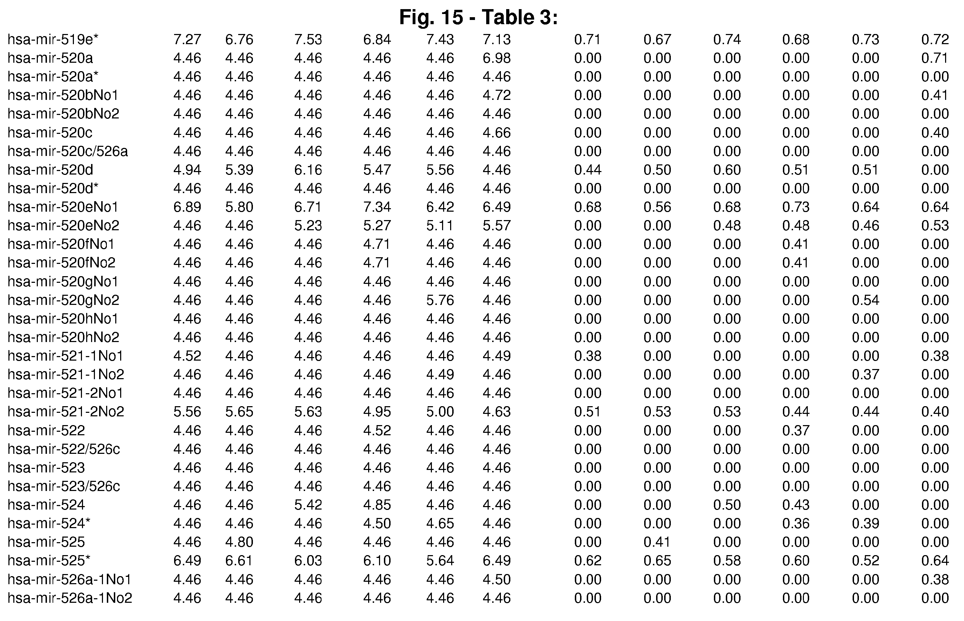

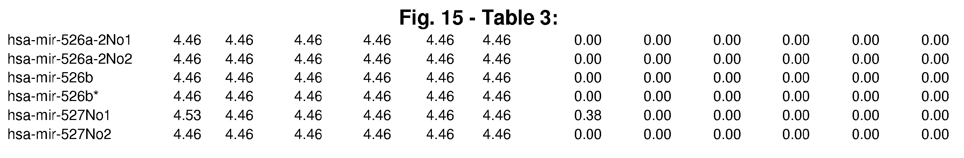

We then examined the miRNA expression profile of gastric primary tumors and cancer cell lines: a total of 14 miRNAs exhibited a 2-fold or greater median overexpression in primary tumors compared to non-tumor controls by paired SAM **(****Figure 1B****,** **Figure 14** **- Table 2).** Of these, 13 out of 14 ranked above the 80th percentile in all gastric cancer cell lines in terms of expression, except for miR-223 that was not expressed **(****Figure 15** **- Table 3).** Only 5 miRNAs were downregulated in cancer **(****Figure 1B****,** **Figure 14** **- Table 2).** Microarray data were confirmed by stem-loop qRT-PCR for 9 out of 10 tested miRNAs **(****Figure 16** **- Table 4).** Among the misregulated miRNAs, miR-21, miR-223, miR-25 and miR-17-5p showed the highest overexpression in tumors, with 4.5, 4.2, 3.7 and 3.7 median fold-changes, respectively.

These results indicate that specific modifications in the miRNA expression pattern are characteristic of human gastric cancer since the earliest steps of tumorigenesis and involve miRNAs with known oncogenic properties, such as miR-21 (Meng et al., 2006) and miR-17-5p (He et al., 2005).

*miR-106b-25 cluster is overexpressed in gastric cancer*

Among the overexpressed miRNAs, miR-25 were discovered to be especially useful an attractive candidate for playing a role in gastric tumorigenesis. In fact, this was the 3rd most strongly upregulated miRNA in primary gastric tumors (median fold-change: 3.7; range 1.0 - 26.8) and ranked among the most highly expressed miRNAs in all human gastric cancer cell lines (above 97th percentile). miR-106b (median fold-change: 2.0; range 1.0 - 6.5) and miR-93 (median fold-change: 2.3; range 1.0 - 7.7) were also upregulated in primary tumors and highly expressed in all gastric cancer cell lines (above 82nd and 89th percentile, respectively).

These three miRNAs (hereafter miR-106b-25) are clustered in the intron 13 of Mcm7 on chromosome 7q22 and actively cotranscribed in the context of Mcm7 primary RNA transcript (Kim et al., 2007 and Figure 1C-E). Several studies reported the amplification of this region in gastric tumors (Weiss et al., 2004; Peng et al., 2003; Takada et al., 2005). However, we could not detect any amplifications of the miR-106b-25 locus in our samples (data not shown), implying that other mechanisms must contribute to miR-106b-25 overexpression in gastric cancer.

Mcm7 plays a pivotal role in the G1/S phase transition, orchestrating the correct assembly of replication forks on chromosomal DNA and ensuring that all the genome is replicated once and not more than once at each cell cycle (Blow and Hodgson, 2002). As overexpression of Mcm7 has been associated with bad prognosis in prostate and endometrial cancer (Ren et al., 2006; Li et al., 2005) we hypothesized that Mcm7 oncogenicity may be linked, at least in part, to overexpression of the hosted miRNAs. Moreover, the miR-106b-25 cluster shares a high degree of homology with the miR-17-92 cluster (Figure 1C), which appears to have an oncogenic role (He et al., 2005; O'Donnell et al., 2005; Dews et al., 2006).

We then investigated the miR-106b-25 cluster. We first determined the specificity of stem-loop qRT-PCR. Primers for miR-106b, miR-93 and miR-25 were highly specific while miR-17-5p and miR-92 probes cross-hybridized with miR-106a and miR-25, respectively **(****Figure 9A**). Next, we used stem-loop qRT-PCR to assay the expression of mature miRNA species in an independent set of ten gastric primary tumors paired with non-tumor gastric mucosa from the same patient.

Mature miR-106b, miR-93 and miR-25 were overexpressed in 6/10, 6/10 and 5/10 of these tumors, respectively, although there was not reciprocal correlation in their level of expression **(****Figure 9B****).**

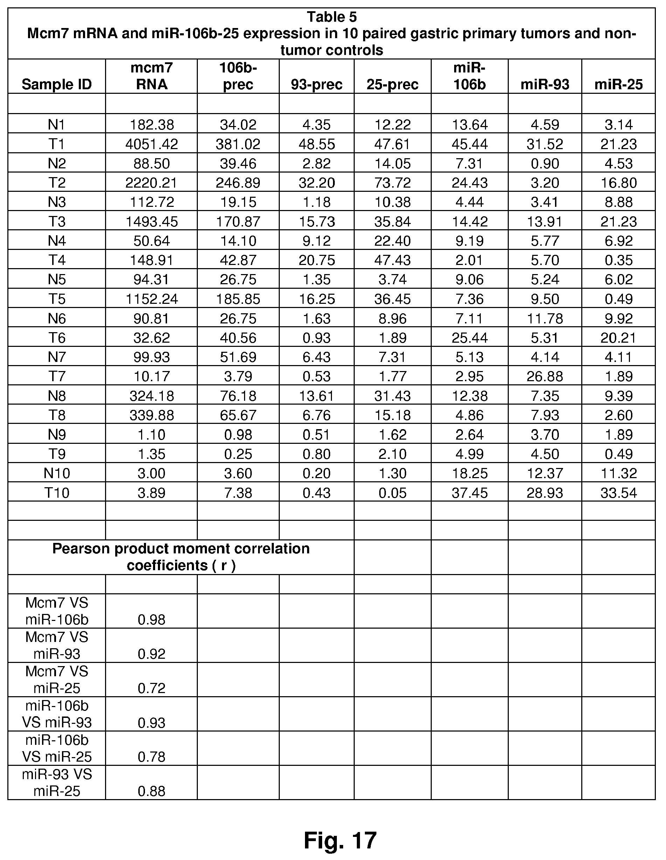

We examined miRNA precursor levels in the same tumors by conventional qRT-PCR **(****Figure 9C****)** and we found miR-106b, miR-93 and miR-25 precursor species to be concordantly expressed in the tumors [r(106b/93)=0.93; r(106b/25)=0.78; r(93/25)=0.88, **Figure 17** **- Table 5].**

Of the 5 tumors overexpressing miR-106b-25 precursors, 3 tumors also expressed high levels of mature miR-106b, miR-93 and miR-25 whereas the remaining tumors displayed variable expression of each mature miRNA, showing an additional level of post-transcriptional regulation controlling individual miRNAs.

Mcm7 mRNA was also overexpressed in 5/10 tumors, showing an almost perfect correlation with miR-106b, miR-93 and miR-25 precursor levels (r=0.98, 0.92, 0.72, respectively, **Figure 9C** and **Figure 17** **- Table 5).**

These data show that miR-106b-25 precursors are specifically overexpressed in a subset of gastric primary tumors in parallel with Mcm7 mRNA. Although we cannot exclude the possibility of a miR-106b-25 independent promoter, our results show that miR-106b-25 transcription in gastric tumors is driven by its host gene Mcm7. Moreover, a post-transcriptional mechanism also plays a major role in determining the levels of mature miR-106b-25, as recently proposed for other miRNAs (Thomson et al., 2006).

### A negative feedback loop controls E2F1 and miR-106b-25 expression.

E2F1 is a transcription factor that transactivates a variety of genes involved in chromosomal DNA replication (Johnson and DeGregori, 2006), including Mcm7 (Suzuki et al., 1998; Arata et al., 2000). The inventors herein now believe that miR-106b-25 transcription may be similarly regulated by E2F1. To test, we first determined whether endogenous fluctuations in E2F1 protein levels corresponded to similar changes in Mcm7 and miR-106b-25 expression. Interestingly, AGS gastric cancer cells, arrested in mitosis by nocodazole treatment for 12 hours did not express E2F1 protein and showed reduction in Mcm7 transcript (2-fold) and miR-106b, miR-93 and miR-25 precursors (4.0, 5.2 and 12.0-fold, respectively), compared to exponentially growing cells. As cells were released and reentered the G1 phase, E2F1 expression paralleled Mcm7, miR-106b, miR-93 and miR-25 precursor RNA reaccumulation. **(Figures 2A-C).**

This process was directly associated with E2F1 expression because its specific overexpression by adenoviral transduction **(****Figure 2D****)** or silencing by RNA interference **(****Figure 2E****)** also induced consistent changes in miR-106b-25 precursor levels. E2F1 loss of function impacted the expression of mature miRNAs after 72 hours, as well **(****Figure 2F****).**

To further validate the data *in vivo*, we analyzed E2F1 protein expression in 10 primary gastric tumors by Western Blot and found a positive correlation between E2F1 protein and Mcm7/miR-106b-25 precursor expression **(****Figure 2G****).** In fact, 4 out of 5 tumors overexpressing E2F1 displayed higher levels of Mcm7 and miR-106b-25 precursors **(****Figure 9C****).** Of these, 3 tumors also overexpressed mature miR-106b, miR-93 and miR-25 **(****Figure 9B****).** However, one tumor showed Mcm7 and miR-106b-25 precursors upregulation without detectable levels of E2F1, showing that other transcription factors are also involved in the regulation of miR-106b-25.

These results indicate that E2F1 regulates miR-106b-25 expression in parallel with Mcm7, showing that overexpression of these miRNAs in gastric cancer is due, at least in part, to E2F1 upregulation.

Recently, miR-17-5p has been proposed as a novel post-transcriptional regulator of E2F1 (O'Donnell et al., 2005). Given the similarity between miR-17-5p, miR-106b and miR-93 sequences, we explored the possibility that also miR-106b and miR-93 may participate in the regulation of E2F1 expression. Because these miRNAs were diffusely expressed in a panel of 12 gastric cancer cell lines analyzed by qRT-PCR **(****Figure 3A****)** we adopted a loss of function approach to antagonize miR-106b-25. Transfection of LNA antisense oligonucleotides (ASOs) against miR-106b and miR-93 induced an accumulation of E2F1 protein in Snu-16 cells indicating that endogenous levels of these miRNAs control its expression **(****Figure 3B****).**

Also, overexpression of these miRNAs by either oligonucleotide transfection or lentiviral transduction **(****Figure 9D****)** clearly decreased E2F1 protein levels in Snu-16 and AGS gastric cancer cell lines **(****Figure 3C,** and **Figure 3D****)** and inhibited the expression of a reporter vector containing E2F1 3'UTR. Mutation of the predicted miRNA binding sites in the reporter vector abrogated this effect indicating that miR-106b and miR-93 directly interact with E2F1 3'UTR **(****Figure 3E****).** However, E2F1 mRNA decreased by 2-fold upon miR-106b and miR-93 transfection, possibly because of partial mRNA degradation or downmodulation of E2F1 transcriptional activators **(****Figure 3F****).**

It has been argued that miR-17-5p may secondarily inhibit E2F1 expression by suppressing AIB-1 protein that in fact activates E2F1 transcription and is also a miR-17-5p target (Hossain et al., 2006). While it is very reasonable that miRNAs act on different targets within the same pathway, we analyzed AIB-1 protein levels in AGS and Snu-16 cells and we found a slight decrease or no difference at all in cells transfected with either miR-106b or miR-93, respectively, showing that AIB-1 is a bona fide low affinity target of miR-106b that may only partially contribute to E2F1 downregulation **(****Figure 3C****).**

Together these results show that E2F1 regulates miR-106b-25 expression but is also a target of miR-106b and miR-93, establishing a negative feedback loop in gastric cancer cells. Because E2F1 is known to self-activate its own promoter through a positive feedback loop these miRNAs may control the rate of E2F1 protein synthesis preventing its excessive accumulation, as recently proposed for homolog miR-17-5p and miR-20a (Sylvestre et al., 2007; Woods et al., 2007).

### miR-106b and miR-93 impair TGF-beta-indatced cell cycle arrest.

These results show that miR-106b-25 transcription is promptly induced by E2F1 as cells exit mitosis and re-enter the G1 phase. On this basis, we hypothesized a possible role for miR-106b-25 in repressing G0/G1 associated activities, ideally cooperating with E2F1. So, we interrogated TargetScan database looking for genes known to be negatively regulated by E2F1 and we identified CDKN1A (p21) as a putative target of miR-106b and miR-93. This gene, frequently dysfunctional in human cancer, is a key inhibitor of the cell cycle (Mattioli et al, 2007). Intriguingly, we confirmed that miR-106b and miR-93 endogenously expressed in Snu-16 cells post-transcriptionally regulate p21. In fact, their inhibition by ASOs enhanced the expression of p21 protein **(****Figure 4A****).** Conversely, upregulation of miR-106b and miR-93 achieved by either oligonucleotide transfection **(****Figure 4B****)** or lentiviral transduction **(****Figure 4C****)** repressed p21 protein expression without significant changes in p21 mRNA levels **(****Figure 4D****).** Moreover, miR-106b and miR-93 mimics inhibited the expression of a reporter vector containing p21 3'UTR while mutation of the predicted miRNA binding site abrogated this effect **(****Figure 4E****).**

Given the importance of p21 in the regulation of cell cycle, we decided to address the role of miR106b-25 in controlling the proliferation of gastric cancer cells. Unexpectedly, loss of miR-106b, miR-93 and/or miR-25 function induced by ASOs transfection did not produce any significant alterations in the cell cycle and proliferation of Snu-16 cells **(****Figure 10A** and **Figure 10C****).** Similarly, overexpression of the three miRNAs by either oligonucleotide transfection or lentiviral transduction did not significantly modify the proliferation rate and colony formation efficiency of AGS cells, although we noticed limited but reproducible cell cycle perturbations upon miR-93 overexpression (+8% of cells in S phase, **Figures 10B****,** **10D** and **10E****).**

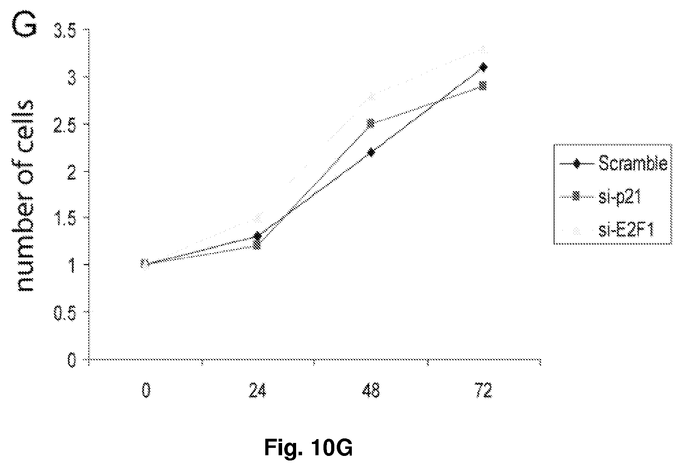

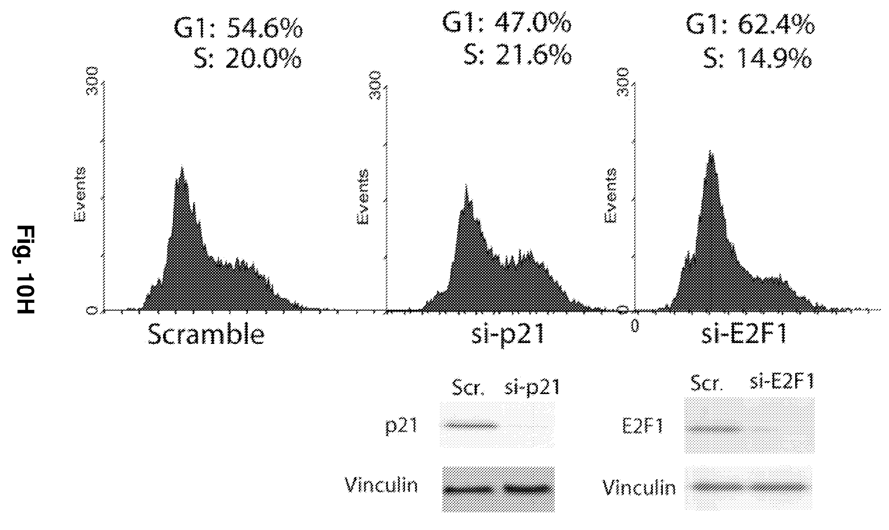

We obtained similar results using GTL-16 and MKN-74 gastric cancer cell lines (data not shown) indicating that miR-106b-25 function is not essential for the survival and the proliferation of gastric cancer cells in vitro. However, specific silencing of either p21 or E2F1 by RNAi produced no significant alterations in the proliferation as well **(****Figures 10G** and **10H****),** confirming that these cancer cell lines are not responsive to p21 basal levels and can well compensate for the loss of E2F1 expression.

We then addressed the role of miR-106b-25 in the presence of TGF-beta: this cytokine, by inducing the expression of p21 and other antiproliferative molecules, ensures timely coordinated cell cycle arrest and apoptosis of mature cells in the gastrointestinal tract, thus controlling the physiological turnover of epithelial cells (van den Brink and Offerhaus, 2007). Impairment of this crucial tumor suppressor pathway is a hallmark of gastric cancer (Ju et al., 2003; Park et al., 1994). However, Snu-16 cells are among the few gastric cancer cell lines still responding to relatively high doses of TGF-beta *in vitro*, undergoing G1/S arrest and subsequent massive apoptosis (Ohgushi et al., 2005 and **Figure 5A**). Nevertheless, cell viability decreases after 24 hours, this opening a window to study early molecular changes associated with TGF-beta.

Interestingly, stimulation with TGF-beta induced marked downregulation of E2F1 protein, Mcm7 mRNA and miR-106b-25 precursors after 16 hours, when cells physiologically undergo G1/S arrest, suggesting that downmodulation of these miRNAs is part of the physiological response to TGF-beta **(****Figures 5B** and **5C****).** To establish the importance of this process, we counteracted miR-106b-25 downregulation by introducing miR-106b, miR-93 and/or miR-25 mimics in Snu-16 cells in the presence of TGF-beta. Notably, overexpression of miR-93 completely abrogated TGF-beta-induced cell cycle arrest while miR-106b partially decreased it (p<0.0002), consistent with the degree of p21 downregulation induced by these miRNAs **(****Figure 5D****).**

Conversely, antagonizing endogenous miR-106b and miR-93 expression by ASOs significantly increased the number of Snu-16 cells undergoing TGF-beta-dependent cell cycle arrest (p<0.0013) and restored sensitivity to suboptimal doses of TGF-beta (p<0.0001) to which these cells are otherwise resistant **(****Figures 6A** and **6B****).**

Accordingly, the degree of p21 upregulation achieved by inhibiting endogenous miR106b and miR-93 in the presence of TGF-beta **(****Figure 6C****)** was double than in basal conditions **(****Figure 4A**), probably supported by the active transcription of p21 mRNA **(****Figure 6D**).

To establish the role of p21 in inducing the phenotype associated with miR-106b and miR-93 gain/loss of function, we specifically silenced p21 by RNAi (si-p21) in Snu-16 cells treated with TGF-beta. This recapitulated almost in full the effect of miR-106b and miR-93 overexpression on cell cycle distribution **(****Figure 5D****)** whereas cotransfection of si-p21 with miR-106b and miR-93 dramatically reduced the effect of these miRNAs on TGF-beta-induced cell cycle arrest, suggesting that p21 is a primary target in this biological context **(****Figure 6E****).** However, a small but statistically significant effect on TGF-beta-dependent cell cycle arrest by miR-93 was still observable in the absence of p21 (p=0.0272), implying that other direct or indirect targets cooperate with p21. Analysis of expression for genes involved in the G1/S checkpoint point at p27 as a possible indirect target of miR-93 **(****Figure 6F****).**

These data show that miR-106b and miR-93 interfere with TGF-beta-induced cell cycle arrest mainly inhibiting the expression of p21 at the post-transcriptional level. However, p21-independent pathways may be also involved in delivering the complete effect of miR-93 on cell cycle control.

### miR-25 cooperates with miR-106b and miR-93 in preventing the onset of TGF-beta-induced apoptosis.

These results show a role for miR-106b and miR-93 in modulating the cell cycle arrest in the early phase of TGF-beta stimulation. We analyzed miR-106b-25 function upon prolonged exposure to TGF-beta that eventually results in apoptosis (Ohgushi et al., 2005, and **Figure 5B**).

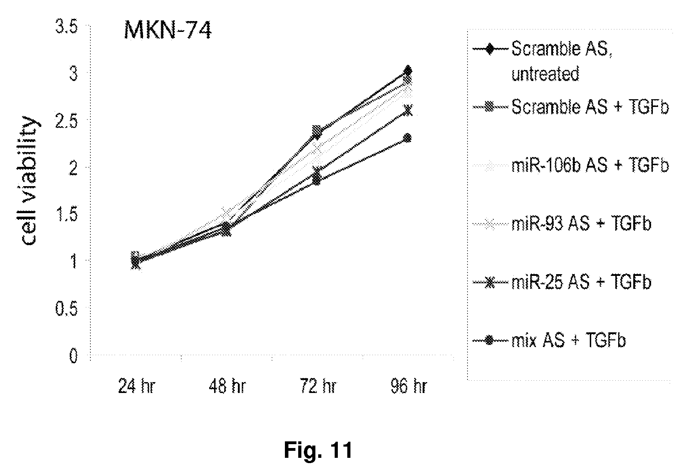

We examined the viability of Snu-16 cells stimulated with TGF-beta for 24-48 hours by tetrazolium reduction assay. Introduction of miR-106b, miR-93 and/or miR-25 mimics in these cells induced marked resistance to TGF-beta **(****Figure 7A**). Conversely, ASOs transfection induced a negative trend in the number of viable cells that reached statistical significance (p=0.003) when all the three miRNAs were inhibited at the same time **(****Figure 7B****).** This result was confirmed by FACS analysis that showed a significant increase in the number of subdiploid cells upon silencing of the three miRNAs (p<0.001). Moreover, the higher sensitivity of this assay allowed detection of smaller but significant changes (p<0.001) in the percentage of subdiploid cells upon individual inhibition of miR-106b, miR-93 or miR-25 **(****Figure 7C****).** Finally, silencing of miR-106b-25 partially restored sensitivity to TGF-beta in otherwise resistant MKN-74 cells **(****Figure 11****).** Together, these results are consistent with a model where endogenous miR-106b, miR-93 and miR-25 cooperate in modulating the expression of one or more targets mediating TGF-beta-dependent apoptosis.

We searched TargetScan database looking for effectors of apoptosis and we identified BCL2L11 (Bim) as the only strong candidate out of 18 human genes harboring putative binding sites for miR-106b, miR-93 and miR-25 at the same time **(****Figure 18** **- Table 6).** Bim is a BH3-only protein that critically regulates apoptosis in a variety of tissues by activating proapoptotic molecules like Box and Bad and antagonizing antiapoptotic molecules like Bcl2 and Bill (Gross et al., 1999). A fine balance in the intracellular concentrations of Bim and its partner proteins is crucial in order to properly regulate apoptosis. Bim is haploinsufficient and inactivation of even a single allele accelerates Myc-induced development of tumors in mice without loss of the other allele (Egle et al., 2004). Notably, Bim is the most downstream apoptotic effector of the TGF-beta pathway and its downmodulation abrogates TGF-beta-dependent apoptosis in Snu-16 cells (Ohgushi et al., 2005).

We determined whether Bim was a direct target of miR-106b-25. Snu-16 cells express all the three major isoforms of Bim, namely Bim EL, Bim L and Bim S. Intriguingly, antagonizing endogenous miR-25 by ASOs transfection induced an accumulation of all the three isoforms in Snu-16 cells whereas miR-25 overexpression by either oligonucleotide transfection or lentiviral transduction reduced their expression. On the contrary, miR-106b and miR-93 did not influence Bim expression in 3 out of 3 tested gastric cancer cell lines **(****Figure 7D****).**

While it is still possible that miR-106b and miR-93 cooperate with miR-25 in regulating Bim expression in other tissues, this supports a model where multiple effectors of apoptosis are coordinately repressed by each of the three miRNAs in gastric cancer.

We focused on Bim as one of these apoptotic effectors and determined that miR-25 predicted binding sites on its 3'UTR mediate target recognition and subsequent inhibition of translation by luciferase assay **(****Figure 7E****).** Moreover, Bim EL and Bim L mRNA levels were unchanged in Snu-16 cells upon miR-25 overexpression, which is indicative of a post-transcriptional regulatory mechanism **(****Figure 7F****).**

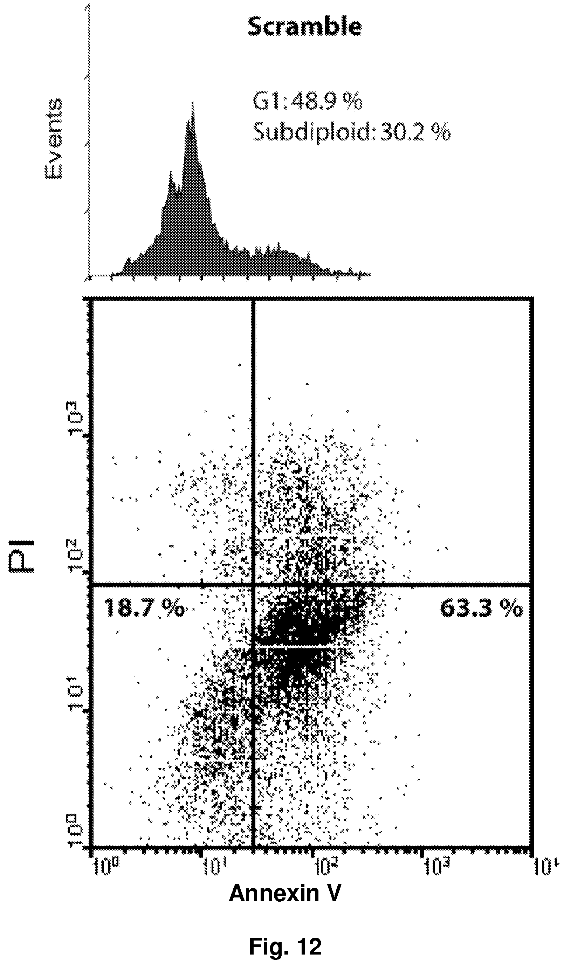

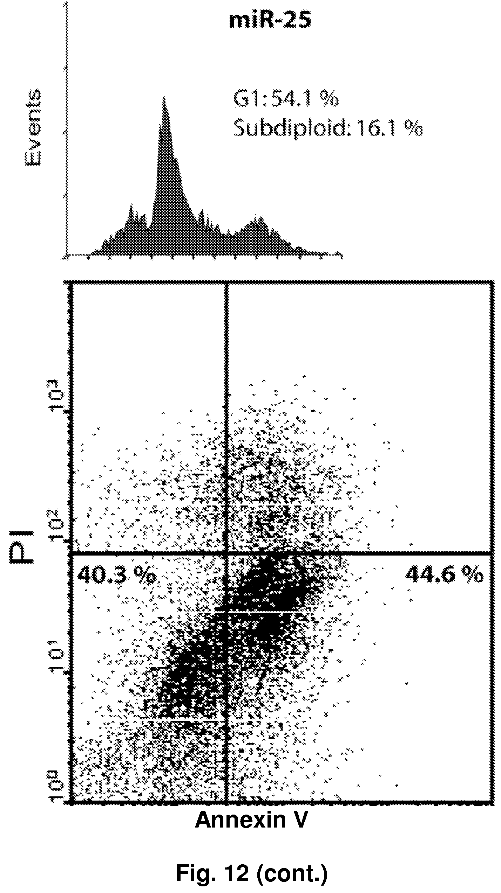

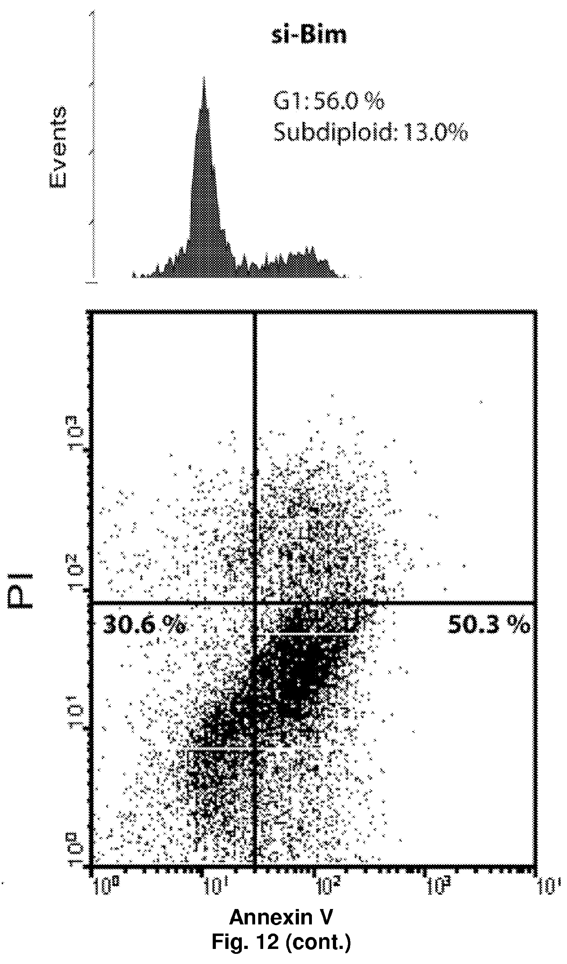

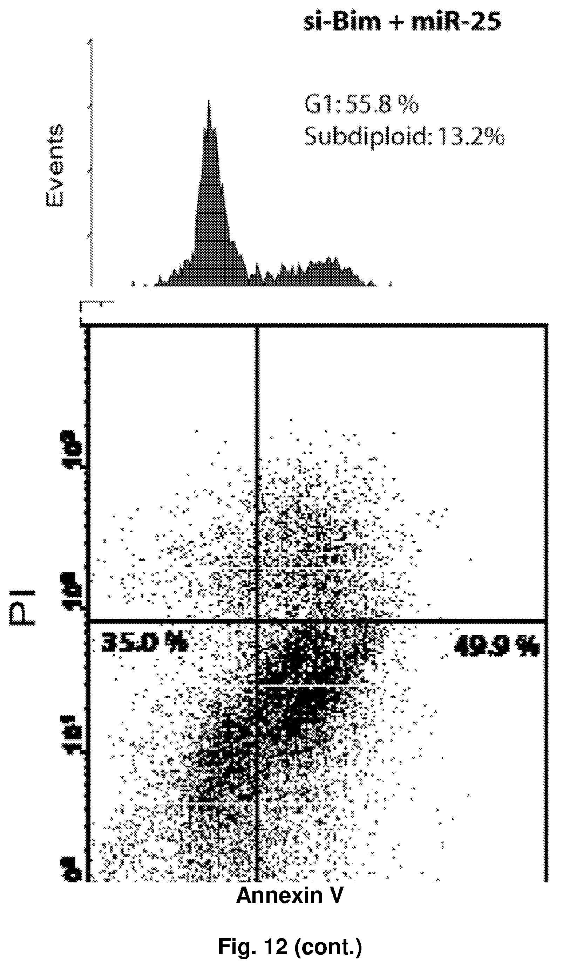

In order to establish the importance of Bim downregulation relative to miR-25 specific antiapoptotic function, we suppressed Bim protein in Snu-16 cells using a siRNA against its three major isoforms (si-Bim, **Figure 7D**) and we subsequently treated these cells with TGF-beta for 24 hours. Notably, protection from apoptosis conferred by si-Bim and miR-25 was very similar, as determined by sub-diploid DNA content and Annexin V staining. Moreover, co-transfection of Bim and miR-25 did not have significant additive effects (p=0.6328), suggesting that Bim downregulation is a main mechanism of resistance to TGF-beta-induced apoptosis in miR-25 overexpressing cells **(****Figure 7G** and **Figure 12****).**

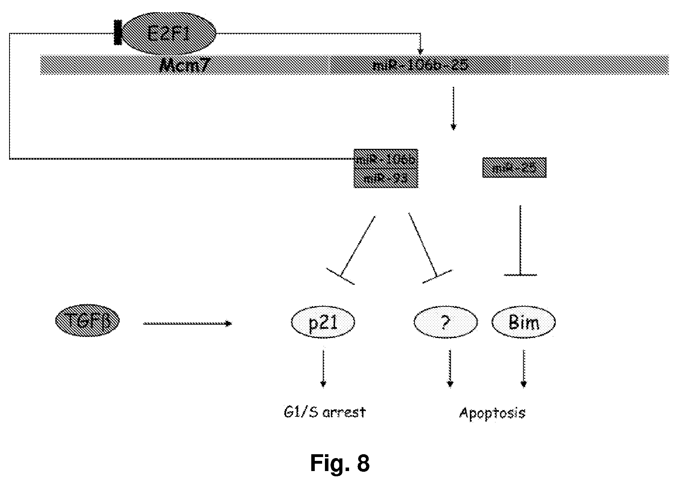

We show that miR-106b-25 cluster, activated by E2F1 and upregulated in human gastric adenocarcinomas, alters the physiological response of gastric cancer cells to TGF-beta affecting both cell cycle arrest and apoptosis **(****Figure 8****).**

These findings are of particular relevance in a gastric cancer model as impairment of the TGF-beta tumor suppressor pathway is a critical step in the development of gastric tumors.

### Discussion

We performed a genome-wide analysis of miRNA expression in different steps of gastric carcinogenesis. Since the vast majority of gastric tumors originate from a chronic inflammatory background (Uemura et al., 2001), we considered of particular relevance discriminating between preneoplastic and tumor-specific alterations.

For the first time, we identified the specific overexpression of a miRNA cluster in human tumors that had been ignored thus far. Although we focused on gastric cancer, overexpression of miR-106b, miR-93 and miR-25 in other types of cancer may be a common, yet underestimated, event.

In fact, miR-106b-25 expression is intimately linked with the expression of E2F1 and Mcm7 that are involved in basic mechanisms of cellular proliferation. For example, Mcm7 is frequently overexpressed in prostate cancer (Ren et al., 2006) and, in fact, we previously described miR-25 upregulation in a large-scale miRNA study on this type of cancer (Volinia et al., 2006). Moreover, we showed that stem-loop qRT-PCR probes commonly used in assaying the expression of miR-92, that is overexpressed in most human tumors (Volinia et al., 2006), cross-hybridize with miR-25. However, given the nearly identical sequences, it is very likely that miR-106b-25 and miR-17-92 cooperate in exerting similar, if not identical, functions: in fact, we found that miR-17-5p, miR-18a and miR-20a also inhibit p21 expression whereas miR-92 represses Bim expression (F.P. and A.V., unpublished data). Moreover, both miR-106b-25 and miR-17-92 are regulated by E2F1. These clusters also exhibit some differences, though. For example, miR-106b resembles miR-17-5p but it is three nucleotides shorter: it has been reported that specific sequences in the 3' termini can define the intracellular localization of miRNAs (Hwang et al., 2007). Moreover, the miR-19 family is not represented in the miR-106b-25 cluster **(****Figure 2A**).

On the other hand, miR-93 belongs to the same family of miR-372 and miR-373: these miRNAs are overexpressed in testicular germ cell tumors where they impair LATS2 expression, making cells insensitive to high p21 levels (Voorhoeve et al., 2006).

As shown herein, miR-93 acts within the same pathway, directly targeting p21 expression. Therefore, this family of miRNAs is now believed to be involved in the control of a crucial hub for the regulation of cell cycle and may have particular relevance in cancer.

Moreover, miR-93 shares high sequence homology with miR-291-3p, miR-294 and miR-295: these miRNAs are specifically expressed in pluripotent ES cells and they are either silenced or downregulated upon differentiation (Houbaviy et al., 2003). While not wishing to be bound by theory, the inventors herein now believe that these miRNAs may be similarly involved in the regulation of p21.

The inventors show that miRNAs play a role in the control of cell cycle through different mechanisms. In the case of E2F1, miRNAs seem to act mainly in the context of regulatory, redundant feedback loops. In fact, miR-106b, miR-93, miR-17-5p and miR-20a, located on separate miRNA clusters, are regulated by E2F1 and presumably cooperate in inhibiting its translation.

At the same time, we found these miRNAs to be involved in the control of p21 expression and early response to TGF-beta. The inventors also believe that they also control other tumor suppressor pathways converging on p21. Loss of p21 function by mutation, deletion, hypermethylation, ubiquination or mislocalization is a frequent event and a negative prognostic factor in human gastric cancer (Mattioli et al., 2007). However, the role of miRNAs in p21 regulation had never been explored before. Since 80% of the studied gastric primary tumors did not express p21 protein at detectable levels we could not establish an inverse correlation between miRNAs and p21 protein expression. However, p21 mRNA levels in primary tumors were often comparable to normal tissues, indicating post-transcriptional regulation as a frequent cause of p21 downregulation in gastric cancer (F.P and A.V., unpublished data).

Interestingly, induction of p21 expression seemed to be a prerequisite to elicit a miR-106b/miR-93 associated response in the early phase of TGF-beta stimulation. Conversely, silencing p21 by RNAi dramatically decreased the effect of these miRNAs on cell cycle. Although hundreds of different targets are predicted for each miRNA by computational methods there is increasing evidence that "primary miRNA targets" may be critical for specific biological functions. For example, miR-10b enhances cell motility and invasiveness of breast cancer cells but this phenotype is completely reverted upon constitutive expression of its target HOXD10, although over one hundred targets are predicted for this miRNA (Ma and Weinberg, 2007). It is to be noted, of course, these observations do not exclude other contexts where parallel regulation of multiple targets by a single miRNA is necessary to exert a specific function. Furthermore, multiple miRNAs may cooperate in exerting the same function.

This is the case of the miR-106b-25 cluster that protects gastric cancer cells from apoptosis. Such effect is partitioned between the three miRNAs that cooperate in repressing the expression of different proapoptotic molecules. We identified Bim, the most downstream apoptotic effector of the TGF-beta pathway (Ohgushi et al., 2005), as a key target of miR-25. This is of particular relevance in a gastric cancer model. In fact, TGF-beta is one of the main regulators of gastric homeostasis and is essential in regulating the physiological turnover of epithelial cells through apoptosis (van den Brink and Offerhaus, 2007). While the identity of miR-106b and miR-93 proapoptotic targets remains elusive, we could clearly detect antiapoptotic and proapoptotic responses associated with miR-106b, miR-93 and/or miR-25 overexpression and inhibition, respectively; these properties emerge in the late phase of TGF-beta stimulation when cell cycle arrest is revoked and apoptosis becomes the dominant process characterizing the response of gastric cells to TGF-beta. The small but significant alterations observed upon inhibition of single miRNAs, readily detected by analysis of subdiploid DNA content, acquire biological consistency when the three ASOs are delivered together, confirming the cooperative relationship between these clustered miRNAs.

Although a negative trend was observed in TGF-beta-stimulated cells transfected with single ASOs by both tetrazolium reduction assay and analysis of subdiploid DNA content, this did not reach statistical significance in the tetrazolium reduction assay. This is to be imputed to the 5-10% standard error associated with this assay that statistically excludes smaller differences. On the contrary, the standard error in the analysis of subdiploid DNA content was below 2% in our hands.

When we looked at Bim expression in primary tumors we noticed general overexpression compared to normal tissues (F.P. and A.V. unpublished data). This is consistent with previous studies showing that Bim is induced by oncogenic stress as a safeguard mechanism to prevent aberrant proliferation. Specifically, Bim is overexpressed in Myc transgenic mice, determining extensive apoptosis of normal cells. However, the onset of tumors in these mice coincides with the loss of one Bim allele that becomes insufficient. Still, Bim remains definitely overexpressed in tumors compared to healthy tissues that are not subject to oncogenic stress (Egle et al., 2004). Therefore, it is hard to define a threshold below which Bim insufficiency occurs and alternative strategies are needed to define the importance of miR-25 upregulation *in vivo*.

Several mechanisms have been described leading to Bim downregulation in cancer, from transcriptional regulation to protein degradation (Yano et al., 2006; Tan et al., 2005). While all of these mechanisms clearly contribute to Bim silencing, we propose miR-25 interference as a novel mechanism of Bim post-transcriptional regulation in gastric cancer.

It has been extensively debated whether miRNAs are just fine-tuning molecules or they act as key gene switches. Recent studies suggest that both hypotheses are probably true, depending on the specific biological context. From this perspective, the therapeutic potential of miRNAs in cancer may be strictly associated with the occurrence of specific miRNA-dependent functional alterations. Knowing the mechanisms of action of tumor-related miRNAs is useful in establishing the molecular diagnosis of miRNA-dependent tumors, allowing the rational selection of those patients eventually responding to miRNA-based therapies.

### Experimental procedures

### Cell culture and treatments:

All cell lines were obtained by ATCC and cultured in RPMI 1640 medium supplemented with 10% fetal bovine serum, penicillin and streptomycin. Cells were transfected with Lipofectamine 2000 (Invitrogen) using 100 nM microRNA precursors (Ambion), 100 nM si-p21 (Santa Cruz), 100 nM si-Bim (Cell Signalling) or 100 nM LNA microRNA antisense oligonucleotides (Exiqon). Protein lysates and total RNA were collected at the time indicated. MiRNA processing and expression were verified by Northern Blot and stem-loop qRT-PCR. We confirmed transfection efficiency (> 95%) using BLOCK-IT Fluorescent Oligo (Invitrogen) for all the cell lines.

For synchronization experiments, AGS cells were grown in 10% FBS RPMI 1640 containing 0.03 Pg/ml nocodazole for 12 hours and subsequently released in fresh medium. Progression through the cell cycle was followed by FACS analysis until 8 hours, after which cells rapidly lost synchronization.

For TGF-beta experiments, 2x106 Snu-16 cells were transfected in 6-well plates in a 1:1 mixture of Optimem (GIBCO) and RPMI 1640 10% FBS (Sigma) using 5 ul Lipofectamine 2000 and 100 nM miRNA precursors (Ambion) or LNA antisense oligonucleotides (Exiqon). After 12 hours, medium was replaced with RPMI 1640 10% FBS containing 1 ng/ml human recombinant TGF-beta1 (Sigma). Number of viable cells was assayed using WST tetrazolium salt (CCK-8, Dojindo) as per the manufacturer instructions. All the experiments were performed in triplicate. Results were expressed as mean ± SD.

### qRT-PCR:

Mature miRNAs and other mRNAs were assayed using the single tube TaqMan MicroRNA Assays and the Gene Expression Assays, respectively, in accordance with manufacturer's instructions (Applied Biosystems, Foster City, CA). All RT reactions, including no-template controls and RT minus controls, were run in a GeneAmp PCR 9700 Thermocycler (Applied Biosystems). RNA concentrations were determined with a NanoDrop (NanoDrop Technologies, Inc.). Samples were normalized to RNU49 or CAPN2 (Applied Biosystems), as indicated. Gene expression levels were quantified using the ABI Prism 7900HT Sequence detection system (Applied Biosystems). Comparative real-time PCR was performed in triplicate, including no-template controls. Relative expression was calculated using the comparative Ct method.

### Luciferase Assays

MKN-74 gastric cancer cells were cotransfected in six-well plates with 1 ug of pGL3 firefly luciferase reporter vector (see supplementary Experimental Procedures), 0.1 ug of the phRLSV40 control vector (Promega) and 100 nM microRNA precursors (Ambion) using Lipofectamine 2000 (Invitrogen). Firefly and Renilla luciferase activities were measured consecutively by using the Dual Luciferase Assay (Promega) 24 h after transfection. Each reporter plasmid was transfected at least twice (on different days) and each sample was assayed in triplicate.

### Flow Cytometry

For cell cycle analysis, 2x106 cells were fixed in cold methanol, RNAse-treated, and stained with propidium iodide (Sigma). Cells were analyzed for DNA content by EPICS-XL scan (Beckman Coulter) by using doublet discrimination gating. All analyses were performed in triplicate and 20,000 gated events/sample were counted. For apoptosis analysis, cells were washed in cold PBS, incubated with AnnexinV-FITC (BD Biopharmingen) and PI (Sigma) for 15 minutes in the dark and analyzed within 1 hour.

### Statistical analysis

Results of experiments are expressed as mean +/- SD. Student's unpaired t test was used to compare values of test and control samples. P < 0.05 indicated significant difference.

### EXAMPLE II

### Tissue samples:

Primary gastric tumor samples were obtained by the Department of Histopathology (Sant'Andrea Hospital, University of Rome "La Sapienza", Italy). All of the samples had patient's informed consent and were histologically confirmed. Protocol for tissue procurement was approved by the Sant'Andrea Hospital Bioethical Committee. Each tumor was paired with a non-tumor gastric mucosa control from the same patient.

### Microarrays:

Microarray analysis was performed as described (Liu et al., 2004). Briefly, 5 ug of total RNA was used for hybridization on 2nd generation miRNA microarray chips (V2). These chips contain gene-specific 40-mer oligonucleotide probes for 250 human miRNAs, spotted by contacting technologies and covalently attached to a polymeric matrix. The microarrays were hybridized in 6X SSPE (0.9 M NaCl_60 mM NaH2PO4_H2O_8 mM EDTA, pH 7.4), 30% formamide at 25°C for 18 h, washed in 0.75X TNT (Tris/HCl/NaCl/Tween 20) at 37°C for 40 min, and processed by using a method of direct detection of the biotin-containing transcripts by streptavidin-Alexa Fluor 647 conjugate. Processed slides were scanned by using a microarray scanner, with the laser set to 635 nm, at fixed PMT setting, and a scan resolution of 10 mm. Array data were normalized using Global Median, Lowess or Quantile methods, obtaining similar results. Data published in this study were derived from Quantile normalization. Differentially expressed miRNAs were identified by using the t test procedure within significance analysis of microarrays (SAM).

### Western Blots:

Antibodies for immunoblotting were as follows: E2F1 (Santa Cruz, mouse monoclonal, 1:500), AIB-1 (Cell Signalling, mouse monoclonal, 1:1000), p21 (Cell Signalling, mouse monoclonal, 1:1000), p27 (Santa Cruz, mouse monoclonal 1:500), CDK2 (Cell Signalling, mouse monoclonal, 1:1000), CDK4 (Cell Signalling, mouse monoclonal, 1:1000), cyclin D1 (Cell Signalling, mouse monoclonal, 1:1000), cyclin E (Santa Cruz, rabbit polyclonal, 1:500) p15 (Cell Signalling, rabbit polyclonal, 1:1000), Bim (Cell Signalling, rabbit polyclonal 1:1000), Vinculin (Santa Cruz, mouse monoclonal, 1:500), GAPDH (Calbiochem, mouse monoclonal, 1:3000). Bands were quantified using GelDoc software (Biorad).

### Adenoviral and Lentiviral infections:

Adeno-E2F1 was kindly provided by G. Leone and infection was performed as described (Leone et al., 1998). MiR-106b, miR-93, miR-25 and miR-106b-25 precursor cDNAs were PCR-amplified from 293T/17 cells genomic DNA and cloned under a CMV promoter into a variant third-generation lentiviral vector, pRRL-CMV-PGK-GFP-WPRE, called Tween, to simultaneously transduce both the reporter GFP and the miRNA. Lentiviral supernatants preparation and infection were performed as described (Bonci et al., 2003). Lentiviral transduction produced a 710 fold-change in miRNA expression, as determined by qRT-PCR. Transduction efficiency >90% was verified by fluorescent microscopy.

### qRT-PCR (miRNA precursors):

For microRNA precursor qRT-PCR, total RNA isolated with TRIzol reagent (Invitrogen) was processed after DNase treatment (Ambion) directly to cDNA by reverse transcription using ThermoScript kit (Invitrogen). Target sequences were amplified by qPCR using Power Syb-Green PCR Master Mix (Applied Biosystems). Samples were normalized to U6. Primer sequences are available upon request.

### Sensor plasmids:

E2F1, p21 and Bim 3'UTRs containing predicted miRNA binding sites were amplified by PCR from genomic DNA (293T/17 cells) and inserted into the pGL3 control vector (Promega) by using the Xba-I site immediately downstream from the stop codon of firefly luciferase. Deletions of the first 3 nucleotides in the miRNA seed-region complementary sites were inserted in mutant constructs using QuikChange-site-directed mutagenesis kit (Stratagene), according to the manufacturer's protocol. Primer sequences are available upon request.

### EXAMPLES of USES and DEFINITIONS THEREOF

The practice of the present invention will employ, unless otherwise indicated, conventional methods of pharmacology, chemistry, biochemistry, recombinant DNA techniques and immunology, within the skill of the art. Such techniques are explained fully in the literature. See, e.g., Handbook of Experimental Immunology, Vols. I-IV (D. M. Weir and C. C. Blackwell eds., Blackwell Scientific Publications); A. L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.).

As such, the definitions herein are provided for further explanation and are not to be construed as limiting.

The articles "a" and "an" are used herein to refer to one or to more than one (i.e., to at least one) of the grammatical object of the article. By way of example, "an element" means one element or more than one element.

A "marker" and "biomarker" is a gene and/or protein and/or functional variants thereof whose altered level of expression in a tissue or cell from its expression level in normal or healthy tissue or cell is associated with a disorder and/or disease state.

The "normal" level of expression of a marker is the level of expression of the marker in cells of a human subject or patient not afflicted with a disorder and/or disease state.

An "over-expression" or "significantly higher level of expression" of a marker refers to an expression level in a test sample that is greater than the standard error of the assay employed to assess expression, and in certain embodiments, at least twice, and in other embodiments, three, four, five or ten times the expression level of the marker in a control sample (e.g., sample from a healthy subject not having the marker associated disorder and/or disease state) and in certain embodiments, the average expression level of the marker in several control samples.

A "significantly lower level of expression" of a marker refers to an expression level in a test sample that is at least twice, and in certain embodiments, three, four, five or ten times lower than the expression level of the marker in a control sample (e.g., sample from a healthy subject not having the marker associated disorder and/or disease state) and in certain embodiments, the average expression level of the marker in several control samples.

A kit is any manufacture (e.g. a package or container) comprising at least one reagent, e.g., a probe, for specifically detecting the expression of a marker. The kit may be promoted, distributed or sold as a unit for performing the method of the present invention.

"Proteins" encompass marker proteins and their fragments; variant marker proteins and their fragments; peptides and polypeptides comprising an at least 15 amino acid segment of a marker or variant marker protein; and fusion proteins comprising a marker or variant marker protein, or an at least 15 amino acid segment of a marker or variant marker protein.

The compositions, kits and methods described herein have the following non-limiting uses, among others:
1) assessing whether a subject is afflicted with a disorder and/or disease state;
2) assessing the stage of a disorder and/or disease state in a subject;
3) assessing the grade of a disorder and/or disease state in a subject;
4) assessing the nature of a disorder and/or disease state in a subject;
5) assessing the potential to develop a disorder and/or disease state in a subject;
6) assessing the histological type of cells associated with a disorder and/or disease state in a subject;
7) making antibodies, antibody fragments or antibody derivatives that are useful for treating a disorder and/or disease state in a subject;
8) assessing the presence of a disorder and/or disease state in a subject's cells;
9) assessing the efficacy of one or more test compounds for inhibiting a disorder and/or disease state in a subject; 1
10) assessing the efficacy of a therapy for inhibiting a disorder and/or disease state in a subject;
11) monitoring the progression of a disorder and/or disease state in a subject;
12) selecting a composition or therapy for inhibiting a disorder and/or disease state in a subject;
13) treating a subject afflicted with a disorder and/or disease state;
14) inhibiting a disorder and/or disease state in a subject;
15) assessing the harmful potential of a test compound; and
16) preventing the onset of a disorder and/or disease state in a subject at risk therefor.

### Screening Methods

Animal models can be created to enable screening of therapeutic agents useful for treating or preventing a disorder and/or disease state in a subject. Accordingly, the methods are useful for identifying therapeutic agents for treating or preventing a disorder and/or disease state in a subject. The methods comprise administering a candidate agent to an animal model made by the methods described herein, assessing at least one response in the animal model as compared to a control animal model to which the candidate agent has not been administered. If at least one response is reduced in symptoms or delayed in onset, the candidate agent is an agent for treating or preventing the disease.

The candidate agents may be pharmacologic agents already known in the art or may be agents previously unknown to have any pharmacological activity. The agents may be naturally arising or designed in the laboratory. They may be isolated from microorganisms, animals or plants, or may be produced recombinantly, or synthesized by any suitable chemical method. They may be small molecules, nucleic acids, proteins, peptides or peptidomimetics. In the present disclosure, candidate agents are small organic compounds having a molecular weight of more than 50 and less than about 2,500 daltons. Candidate agents comprise functional groups necessary for structural interaction with proteins. Candidate agents are also found among biomolecules including, but not limited to: peptides, saccharides, fatty acids, steroids, purines, pyrimidines, derivatives, structural analogs or combinations thereof.

Candidate agents are obtained from a wide variety of sources including libraries of synthetic or natural compounds. There are, for example, numerous means available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. In the present disclosure, the candidate agents can be obtained using any of the numerous approaches in combinatorial library methods art, including, by non-limiting example: biological libraries; spatially addressable parallel solid phase or solution phase libraries; synthetic library methods requiring deconvolution; the "one-bead one-compound" library method; and synthetic library methods using affinity chromatography selection.

In the present disclosure, certain pharmacological agents may be subjected to directed or random chemical modifications, such as acylation, alkylation, esterification, amidification, etc. to produce structural analogs.

The same methods for identifying therapeutic agents for treating a disorder and/or disease state in a subject can also be used to validate lead compounds/agents generated from in vitro studies.

The candidate agent may be an agent that up- or down-regulates one or more a disorder and/or disease state in a subject response pathway. In the present disclosure, the candidate agent may be an antagonist that affects such pathway.

### Methods for Treating a Disorder and/or Disease State

There is provided herein methods for treating, inhibiting, relieving or reversing a disorder and/or disease state response. In the methods described herein, an agent that interferes with a signaling cascade is administered to an individual in need thereof, such as, but not limited to, subjects in whom such complications are not yet evident and those who already have at least one such response.

In the former instance, such treatment is useful to prevent the occurrence of such response and/or reduce the extent to which they occur. In the latter instance, such treatment is useful to reduce the extent to which such response occurs, prevent their further development or reverse the response.

In the present disclosure, the agent that interferes with the response cascade may be an antibody specific for such response.

### Expression of Biomarker(s)

Expression of a marker can be inhibited in a number of ways, including, by way of a non-limiting example, an antisense oligonucleotide can be provided to the disease cells in order to inhibit transcription, translation, or both, of the marker(s). Alternately, a polynucleotide encoding an antibody, an antibody derivative, or an antibody fragment which specifically binds a marker protein, and operably linked with an appropriate promoter/regulator region, can be provided to the cell in order to generate intracellular antibodies which will inhibit the function or activity of the protein. The expression and/or function of a marker may also be inhibited by treating the disease cell with an antibody, antibody derivative or antibody fragment that specifically binds a marker protein. Using the methods described herein, a variety of molecules, particularly including molecules sufficiently small that they are able to cross the cell membrane, can be screened in order to identify molecules which inhibit expression of a marker or inhibit the function of a marker protein. The compound so identified can be provided to the subject in order to inhibit disease cells of the subject.

Any marker or combination of markers, as well as any certain markers in combination with the markers, may be used in the compositions, kits and methods described herein. In general, it is desirable to use markers for which the difference between the level of expression of the marker in disease cells and the level of expression of the same marker in normal colon system cells is as great as possible. Although this difference can be as small as the limit of detection of the method for assessing expression of the marker, it is desirable that the difference be at least greater than the standard error of the assessment method, and, in certain embodiments, a difference of at least 2-, 3-, 4-, 5-, 6-, 7-, 8-, 9-, 10-, 15-, 20-, 100-, 500-, 1000-fold or greater than the level of expression of the same marker in normal tissue.

It is recognized that certain marker proteins are secreted to the extracellular space surrounding the cells. These markers are used in the present disclosure of the compositions, kits and methods, owing to the fact that such marker proteins can be detected in a body fluid sample, which may be more easily collected from a human subject than a tissue biopsy sample. In addition, in vivo techniques for detection of a marker protein include introducing into a subject a labeled antibody directed against the protein. For example, the antibody can be labeled with a radioactive marker whose presence and location in a subject can be detected by standard imaging techniques.

In order to determine whether any particular marker protein is a secreted protein, the marker protein is expressed in, for example, a mammalian cell, such as a human cell line, extracellular fluid is collected, and the presence or absence of the protein in the extracellular fluid is assessed (e.g. using a labeled antibody which binds specifically with the protein).

It will be appreciated that subject samples containing such cells may be used in the methods described herein. In this disclosure, the level of expression of the marker can be assessed by assessing the amount (e.g., absolute amount or concentration) of the marker in a sample. The cell sample can, of course, be subjected to a variety of post-collection preparative and storage techniques (e.g., nucleic acid and/or protein extraction, fixation, storage, freezing, ultrafiltration, concentration, evaporation, centrifugation, etc.) prior to assessing the amount of the marker in the sample.

It will also be appreciated that the markers may be shed from the cells into, for example, the respiratory system, digestive system, the blood stream and/or interstitial spaces. The shed markers can be tested, for example, by examining the sputum, BAL, serum, plasma, urine, stool, etc.

The compositions, kits and methods can be used to detect expression of marker proteins having at least one portion which is displayed on the surface of cells which express it. For example, immunological methods may be used to detect such proteins on whole cells, or computer-based sequence analysis methods may be used to predict the presence of at least one extracellular domain (i.e., including both secreted proteins and proteins having at least one cell-surface domain). Expression of a marker protein having at least one portion which is displayed on the surface of a cell which expresses it may be detected without necessarily lysing the cell (e.g., using a labeled antibody which binds specifically with a cell-surface domain of the protein).

Expression of a marker may be assessed by any of a wide variety of methods for detecting expression of a transcribed nucleic acid or protein. Non-limiting examples of such methods include immunological methods for detection of secreted, cell-surface, cytoplasmic or nuclear proteins, protein purification methods, protein function or activity assays, nucleic acid hybridization methods, nucleic acid reverse transcription methods and nucleic acid amplification methods.

In a particular embodiment, expression of a marker is assessed using an antibody (e.g., a radio-labeled, chromophore-labeled, fluorophore-labeled or enzyme-labeled antibody), an antibody derivative (e.g., an antibody conjugated with a substrate or with the protein or ligand of a protein-ligand pair), or an antibody fragment (e.g., a single-chain antibody, an isolated antibody hypervariable domain, etc.) which binds specifically with a marker protein or fragment thereof, including a marker protein which has undergone all or a portion of its normal post-translational modification.

In another particular embodiment, expression of a marker is assessed by preparing mRNA/cDNA (i.e., a transcribed polynucleotide) from cells in a subject sample, and by hybridizing the mRNA/cDNA with a reference polynucleotide which is a complement of a marker nucleic acid, or a fragment thereof. cDNA can, optionally, be amplified using any of a variety of polymerase chain reaction methods prior to hybridization with the reference polynucleotide; preferably, it is not amplified. Expression of one or more markers can likewise be detected using quantitative PCR to assess the level of expression of the marker(s). Alternatively, any of the many methods of detecting mutations or variants (e.g., single nucleotide polymorphisms, deletions, etc.) of a marker may be used to detect occurrence of a marker in a subject.

In a related embodiment, a mixture of transcribed polynucleotides obtained from the sample is contacted with a substrate having fixed thereto a polynucleotide complementary to or homologous with at least a portion (e.g., at least 7, 10, 15, 20, 25, 30, 40, 50, 100, 500, or more nucleotide residues) of a marker nucleic acid. If polynucleotides complementary to or homologous with are differentially detectable on the substrate (e.g., detectable using different chromophores or fluorophores, or fixed to different selected positions), then the levels of expression of a plurality of markers can be assessed simultaneously using a single substrate (e.g., a "gene chip" microarray of polynucleotides fixed at selected positions). When a method of assessing marker expression is used which involves hybridization of one nucleic acid with another, it is desired that the hybridization be performed under stringent hybridization conditions.

In certain embodiments, the biomarker assays can be performed using mass spectrometry or surface plasmon resonance. In the present disclosure, the method of identifying an agent active against a disorder and/or disease state in a subject can include one or more of:
a) providing a sample of cells containing one or more markers or derivative thereof;
b) preparing an extract from such cells;
c) mixing the extract with a labeled nucleic acid probe containing a marker binding site; and,
d) determining the formation of a complex between the marker and the nucleic acid probe in the presence or absence of the test agent. The determining step can include subjecting said extract/nucleic acid probe mixture to an electrophoretic mobility shift assay.

In the present disclosure, the determining step comprises an assay selected from an enzyme linked immunoabsorption assay (ELISA), fluorescence based assays and ultra high throughput assays, for example surface plasmon resonance (SPR) or fluorescence correlation spectroscopy (FCS) assays. In such embodiments, the SPR sensor is useful for direct real-time observation of biomolecular interactions since SPR is sensitive to minute refractive index changes at a metal-dielectric surface. SPR is a surface technique that is sensitive to changes of 10⁵ to 10⁻⁶ refractive index (RI) units within approximately 200 nm of the SPR sensor/sample interface. Thus, SPR spectroscopy is useful for monitoring the growth of thin organic films deposited on the sensing layer.

Because the compositions, kits, and methods rely on detection of a difference in expression levels of one or more markers, it is desired that the level of expression of the marker is significantly greater than the minimum detection limit of the method used to assess expression in at least one of normal cells and colon cancer-affected cells.

It is understood that by routine screening of additional subject samples using one or more of the markers, it will be realized that certain of the markers are over-expressed in cells of various types, including a specific disorders and/or disease state in a subject.

In addition, as a greater number of subject samples are assessed for expression of the markers and the outcomes of the individual subjects from whom the samples were obtained are correlated, it will also be confirmed that altered expression of certain of the markers are strongly correlated with a disorder and/or disease state in a subject and that altered expression of other markers are strongly correlated with other diseases. The compositions, kits, and methods are thus useful for characterizing one or more of the stage, grade, histological type, and nature of a disorder and/or disease state in a subject.

When the compositions, kits, and methods are used for characterizing one or more of the stage, grade, histological type, and nature of a disorder and/or disease state in a subject, it is desired that the marker or panel of markers is selected such that a positive result is obtained in at least about 20%, and in certain embodiments, at least about 40%, 60%, or 80%, and in substantially all subjects afflicted with a disorder and/or disease state of the corresponding stage, grade, histological type, or nature. The marker or panel of markers of the invention can be selected such that a positive predictive value of greater than about 10% is obtained for the general population (in a non-limiting example, coupled with an assay specificity greater than 80%).

When a plurality of markers are used in the compositions, kits, and methods, the level of expression of each marker in a subject sample can be compared with the normal level of expression of each of the plurality of markers in non- disorder and/or non-disease samples of the same type, either in a single reaction mixture (i.e. using reagents, such as different fluorescent probes, for each marker) or in individual reaction mixtures corresponding to one or more of the markers. In the present disclosure, a significantly increased level of expression of more than one of the plurality of markers in the sample, relative to the corresponding normal levels, is an indication that the subject is afflicted with a disorder and/or disease state. When a plurality of markers is used, 2, 3, 4, 5, 8, 10, 12, 15, 20, 30, or 50 or more individual markers can be used; in the present disclosure, the use of fewer markers may be desired.

In order to maximize the sensitivity of the compositions, kits, and methods (i.e. by interference attributable to cells of system origin in a subject sample), it is desirable that the marker used therein be a marker which has a restricted tissue distribution, e.g., normally not expressed in a non-system tissue.

It is recognized that the compositions, kits, and methods will be of particular utility to subjects having an enhanced risk of developing a disorder and/or disease state in a subject and their medical advisors. Subjects recognized as having an enhanced risk of developing a disorder and/or disease include, for example, subjects having a familial history of such disorder or disease.

The level of expression of a marker in normal human system tissue can be assessed in a variety of ways. In one embodiment, this normal level of expression is assessed by assessing the level of expression of the marker in a portion of system cells which appear to be normal and by comparing this normal level of expression with the level of expression in a portion of the system cells which is suspected of being abnormal. Alternately, and particularly as further information becomes available as a result of routine performance of the methods described herein, population-average values for normal expression of the markers may be used. In other embodiments, the 'normal' level of expression of a marker may be determined by assessing expression of the marker in a subject sample obtained from a non-afflicted subject, from a subject sample obtained from a subject before the suspected onset of a disorder and/or disease state in the subject, from archived subject samples, and the like.

The present disclosure relates to compositions, kits, and methods for assessing the presence of disorder and/or disease state cells in a sample (e.g. an archived tissue sample or a sample obtained from a subject). These compositions, kits, and methods are substantially the same as those described above, except that, where necessary, the compositions, kits, and methods are adapted for use with samples other than subject samples. For example, when the sample to be used is a parafinized, archived human tissue sample, it can be necessary to adjust the ratio of compounds in the compositions, in the kits, or the methods used to assess levels of marker expression in the sample.

### Kits and Reagents

The kits are useful for assessing the presence of disease cells (e.g. in a sample such as a subject sample). The kit comprises a plurality of reagents, each of which is capable of binding specifically with a marker nucleic acid or protein. Suitable reagents for binding with a marker protein include antibodies, antibody derivatives, antibody fragments, and the like. Suitable reagents for binding with a marker nucleic acid (e.g. a genomic DNA, an MRNA, a spliced MRNA, a cDNA, or the like) include complementary nucleic acids. For example, the nucleic acid reagents may include oligonucleotides (labeled or non-labeled) fixed to a substrate, labeled oligonucleotides not bound with a substrate, pairs of PCR primers, molecular beacon probes, and the like.

The kits may optionally comprise additional components useful for performing the methods described herein. By way of example, the kit may comprise fluids (e.g. SSC buffer) suitable for annealing complementary nucleic acids or for binding an antibody with a protein with which it specifically binds, one or more sample compartments, an instructional material which describes performance of the method, a sample of normal colon system cells, a sample of colon cancer-related disease cells, and the like.

### Methods of Producing Antibodies

Disclosed herein is a method of making an isolated hybridoma which produces an antibody useful for assessing whether a subject is afflicted with a disorder and/or disease state. In this method, a protein or peptide comprising the entirety or a segment of a marker protein is synthesized or isolated (e.g. by purification from a cell in which it is expressed or by transcription and translation of a nucleic acid encoding the protein or peptide in vivo or in vitro). A vertebrate, for example, a mammal such as a mouse, rat, rabbit, or sheep, is immunized using the protein or peptide. The vertebrate may optionally (and preferably) be immunized at least one additional time with the protein or peptide, so that the vertebrate exhibits a robust immune response to the protein or peptide. Splenocytes are isolated from the immunized vertebrate and fused with an immortalized cell line to form hybridomas, using any of a variety of methods. Hybridomas formed in this manner are then screened using standard methods to identify one or more hybridomas which produce an antibody which specifically binds with the marker protein or a fragment thereof. Also disclosed provided herein are hybridomas made by this method and antibodies made using such hybridomas.

### Methods of Assessing Efficacy

Also disclosed herein a method of assessing the efficacy of a test compound for inhibiting disease cells. As described above, differences in the level of expression of the markers correlate with the abnormal state of the subject's cells. Although it is recognized that changes in the levels of expression of certain of the markers likely result from the abnormal state of such cells, it is likewise recognized that changes in the levels of expression of other of the markers induce, maintain, and promote the abnormal state of those cells. Thus, compounds which inhibit a disorder and/or disease state in a subject will cause the level of expression of one or more of the markers to change to a level nearer the normal level of expression for that marker (i.e. the level of expression for the marker in normal cells).

This method thus comprises comparing expression of a marker in a first cell sample and maintained in the presence of the test compound and expression of the marker in a second colon cell sample and maintained in the absence of the test compound. A significantly reduced expression of a marker in the presence of the test compound is an indication that the test compound inhibits a related disease. The cell samples may, for example, be aliquots of a single sample of normal cells obtained from a subject, pooled samples of normal cells obtained from a subject, cells of a normal cell line, aliquots of a single sample of related disease cells obtained from a subject, pooled samples of related disease cells obtained from a subject, cells of a related disease cell line, or the like.

In the disclosure, the samples are cancer-related disease cells obtained from a subject and a plurality of compounds believed to be effective for inhibiting various cancer-related diseases are tested in order to identify the compound which is likely to best inhibit the cancer-related disease in the subject.

This method may likewise be used to assess the efficacy of a therapy for inhibiting a related disease in a subject. In this method, the level of expression of one or more markers in a pair of samples (one subjected to the therapy, the other not subjected to the therapy) is assessed. As with the method of assessing the efficacy of test compounds, if the therapy induces a significantly lower level of expression of a marker then the therapy is efficacious for inhibiting a cancer-related disease. As above, if samples from a selected subject are used in this method, then alternative therapies can be assessed in vitro in order to select a therapy most likely to be efficacious for inhibiting a cancer-related disease in the subject.

As described herein, the abnormal state of human cells is correlated with changes in the levels of expression of the markers. There is also provided a method for assessing the harmful potential of a test compound. This method comprises maintaining separate aliquots of human cells in the presence and absence of the test compound. Expression of a marker in each of the aliquots is compared. A significantly higher level of expression of a marker in the aliquot maintained in the presence of the test compound (relative to the aliquot maintained in the absence of the test compound) is an indication that the test compound possesses a harmful potential. The relative harmful potential of various test compounds can be assessed by comparing the degree of enhancement or inhibition of the level of expression of the relevant markers, by comparing the number of markers for which the level of expression is enhanced or inhibited, or by comparing both. Various aspects are described in further detail in the following subsections.

### Isolated Proteins and Antibodies

One aspect pertains to isolated marker proteins and biologically active portions thereof, as well as polypeptide fragments suitable for use as immunogens to raise antibodies directed against a marker protein or a fragment thereof. In the present disclosure, the native marker protein can be isolated from cells or tissue sources by an appropriate purification scheme using standard protein purification techniques. In the present disclosure, a protein or peptide comprising the whole or a segment of the marker protein is produced by recombinant DNA techniques. Alternative to recombinant expression, such protein or peptide can be synthesized chemically using standard peptide synthesis techniques.

An "isolated" or "purified" protein or biologically active portion thereof is substantially free of cellular material or other contaminating proteins from the cell or tissue source from which the protein is derived, or substantially free of chemical precursors or other chemicals when chemically synthesized. The language "substantially free of cellular material" includes preparations of protein in which the protein is separated from cellular components of the cells from which it is isolated or recombinantly produced. Thus, protein that is substantially free of cellular material includes preparations of protein having less than about 30%, 20%, 10%, or 5% (by dry weight) of heterologous protein (also referred to herein as a "contaminating protein").

When the protein or biologically active portion thereof is recombinantly produced, it is also preferably substantially free of culture medium, i.e., culture medium represents less than about 20%, 10%, or 5% of the volume of the protein preparation. When the protein is produced by chemical synthesis, it is preferably substantially free of chemical precursors or other chemicals, i.e., it is separated from chemical precursors or other chemicals which are involved in the synthesis of the protein. Accordingly such preparations of the protein have less than about 30%, 20%, 10%, 5% (by dry weight) of chemical precursors or compounds other than the polypeptide of interest.

Biologically active portions of a marker protein include polypeptides comprising amino acid sequences sufficiently identical to or derived from the amino acid sequence of the marker protein, which include fewer amino acids than the full length protein, and exhibit at least one activity of the corresponding full-length protein. Typically, biologically active portions comprise a domain or motif with at least one activity of the corresponding full-length protein. A biologically active portion of a marker protein can be a polypeptide which is, for example, 10, 25, 50, 100 or more amino acids in length. Moreover, other biologically active portions, in which other regions of the marker protein are deleted, can be prepared by recombinant techniques and evaluated for one or more of the functional activities of the native form of the marker protein. In the present disclosure, useful proteins are substantially identical (e.g., at least about 40%, and in certain embodiments, 50%, 60%, 70%, 80%, 90%, 95%, or 99%) to one of these sequences and retain the functional activity of the corresponding naturally-occurring marker protein yet differ in amino acid sequence due to natural allelic variation or mutagenesis.

In addition, libraries of segments of a marker protein can be used to generate a variegated population of polypeptides for screening and subsequent selection of variant marker proteins or segments thereof.

### Predictive Medicine

The present disclosure also relates to uses of the animal models and markers in the field of predictive medicine in which diagnostic assays, prognostic assays, pharmacogenomics, and monitoring clinical trials are used for prognostic (predictive) purposes to thereby treat an individual prophylactically. Accordingly, also disclosed herein are diagnostic assays for determining the level of expression of one or more marker proteins or nucleic acids, in order to determine whether an individual is at risk of developing a particular disorder and/or disease. Such assays can be used for prognostic or predictive purposes to thereby prophylactically treat an individual prior to the onset of the disorder and/or disease.

In the present disclosure, the methods are useful for at least periodic screening of the same individual to see if that individual has been exposed to chemicals or toxins that change his/her expression patterns.

The present disclosure relates to monitoring the influence of agents (e.g., drugs or other compounds administered either to inhibit a disorder and/or disease or to treat or prevent any other disorder (e.g., in order to understand any system effects that such treatment may have) on the expression or activity of a marker in clinical trials.

### Pharmaceutical Compositions

The compounds may be in a formulation for administration topically, locally or systemically in a suitable pharmaceutical carrier. Remington's Pharmaceutical Sciences, 15th Edition by E. W. Martin (Mark Publishing Company, 1975), discloses typical carriers and methods of preparation. The compound may also be encapsulated in suitable biocompatible microcapsules, microparticles or micro spheres formed of biodegradable or non-biodegradable polymers or proteins or liposomes for targeting to cells. Such systems are well known to those skilled in the art and may be optimized for use with the appropriate nucleic acid.

Various methods for nucleic acid delivery are described, for example in Sambrook et al., 1989, Molecular Cloning: A Laboratory Manual, Cold Spring Harbor Laboratory, New York; and Ausubel et al., 1994, Current Protocols in Molecular Biology, John Wiley & Sons, New York. Such nucleic acid delivery systems comprise the desired nucleic acid, by way of example and not by limitation, in either "naked" form as a "naked" nucleic acid, or formulated in a vehicle suitable for delivery, such as in a complex with a cationic molecule or a liposome forming lipid, or as a component of a vector, or a component of a pharmaceutical composition. The nucleic acid delivery system can be provided to the cell either directly, such as by contacting it with the cell, or indirectly, such as through the action of any biological process.

Formulations for topical administration may include ointments, lotions, creams, gels, drops, suppositories, sprays, liquids and powders. Conventional pharmaceutical carriers, aqueous, powder or oily bases, or thickeners can be used as desired.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions, solutions or emulsions that can include suspending agents, solubilizers, thickening agents, dispersing agents, stabilizers, and preservatives. Formulations for injection may be presented in unit dosage form, e.g., in ampules or in multi-dose containers, with an added preservative. Those of skill in the art can readily determine the various parameters for preparing and formulating the compositions without resort to undue experimentation. The compound can be used alone or in combination with other suitable components.

In general, methods of administering compounds, including nucleic acids, are well known in the art. In particular, the routes of administration already in use for nucleic acid therapeutics, along with formulations in current use, provide preferred routes of administration and formulation for the nucleic acids selected will depend of course, upon factors such as the particular formulation, the severity of the state of the subject being treated, and the dosage required for therapeutic efficacy. As generally used herein, an "effective amount" is that amount which is able to treat one or more symptoms of the disorder, reverse the progression of one or more symptoms of the disorder, halt the progression of one or more symptoms of the disorder, or prevent the occurrence of one or more symptoms of the disorder in a subject to whom the formulation is administered, as compared to a matched subject not receiving the compound. The actual effective amounts of compound can vary according to the specific compound or combination thereof being utilized, the particular composition formulated, the mode of administration, and the age, weight, condition of the individual, and severity of the symptoms or condition being treated.

Any acceptable method known to one of ordinary skill in the art may be used to administer a formulation to the subject. The administration may be localized (i.e., to a particular region, physiological system, tissue, organ, or cell type) or systemic, depending on the condition being treated.

### Pharmacogenomics

The markers are also useful as pharmacogenomic markers. As used herein, a "pharmacogenomic marker" is an objective biochemical marker whose expression level correlates with a specific clinical drug response or susceptibility in a subject. The presence or quantity of the pharmacogenomic marker expression is related to the predicted response of the subject and more particularly the subject's tumor to therapy with a specific drug or class of drugs. By assessing the presence or quantity of the expression of one or more pharmacogenomic markers in a subject, a drug therapy which is most appropriate for the subject, or which is predicted to have a greater degree of success, may be selected.

### Monitoring Clinical Trials

Monitoring the influence of agents (e.g., drug compounds) on the level of expression of a marker can be applied not only in basic drug screening, but also in clinical trials. For example, the effectiveness of an agent to affect marker expression can be monitored in clinical trials of subjects receiving treatment for a colon cancer-related disease.

The present disclosure relates to a method for monitoring the effectiveness of treatment of a subject with an agent (e.g., an agonist, antagonist, peptidomimetic, protein, peptide, nucleic acid, small molecule, or other drug candidate) comprising the steps of:
i) obtaining a pre-administration sample from a subject prior to administration of the agent;
ii) detecting the level of expression of one or more selected markers in the pre-administration sample;
iii) obtaining one or more post-administration samples from the subject;
iv) detecting the level of expression of the marker(s) in the post-administration samples;
v) comparing the level of expression of the marker(s) in the pre-administration sample with the level of expression of the marker(s) in the post-administration sample or samples; and
vi) altering the administration of the agent to the subject accordingly.

For example, increased expression of the marker gene(s) during the course of treatment may indicate ineffective dosage and the desirability of increasing the dosage. Conversely, decreased expression of the marker gene(s) may indicate efficacious treatment and no need to change dosage.

### Electronic Apparatus Readable Media, Systems, Arrays and Methods of Using Same

As used herein, "electronic apparatus readable media" refers to any suitable medium for storing, holding or containing data or information that can be read and accessed directly by an electronic apparatus. Such media can include, but are not limited to: magnetic storage media, such as floppy discs, hard disc storage medium, and magnetic tape; optical storage media such as compact disc; electronic storage media such as RAM, ROM, EPROM, EEPROM and the like; and general hard disks and hybrids of these categories such as magnetic/optical storage media. The medium is adapted or configured for having recorded thereon a marker as described herein.

As used herein, the term "electronic apparatus" is intended to include any suitable computing or processing apparatus or other device configured or adapted for storing data or information. Examples of electronic apparatus suitable for use with the present invention include stand-alone computing apparatus; networks, including a local area network (LAN), a wide area network (WAN) Internet, Intranet, and Extranet; electronic appliances such as personal digital assistants (PDAs), cellular phone, pager and the like; and local and distributed processing systems.

As used herein, "recorded" refers to a process for storing or encoding information on the electronic apparatus readable medium. Those skilled in the art can readily adopt any method for recording information on media to generate materials comprising the markers described herein.

A variety of software programs and formats can be used to store the marker information of the present invention on the electronic apparatus readable medium. Any number of data processor structuring formats (e.g., text file or database) may be employed in order to obtain or create a medium having recorded thereon the markers. By providing the markers in readable form, one can routinely access the marker sequence information for a variety of purposes. For example, one skilled in the art can use the nucleotide or amino acid sequences in readable form to compare a target sequence or target structural motif with the sequence information stored within the data storage means. Search means are used to identify fragments or regions of the sequences which match a particular target sequence or target motif.

Thus, also disclosed herein is a medium for holding instructions for performing a method for determining whether a subject has a cancer-related disease or a pre-disposition to a cancer-related disease, wherein the method comprises the steps of determining the presence or absence of a marker and based on the presence or absence of the marker, determining whether the subject has a cancer-related disease or a pre-disposition to a cancer-related disease and/or recommending a particular treatment for a cancer-related disease or pre-cancer-related disease condition.

There is also disclosed herein an electronic system and/or in a network, a method for determining whether a subject has a cancer-related disease or a pre-disposition to a cancer-related disease associated with a marker wherein the method comprises the steps of determining the presence or absence of the marker, and based on the presence or absence of the marker, determining whether the subject has a particular disorder and/or disease or a pre-disposition to such disorder and/or disease, and/or recommending a particular treatment for such disease or disease and/or such pre-cancer-related disease condition. The method may further comprise the step of receiving phenotypic information associated with the subject and/or acquiring from a network phenotypic information associated with the subject.

Also disclosed herein is a network, a method for determining whether a subject has a disorder and/or disease or a pre-disposition to a disorder and/or disease associated with a marker, the method comprising the steps of receiving information associated with the marker, receiving phenotypic information associated with the subject, acquiring information from the network corresponding to the marker and/or disorder and/or disease, and based on one or more of the phenotypic information, the marker, and the acquired information, determining whether the subject has a disorder and/or disease or a pre-disposition thereto. The method may further comprise the step of recommending a particular treatment for the disorder and/or disease or pre-disposition thereto.

There is also disclosed herein a business method for determining whether a subject has a disorder and/or disease or a pre-disposition thereto, the method comprising the steps of receiving information associated with the marker, receiving phenotypic information associated with the subject, acquiring information from the network corresponding to the marker and/or a disorder and/or disease, and based on one or more of the phenotypic information, the marker, and the acquired information, determining whether the subject has a disorder and/or disease or a pre-disposition thereto. The method may further comprise the step of recommending a particular treatment therefor.

There is also disclosed herein an array that can be used to assay expression of one or more genes in the array. In the present disclosure, the array can be used to assay gene expression in a tissue to ascertain tissue specificity of genes in the array. In this manner, up to about 7000 or more genes can be simultaneously assayed for expression. This allows a profile to be developed showing a battery of genes specifically expressed in one or more tissues.

In addition to such qualitative determination, there is disclosed herein the quantitation of gene expression. Thus, not only tissue specificity, but also the level of expression of a battery of genes in the tissue is ascertainable. Thus, genes can be grouped on the basis of their tissue expression per se and level of expression in that tissue. This is useful, for example, in ascertaining the relationship of gene expression between or among tissues. Thus, one tissue can be perturbed and the effect on gene expression in a second tissue can be determined. In this context, the effect of one cell type on another cell type in response to a biological stimulus can be determined.

Such a determination is useful, for example, to know the effect of cell-cell interaction at the level of gene expression. If an agent is administered therapeutically to treat one cell type but has an undesirable effect on another cell type, the method provides an assay to determine the molecular basis of the undesirable effect and thus provides the opportunity to co-administer a counteracting agent or otherwise treat the undesired effect. Similarly, even within a single cell type, undesirable biological effects can be determined at the molecular level. Thus, the effects of an agent on expression of other than the target gene can be ascertained and counteracted.

In the present disclosure, the array can be used to monitor the time course of expression of one or more genes in the array. This can occur in various biological contexts, as disclosed herein, for example development of a disorder and/or disease, progression thereof, and processes, such as cellular transformation associated therewith.

The array is also useful for ascertaining the effect of the expression of a gene or the expression of other genes in the same cell or in different cells. This provides, for example, for a selection of alternate molecular targets for therapeutic intervention if the ultimate or downstream target cannot be regulated.

The array is also useful for ascertaining differential expression patterns of one or more genes in normal and abnormal cells. This provides a battery of genes that could serve as a molecular target for diagnosis or therapeutic intervention.

### Surrogate Markers

The markers may serve as surrogate markers for one or more disorders or disease states or for conditions leading up thereto. As used herein, a "surrogate marker" is an objective biochemical marker which correlates with the absence or presence of a disease or disorder, or with the progression of a disease or disorder. The presence or quantity of such markers is independent of the disease. Therefore, these markers may serve to indicate whether a particular course of treatment is effective in lessening a disease state or disorder. Surrogate markers are of particular use when the presence or extent of a disease state or disorder is difficult to assess through standard methodologies, or when an assessment of disease progression is desired before a potentially dangerous clinical endpoint is reached.

The markers are also useful as pharmacodynamic markers. As used herein, a "pharmacodynamic marker" is an objective biochemical marker which correlates specifically with drug effects. The presence or quantity of a pharmacodynamic marker is not related to the disease state or disorder for which the drug is being administered; therefore, the presence or quantity of the marker is indicative of the presence or activity of the drug in a subject. For example, a pharmacodynamic marker may be indicative of the concentration of the drug in a biological tissue, in that the marker is either expressed or transcribed or not expressed or transcribed in that tissue in relationship to the level of the drug. In this fashion, the distribution or uptake of the drug may be monitored by the pharmacodynamic marker. Similarly, the presence or quantity of the pharmacodynamic marker may be related to the presence or quantity of the metabolic product of a drug, such that the presence or quantity of the marker is indicative of the relative breakdown rate of the drug in vivo.

Pharmacodynamic markers are of particular use in increasing the sensitivity of detection of drug effects, particularly when the drug is administered in low doses. Since even a small amount of a drug may be sufficient to activate multiple rounds of marker transcription or expression, the amplified marker may be in a quantity which is more readily detectable than the drug itself. Also, the marker may be more easily detected due to the nature of the marker itself; for example, using the methods described herein, antibodies may be employed in an immune-based detection system for a protein marker, or marker-specific radiolabeled probes may be used to detect a mRNA marker. Furthermore, the use of a pharmacodynamic marker may offer mechanism-based prediction of risk due to drug treatment beyond the range of possible direct observations.

### Protocols for Testing

The method of testing for a disorder and/or disease may comprise, for example measuring the expression level of each marker gene in a biological sample from a subject over time and comparing the level with that of the marker gene in a control biological sample.

When the marker gene is one of the genes described herein and the expression level is differentially expressed (for examples, higher or lower than that in the control), the subject is judged to be affected with a disorder and/or disease. When the expression level of the marker gene falls within the permissible range, the subject is unlikely to be affected therewith.

The standard value for the control may be pre-determined by measuring the expression level of the marker gene in the control, in order to compare the expression levels. For example, the standard value can be determined based on the expression level of the above-mentioned marker gene in the control. For example, in the present dislosure, the permissible range is taken as ± 2S.D. based on the standard value. Once the standard value is determined, the testing method may be performed by measuring only the expression level in a biological sample from a subject and comparing the value with the determined standard value for the control.

Expression levels of marker genes include transcription of the marker genes to mRNA, and translation into proteins. Therefore, one method of testing for a disorder and/or disease is performed based on a comparison of the intensity of expression of mRNA corresponding to the marker genes, or the expression level of proteins encoded by the marker genes.

The measurement of the expression levels of marker genes in the testing for a disorder and/or disease can be carried out according to various gene analysis methods. Specifically, one can use, for example, a hybridization technique using nucleic acids that hybridize to these genes as probes, or a gene amplification technique using DNA that hybridize to the marker genes as primers.

The probes or primers used for the testing can be designed based on the nucleotide sequences of the marker genes. The identification numbers for the nucleotide sequences of the respective marker genes are describer herein.

Further, it is to be understood that genes of higher animals generally accompany polymorphism in a high frequency. There are also many molecules that produce isoforms comprising mutually different amino acid sequences during the splicing process. Any gene associated with a colon cancer-related disease that has an activity similar to that of a marker gene is included in the marker genes, even if it has nucleotide sequence differences due to polymorphism or being an isoform.

It is also to be understood that the marker genes can include homologs of other species in addition to humans. Thus, unless otherwise specified, the expression "marker gene" refers to a homolog of the marker gene unique to the species or a foreign marker gene which has been introduced into an individual.

Also, it is to be understood that a "homolog of a marker gene" refers to a gene derived from a species other than a human, which can hybridize to the human marker gene as a probe under stringent conditions. Such stringent conditions are known to one skilled in the art who can select an appropriate condition to produce an equal stringency experimentally or empirically.

A polynucleotide comprising the nucleotide sequence of a marker gene or a nucleotide sequence that is complementary to the complementary strand of the nucleotide sequence of a marker gene and has at least 15 nucleotides, can be used as a primer or probe. Thus, a "complementary strand" means one strand of a double stranded DNA with respect to the other strand and which is composed of A:T (U for RNA) and G:C base pairs.

In addition, "complementary" means not only those that are completely complementary to a region of at least 15 continuous nucleotides, but also those that have a nucleotide sequence homology of at least 40% in certain instances, 50% in certain instances, 60% in certain instances, 70% in certain instances, at least 80%, 90%, and 95% or higher. The degree of homology between nucleotide sequences can be determined by an algorithm, BLAST, etc.

Such polynucleotides are useful as a probe to detect a marker gene, or as a primer to amplify a marker gene. When used as a primer, the polynucleotide comprises usually 15 bp to 100 bp, and in certain embodiments 15 bp to 35 bp of nucleotides. When used as a probe, a DNA comprises the whole nucleotide sequence of the marker gene (or the complementary strand thereof), or a partial sequence thereof that has at least 15 bp nucleotides. When used as a primer, the 3' region must be complementary to the marker gene, while the 5' region can be linked to a restriction enzyme-recognition sequence or a tag.

"Polynucleotides" may be either DNA or RNA. These polynucleotides may be either synthetic or naturally-occurring. Also, DNA used as a probe for hybridization is usually labeled. Those skilled in the art readily understand such labeling methods. Herein, the term "oligonucleotide" means a polynucleotide with a relatively low degree of polymerization. Oligonucleotides are included in polynucleotides.

Tests for a disorder and/or disease using hybridization techniques can be performed using, for example, Northern hybridization, dot blot hybridization, or the DNA microarray technique. Furthermore, gene amplification techniques, such as the RT-PCR method may be used. By using the PCR amplification monitoring method during the gene amplification step in RT-PCR, one can achieve a more quantitative analysis of the expression of a marker gene.

In the PCR gene amplification monitoring method, the detection target (DNA or reverse transcript of RNA) is hybridized to probes that are labeled with a fluorescent dye and a quencher which absorbs the fluorescence. When the PCR proceeds and Taq polymerase degrades the probe with its 5'-3' exonuclease activity, the fluorescent dye and the quencher draw away from each other and the fluorescence is detected. The fluorescence is detected in real time. By simultaneously measuring a standard sample in which the copy number of a target is known, it is possible to determine the copy number of the target in the subject sample with the cycle number where PCR amplification is linear. Also, one skilled in the art recognizes that the PCR amplification monitoring method can be carried out using any suitable method.

The method of testing for a colon cancer-related disease can be also carried out by detecting a protein encoded by a marker gene. Hereinafter, a protein encoded by a marker gene is described as a "marker protein." For such test methods, for example, the Western blotting method, the immunoprecipitation method, and the ELISA method may be employed using an antibody that binds to each marker protein.

Antibodies used in the detection that bind to the marker protein may be produced by any suitable technique. Also, in order to detect a marker protein, such an antibody may be appropriately labeled. Alternatively, instead of labeling the antibody, a substance that specifically binds to the antibody, for example, protein A or protein G, may be labeled to detect the marker protein indirectly. More specifically, such a detection method can include the ELISA method.

A protein or a partial peptide thereof used as an antigen may be obtained, for example, by inserting a marker gene or a portion thereof into an expression vector, introducing the construct into an appropriate host cell to produce a transformant, culturing the transformant to express the recombinant protein, and purifying the expressed recombinant protein from the culture or the culture supernatant. Alternatively, the amino acid sequence encoded by a gene or an oligopeptide comprising a portion of the amino acid sequence encoded by a full-length cDNA are chemically synthesized to be used as an immunogen.

Furthermore, a test for a colon cancer-related disease can be performed using as an index not only the expression level of a marker gene but also the activity of a marker protein in a biological sample. Activity of a marker protein means the biological activity intrinsic to the protein. Various methods can be used for measuring the activity of each protein.

Even if a subject is not diagnosed as being affected with a disorder and/or disease in a routine test in spite of symptoms suggesting these diseases, whether or not such a subject is suffering from a disorder and/or disease can be easily determined by performing a test according to the methods described herein.

More specifically, in the present disclosure, when the marker gene is one of the genes described herein, an increase or decrease in the expression level of the marker gene in a subject whose symptoms suggest at least a susceptibility to a disorder and/or disease indicates that the symptoms are primarily caused thereby.

In addition, the tests are useful to determine whether a disorder and/or disease is improving in a subject. In other words, the methods described herein can be used to judge the therapeutic effect of a treatment therefor. Furthermore, when the marker gene is one of the genes described herein, an increase or decrease in the expression level of the marker gene in a subject, who has been diagnosed as being affected thereby, implies that the disease has progressed more.

The severity and/or susceptibility to a disorder and/or disease may also be determined based on the difference in expression levels. For example, when the marker gene is one of the genes described herein, the degree of increase in the expression level of the marker gene is correlated with the presence and/or severity of a disorder and/or disease.

### Animal Models

Animal models for a disorder and/or disease where the expression level of one or more marker genes or a gene functionally equivalent to the marker gene has been elevated in the animal model can also be made. A "functionally equivalent gene" as used herein generally is a gene that encodes a protein having an activity similar to a known activity of a protein encoded by the marker gene. A representative example of a functionally equivalent gene includes a counterpart of a marker gene of a subject animal, which is intrinsic to the animal.

The animal model is useful for detecting physiological changes due to a disorder and/or disease. In the present disclosure, the animal model is useful to reveal additional functions of marker genes and to evaluate drugs whose targets are the marker genes.

An animal model can be created by controlling the expression level of a counterpart gene or administering a counterpart gene. The method can include creating an animal model by controlling the expression level of a gene selected from the group of genes described herein. In the present disclosure, the method can include creating an animal model by administering the protein encoded by a gene described herein, or administering an antibody against the protein. It is to be also understood, that in the present disclosure, the marker can be over-expressed such that the marker can then be measured using appropriate methods. In the present disclosure, an animal model can be created by introducing a gene selected from such groups of genes, or by administering a protein encoded by such a gene. In the present disclosure, a disorder and/or disease can be induced by suppressing the expression of a gene selected from such groups of genes or the activity of a protein encoded by such a gene. An antisense nucleic acid, a ribozyme, or an RNAi can be used to suppress the expression. The activity of a protein can be controlled effectively by administering a substance that inhibits the activity, such as an antibody.

The animal model is useful to elucidate the mechanism underlying a disorder and/or disease and also to test the safety of compounds obtained by screening. For example, when an animal model develops the symptoms of a particular disorder and/or disease, or when a measured value involved in a certain a disorder and/or disease alters in the animal, a screening system can be constructed to explore compounds having activity to alleviate the disease.

As used herein, the expression "an increase in the expression level" refers to any one of the following: where a marker gene introduced as a foreign gene is expressed artificially; where the transcription of a marker gene intrinsic to the subject animal and the translation thereof into the protein are enhanced; or where the hydrolysis of the protein, which is the translation product, is suppressed.

As used herein, the expression "a decrease in the expression level" refers to either the state in which the transcription of a marker gene of the subject animal and the translation thereof into the protein are inhibited, or the state in which the hydrolysis of the protein, which is the translation product, is enhanced. The expression level of a gene can be determined, for example, by a difference in signal intensity on a DNA chip. Furthermore, the activity of the translation product--the protein--can be determined by comparing with that in the normal state.

It is also within the contemplated scope that the animal model can include transgenic animals, including, for example animals where a marker gene has been introduced and expressed artificially; marker gene knockout animals; and knock-in animals in which another gene has been substituted for a marker gene. A transgenic animal, into which an antisense nucleic acid of a marker gene, a ribozyme, a polynucleotide having an RNAi effect, or a DNA functioning as a decoy nucleic acid or such has been introduced, can be used as the transgenic animal. Such transgenic animals also include, for example, animals in which the activity of a marker protein has been enhanced or suppressed by introducing a mutation(s) into the coding region of the gene, or the amino acid sequence has been modified to become resistant or susceptible to hydrolysis. Mutations in an amino acid sequence include substitutions, deletions, insertions, and additions.

### Examples of Expression

In addition, the expression itself of a marker gene can be controlled by introducing a mutation(s) into the transcriptional regulatory region of the gene. Those skilled in the art understand such amino acid substitutions. Also, the number of amino acids that are mutated is not particularly restricted, as long as the activity is maintained. Normally, it is within 50 amino acids, within 30 amino acids, within 10 amino acids, or within 3 amino acids. The site of mutation may be any site, as long as the activity is maintained.

The present disclosure relates to screening methods for candidate compounds for therapeutic agents to treat a particular disorder and/or disease. One or more marker genes are selected from the group of genes described herein. A therapeutic agent for a colon cancer-related disease can be obtained by selecting a compound capable of increasing or decreasing the expression level of the marker gene(s).

It is to be understood that the expression "a compound that increases the expression level of a gene" refers to a compound that promotes any one of the steps of gene transcription, gene translation, or expression of a protein activity. On the other hand, the expression "a compound that decreases the expression level of a gene", as used herein, refers to a compound that inhibits any one of these steps.

In the present disclosure, the method of screening for a therapeutic agent for a disorder and/or disease can be carried out either in vivo or in vitro. This screening method can be performed, for example, by:
1) administering a candidate compound to an animal subject;
2) measuring the expression level of a marker gene(s) in a biological sample from the animal subject; or
3) selecting a compound that increases or decreases the expression level of a marker gene(s) as compared to that in a control with which the candidate compound has not been contacted.

The present disclosure relates to a method to assess the efficacy of a candidate compound for a pharmaceutical agent on the expression level of a marker gene(s) by contacting an animal subject with the candidate compound and monitoring the effect of the compound on the expression level of the marker gene(s) in a biological sample derived from the animal subject. The variation in the expression level of the marker gene(s) in a biological sample derived from the animal subject can be monitored using the same technique as used in the testing method described above. Furthermore, based on the evaluation, a candidate compound for a pharmaceutical agent can be selected by screening.

### Certain Nucleobase Sequences

Nucleobase sequences of mature miRNAs and their corresponding stem-loop sequences described herein are the sequences found in miRBase, an online searchable database of miRNA sequences and annotation, found at http://microrna.sanger.ac.uk/. Entries in the miRBase Sequence database represent a predicted hairpin portion of a miRNA transcript (the stem-loop), with information on the location and sequence of the mature miRNA sequence. The miRNA stem-loop sequences in the database are not strictly precursor miRNAs (pre-miRNAs), and may in some instances include the pre-miRNA and some flanking sequence from the presumed primary transcript. The miRNA nucleobase sequences described herein encompass any version of the miRNA, including the sequences described in Release 10.0 of the miRBase sequence database and sequences described in any earlier Release of the miRBase sequence database. A sequence database release may result in the renaming of certain miRNAs. A sequence database release may result in a variation of a mature miRNA sequence. The compounds that may encompass such modified oligonucleotides may be complementary any nucleobase sequence version of the miRNAs described herein.

It is understood that any nucleobase sequence set forth herein is independent of any modification to a sugar moiety, an internucleoside linkage, or a nucleobase. It is further understood that a nucleobase sequence comprising U's also encompasses the same nucleobase sequence wherein 'U' is replaced by 'T' at one or more positions having 'U." Conversely, it is understood that a nucleobase sequence comprising T's also encompasses the same nucleobase sequence wherein 'T; is replaced by 'U' at one or more positions having 'T."

In certain embodiments, a modified oligonucleotide has a nucleobase sequence that is complementary to a miRNA or a precursor thereof, meaning that the nucleobase sequence of a modified oligonucleotide is a least 60%, 65%, 70%, 75%, 80%, 85%, 90%, 95%, 97%, 98% or 99% identical to the complement of a miRNA or precursor thereof over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleobases, or that the two sequences hybridize under stringent hybridization conditions. Accordingly, in certain embodiments the nucleobase sequence of a modified oligonucleotide may have one or more mismatched basepairs with respect to its target miRNA or target miRNA precursor sequence, and is capable of hybridizing to its target sequence. In certain embodiments, a modified oligonucleotide has a nucleobase sequence that is 100% complementary to a miRNA or a precursor thereof. In certain embodiments, the nucleobase sequence of a modified oligonucleotide has full-length complementary to a miRNA.

### miRNA (miR) Therapies

The present disclosure relates to microRNAs that inhibit the expression of one or more genes in a subject. MicroRNA expression profiles can serve as a new class of cancer biomarkers.

Disclosed herein are methods of inhibiting gene expression and/or activity using one or more MiRs. In the present disclosure, the miR(s) inhibit the expression of a protein. In the present disclosure, the miRNA(s) inhibits gene activity (e.g., cell invasion activity).

The miRNA can be isolated from cells or tissues, recombinantly produced, or synthesized in vitro by a variety of techniques well known to one of ordinary skill in the art. In one embodiment, miRNA is isolated from cells or tissues. Techniques for isolating miRNA from cells or tissues are well known to one of ordinary skill in the art. For example, miRNA can be isolated from total RNA using the mirVana miRNA isolation kit from Ambion, Inc. Another techniques utilizes the flashIPAGE™ Fractionator System (Ambion, Inc.) for PAGE purification of small nucleic acids.

For the use of miRNA therapeutics, it is understood by one of ordinary skill in the art that nucleic acids administered in vivo are taken up and distributed to cells and tissues.

The nucleic acid may be delivered in a suitable manner which enables tissue-specific uptake of the agent and/or nucleic acid delivery system. The formulations described herein can supplement treatment conditions by any known conventional therapy, including, but not limited to, antibody administration, vaccine administration, administration of cytotoxic agents, natural amino acid polypeptides, nucleic acids, nucleotide analogues, and biologic response modifiers. Two or more combined compounds may be used together or sequentially.

The present disclosure relates to pharmaceutical compositions containing (a) one or more nucleic acid or small molecule compounds and (b) one or more other chemotherapeutic agents.

### Additional Useful Definitions

"Subject" means a human or non-human animal selected for treatment or therapy. Subject suspected of having" means a subject exhibiting one or more clinical indicators of a disorder, disease or condition.

Preventing" or "prevention" refers to delaying or forestalling the onset, development or progression of a condition or disease for a period of time, including weeks, months, or years. Treatment" or "treat" means the application of one or more specific procedures used for the cure or amelioration of a disorder and/or disease. In the present disclosure, the specific procedure is the administration of one or more pharmaceutical agents.

"Amelioration" means a lessening of severity of at least one indicator of a condition or disease. In the present disclosure, amelioration includes a delay or slowing in the progression of one or more indicators of a condition or disease. The severity of indicators may be determined by subjective or objective measures which are known to those skilled in the art.

Subject in need thereof" means a subject identified as in need of a therapy or treatment.

"Administering" means providing a pharmaceutical agent or composition to a subject, and includes, but is not limited to, administering by a medical professional and self-administering.

"Parenteral administration," means administration through injection or infusion. Parenteral administration includes, but is not limited to, subcutaneous administration, intravenous administration, intramuscular administration, intraarterial administration, and intracranial administration. Subcutaneous administration" means administration just below the skin.

"Improves function" means the changes function toward normal parameters. In the present disclosure, function is assessed by measuring molecules found in a subject's bodily fluids. Pharmaceutical composition" means a mixture of substances suitable for administering to an individual that includes a pharmaceutical agent. For example, a pharmaceutical composition may comprise a modified oligonucleotide and a sterile aqueous solution.

"Target nucleic acid," "target RNA," "target RNA transcript" and "nucleic acid target" all mean a nucleic acid capable of being targeted by antisense compounds. Targeting" means the process of design and selection of nucleobase sequence that will hybridize to a target nucleic acid and induce a desired effect. "Targeted to" means having a nucleobase sequence that will allow hybridization to a target nucleic acid to induce a desired effect. In the present disclosure, a desired effect is reduction of a target nucleic acid.

"Modulation" means to a perturbation of function or activity. In the present disclosure, modulation means an increase in gene expression. In the present disclosure, modulation means a decrease in gene expression.

"Expression" means any functions and steps by which a gene's coded information is converted into structures present and operating in a cell.

"Region" means a portion of linked nucleosides within a nucleic acid. In certain embodiments, a modified oligonucleotide has a nucleobase sequence that is complementary to a region of a target nucleic acid. For example, in certain such embodiments a modified oligonucleotide is complementary to a region of a miRNA stem-loop sequence. In certain such embodiments, a modified oligonucleotide is 100% identical to a region of a miRNA sequence.

"Segment" means a smaller or sub-portion of a region.

"Nucleobase sequence" means the order of contiguous nucleobases, in a 5' to 3' orientation, independent of any sugar, linkage, and/or nucleobase modification.

Contiguous nucleobases" means nucleobases immediately adjacent to each other in a nucleic acid.

"Nucleobase complementarity" means the ability of two nucleobases to pair non-covalently via hydrogen bonding. "Complementary" means a first nucleobase sequence is at least 60%, 65%, 70%, 75%, 80%, 85%, 90%, 95%, 97%, 98% or 99% identical, or is 100% identical, to the complement of a second nucleobase sequence over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleobases, or that the two sequences hybridize under stringent hybridization conditions. In the present disclosure a modified oligonucleotide that has a nucleobase sequence which is 100% complementary to a miRNA, or precursor thereof, may not be 100% complementary to the miRNA, or precursor thereof, over the entire length of the modified oligonucleotide.

"Complementarity" means the nucleobase pairing ability between a first nucleic acid and a second nucleic acid. "Full-length complementarity" means each nucleobase of a first nucleic acid is capable of pairing with each nucleobase at a corresponding position in a second nucleic acid. For example, in the present disclosure, a modified oligonucleotide wherein each nucleobase has complementarity to a nucleobase in an miRNA has full-length complementarity to the miRNA.

"Percent complementary" means the number of complementary nucleobases in a nucleic acid divided by the length of the nucleic acid. In the present disclosure, percent complementarity of a modified oligonucleotide means the number of nucleobases that are complementary to the target nucleic acid, divided by the number of nucleobases of the modified oligonucleotide. In the present disclosure, percent complementarity of a modified oligonucleotide means the number of nucleobases that are complementary to a miRNA, divided by the number of nucleobases of the modified oligonucleotide.

"Percent region bound" means the percent of a region complementary to an oligonucleotide region. Percent region bound is calculated by dividing the number of nucleobases of the target region that are complementary to the oligonucleotide by the length of the target region. In the present disclosure, percent region bound is at least 80%, at least 85%, at least 90%, at least 95%, at least 96%, at least 97%, at least 98%, at least 99%, or 100%.

"Percent identity" means the number of nucleobases in first nucleic acid that are identical to nucleobases at corresponding positions in a second nucleic acid, divided by the total number of nucleobases in the first nucleic acid.

"Substantially identical" used herein may mean that a first and second nucleobase sequence are at least 60%, 65%, 70%, 75%, 80%, 85%, 90%, 95%, 97%, 98% or 99% identical, or 100% identical, over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleobases.

"Hybridize" means the annealing of complementary nucleic acids that occurs through nucleobase complementarity.

"Mismatch" means a nucleobase of a first nucleic acid that is not capable of pairing with a nucleobase at a corresponding position of a second nucleic acid.

"Non-complementary nucleobase" means two nucleobases that are not capable of pairing through hydrogen bonding.

"Identical" means having the same nucleobase sequence.

"miRNA" or "miR" means a non-coding RNA between 18 and 25 nucleobases in length which hybridizes to and regulates the expression of a coding RNA. In certain embodiments, a miRNA is the product of cleavage of a pre-miRNA by the enzyme Dicer. Examples of miRNAs are found in the miRNA database known as miRBase (http://microrna.sanger.ac.uk/).

"Pre-miRNA" or "pre-miR" means a non-coding RNA having a hairpin structure, which contains a miRNA. In the present disclosure, a pre-miRNA is the product of cleavage of a pri-miR by the double-stranded RNA-specific ribonuclease known as Drosha.

"Stem-loop sequence" means an RNA having a hairpin structure and containing a mature miRNA sequence. Pre-miRNA sequences and stem-loop sequences may overlap. Examples of stem-loop sequences are found in the miRNA database known as miRBase (http://microrna.sanger.ac.uk/.

"miRNA precursor" means a transcript that originates from a genomic DNA and that comprises a non-coding, structured RNA comprising one or more miRNA sequences. For example, in the present disclosure a miRNA precursor is a pre-miRNA. In present disclosure, a miRNA precursor is a pri-miRNA.

"Antisense compound" means a compound having a nucleobase sequence that will allow hybridization to a target nucleic acid. In the present disclosure, an antisense compound is an oligonucleotide having a nucleobase sequence complementary to a target nucleic acid.

"Oligonucleotide" means a polymer of linked nucleosides, each of which can be modified or unmodified, independent from one another. "Naturally occurring internucleoside linkage" means a 3' to 5' phosphodiester linkage between nucleosides. "Natural nucleobase" means a nucleobase that is unmodified relative to its naturally occurring form. "miR antagonist" means an agent designed to interfere with or inhibit the activity of a miRNA. In the present disclosure, a miR antagonist comprises an antisense compound targeted to a miRNA. In the present disclosure, a miR antagonist comprises a modified oligonucleotide having a nucleobase sequence that is complementary to the nucleobase sequence of a miRNA, or a precursor thereof. In the present disclosure, an miR antagonist comprises a small molecule, or the like that interferes with or inhibits the activity of an miRNA.

The methods and reagents described herein are representative of preferred embodiments, are exemplary, and are not intended as limitations on the scope of the invention. Modifications therein and other uses will occur to those skilled in the art. These modifications are encompassed within the invention and are defined by the scope of the claims. It will also be readily apparent to a person skilled in the art that varying substitutions and modifications may be made to the invention disclosed herein without departing from the scope of the invention.

### REFERENCES

The publication and other material used herein to illuminate the invention or provide additional details respecting the practice of the invention, are for convenience provided in the following bibliography.

Citation of the any of the documents recited herein is not intended as an admission that any of the foregoing is pertinent prior art. All statements as to the date or representation as to the contents of these documents is based on the information available to the applicant and does not constitute any admission as to the correctness of the dates or contents of these documents.

Arata, Y., Fujita, M., Ohtani, K., Kijima, S., and Kato, J. Y. (2000). Cdk2-dependent and - independent pathways in E2F-mediated S phase induction. J Biol Chem 275, 6337-6345. Attwooll, C., Lazzerini Denchi, E., and Helin, K. (2004). The E2F family: specific functions and overlapping interests. EMBO J 23, 4709-4716.
Blow, J. J., and Hodgson, B. (2002). Replication licensing--defining the proliferative state? Trends Cell Biol 12, 72-78.
Bonci, D., Cittadini, A., Latronico, M. V. G., Borello, U., Aycock, J. K., Drusco, A., Innocenzi, A., Follenzi, A., Lavitrano, M., Monti, M. G., et al. (2003). 'Advanced' generation lentiviruses as efficient vectors for cardiomyocyte gene transduction in vitro and in vivo. Gene Therapy 10, 630636.
Costinean, S., Zanesi, N., Pekarsky, Y., Tili, E., Volinia, S., Heerema, N., and Croce, C. M. (2006). Pre-B cell proliferation and lymphoblastic leukemia/high-grade lymphoma in E(mu)miR155 transgenic mice. Proc Natl Acad Sci U S A 103, 7024-7029.
De Caestecker, M. P., Piek, E., and Roberts, A. B. (2000). Role of transforming growth factor-beta signaling in cancer. J Natl Cancer Inst 92, 1388-1402.
DeGregori, J. (2002). The genetics of the E2F family of transcription factors: shared functions and unique roles. Biochim Biophys Acta 1602, 131-150.
Dews, M., Homayouni, A., Yu, D., Murphy, D., Sevignani, C., Wentzel, E., Furth, E. E., Lee, W. M., Enders, G. H., Mendell, J. T., and Thomas-Tikhonenko, A. (2006). Augmentation of tumor angiogenesis by a Myc-activated microRNA cluster. Nat Genet 38, 1060-1065.
Dyson, N. (1998). The regulation of E2F by pRB-family proteins. Genes Dev 12, 2245-2262.
Egle, A., Harris, A. W., Bouillet, P., and Cory, S. (2004). Bim is a suppressor of Myc-induced mouse B cell leukemia. Proc Natl Acad Sci 101, 6164-6169.
Esquela-Kerscher A, Slack FJ. (2006) Oncomirs - microRNAs with a role in cancer. Nat Rev Cancer. 6, 259-69
Fox, J. G., Beck, P., Dangler, C. A., Whary, M. T., Wang, T. C., Shi, H. N., and Nagler-Anderson, C. (2000). Concurrent enteric helminth infection modulates inflammation and gastric immune responses and reduces helicobacter-induced gastric atrophy. Nat Med 6, 536-542. Gross, A., McDonnell, J. M., and Korsmeyer, S. J. (1999). BCL-2 family members and the mitochondria in apoptosis. Genes Dev 13, 1899-1911.
He, L., Thomson, J. M., Hemann, M. T., Hernando-Monge, E., Mu, D., Goodson, S., Powers, S., Cordon-Cardo, C., Lowe, S. W., Hannon, G. J., and Hammond, S. M. (2005). A microRNA polycistron as a potential human oncogene. Nature 435, 828-833.
Hossain, A., Kuo, M. T., and Saunders, G. F. (2006). Mir-17-5p regulates breast cancer cell proliferation by inhibiting translation of AIB1 mRNA. Mol Cell Biol 26, 8191-8201.
Houbaviy, H. B., Murray, M. F., and Sharp, P. A. (2003). Embryonic stem cell-specific MicroRNAs. Dev Cell 5, 351-358.
Houghton, J., Fox, J. G., and Wang, T. C. (2002). Gastric cancer: laboratory bench to clinic. J Gastroenterol Hepatol 17, 495-502.
Hwang, H. W., Wentzel, E. A., and Mendell, J. T. (2007). A hexanucleotide element directs microRNA nuclear import. Science 315, 97-100.
Johnson, D. G., and DeGregori, J. (2006). Putting the Oncogenic and Tumor Suppressive Activities of E2F into Context. Curr Mol Med 6, 731-738.
Ju, H. R., Jung, U., Sonn, C. H., Yoon, S. R., Jeon, J. H., Yang, Y., Lee, K. N., and Choi, I. (2003). Aberrant signaling of TGF-beta1 by the mutant Smad4 in gastric cancer cells. Cancer Lett 196, 197-206.
Kim, Y.K. and Kim, V.K. (2007). Processing of intronic microRNAs. EMBO J 26, 775-783.
Kloosterman, W. P., and Plasterk, R. H. (2006). The diverse functions of microRNAs in animal development and disease. Dev Cell 11, 441-450.
Lauren P (1965) The two histological main types of gastric carcinoma: Diffuse and so-called intestinal-type carcinoma. An attempt at a histo-clnical classification. Acta Pathol Microbiol Scand 64: 31-49.
Lazzerini Denchi, E., and Helin, K. (2005). E2F1 is crucial for E2F-dependent apoptosis. EMBO Rep 6, 661-668.
Leone, G., DeGregori, J., Yan, Z., Jakoi, L., Ishida, S., Williams, R. S., and Nevins, J. R. (1998). E2F3 activity is regulated during the cell cycle and is required for the induction of S phase. Genes Dev 12, 2120-2130.
Lewis, B. P., Burge, C. B., and Bartel, D. P. (2005). Conserved Seed Pairing, Often Flanked by Adenosines, Indicates that Thousands of Human Genes are MicroRNA Targets. Cell 120 15-20. Li, S. S., Xue, W. C., Khoo, U. S., Ngan, H. Y., Chan, K. Y., Tam, I. Y., Chiu, P. M., Ip, P. P., Tam, K. F., and Cheung, A. N. (2005). Replicative MCM7 protein as a proliferation marker in endometrial carcinoma: a tissue microarray and clinicopathological analysis. Histopathology 46, 307-313.
Liu, C.-G., Calin, G. A., Meloon, B., Gamliel, N., Sevignani, C., Ferracin, M., Dumitru, C. D., Shimizu, M., Zupo, S., Dono, M., et al. (2004). An oligonucleotide microchip for genome-wide microRNA profiling in human and mouse tissues. Proc Natl Acad Sci 101, 9740-9744. Lu, J., Getz, G., Miska, E. A., Alvarez-Saavedra, E., Lamb, J., Peck, D., Sweet-Cordero, A., Ebert, B. L., Mak, R. H., Ferrando, A. A., et al. (2005). MicroRNA expression profiles classify human cancers. Nature 435, 834-838.
Ma L., Teruya-Feldstein J. and Weinberg R.A. (2007) Tumour invasion and metastasis initiated by microRNA-10b in breast cancer. Nature. 449, 682-8.
Mattioli, E., Vogiatzi, P., Sun, A., Abbadessa, G., Angeloni, G., D'Ugo, D., Trani, D., Gaughan, J. P., Vecchio, F. M., Cevenini, G., et al. (2007). Immunohistochemical analysis of pRb2/p130, VEGF, EZH2, p53, p16(INK4A), p27(KIP1), p21(WAF1), Ki-67 expression patterns in gastric cancer. J Cell Physiol 210, 183-191.
Meng, F., Henson, R., Lang, M., Wehbe, H., Maheshwari, S., Mendell, J. T., Jiang, J., Schmittgen, T. D., and Patel, T. (2006). Involvement of human micro-RNA in growth and response to chemotherapy in human cholangiocarcinoma cell lines. Gastroenterology 130, 21132129.
Nahle, Z., Polakoff, J., Davuluri, R. V., McCurrach, M. E., Jacobson, M. D., Narita, M., Zhang, M. Q., Lazebnik, Y., Bar-Sagi, D., and Lowe, S. W. (2002). Direct coupling of the cell cycle and cell death machinery by E2F. Nat Cell Biol 4, 859-864.
O'Donnell, K. A., Wentzel, E. A., Zeller, K. I., Dang, C. V., and Mendell, J. T. (2005). c-Myc-regulated microRNAs modulate E2F1 expression. Nature 435, 839-843.
Ohgushi, M., Kuroki, S., Fukamachi, H., O'Reilly, L. A., Kuida, K., Strasser, A., and Yonehara, S. (2005). Transforming growth factor beta-dependent sequential activation of Smad, Bim, and caspase-9 mediates physiological apoptosis in gastric epithelial cells. Mol Cell Biol 25, 1001710028.
Park, K., Kim, S. J., Bang, Y. J., Park, J. G., Kim, N. K., Roberts, A. B., and Sporn, M. B. (1994). Genetic changes in the transforming growth factor beta (TGF-beta) type II receptor gene in human gastric cancer cells: correlation with sensitivity to growth inhibition by TGF-beta. Proc Natl Acad Sci U S A 91, 8772-8776.
Peng, D. F., Sugihara, H., Mukaisho, K., Tsubosa, Y., and Hattori, T. (2003). Alterations of chromosomal copy number during progression of diffuse-type gastric carcinomas: metaphase- and array-based comparative genomic hybridization analyses of multiple samples from individual tumours. J Pathol 201, 439-450.
Pierce, A. M., Schneider-Broussard, R., Gimenez-Conti, I. B., Russell, J. L., Conti, C. J., and Johnson, D. G. (1999). E2F1 has both oncogenic and tumor-suppressive properties in a transgenic model. Mol Cell Biol 19, 6408-6414.
Ren, B., Yu, G., Tseng, G. C., Cieply, K., Gavel, T., Nelson, J., Michalopoulos, G., Yu, Y. P., and Luo, J. H. (2006). MCM7 amplification and overexpression are associated with prostate cancer progression. Oncogene 25, 1090-1098.
Rodriguez, A., Vigorito, E., Clare, S., Warren, M. V., Couttet, P., Soond, D. R., van Dongen, S., Grocock, R. J., Das, P. P., Miska, E. A., et al. (2007). Requirement of bic/microRNA-155 for normal immune function. Science 316, 608-611.
Scandura, J. M., Boccuni, P., Massague, J., and Nimer, S. D. (2004). Transforming growth factor beta-induced cell cycle arrest of human hematopoietic cells requires p57KIP2 up-regulation. Proc Natl Acad Sci U S A 101, 15231-15236.
Suzuki, S., Adachi, A., Hiraiwa, A., Ohashi, M., Ishibashi, M., and Kiyono, T. (1998). Cloning and characterization of human MCM7 promoter. Gene 216, 85-91.
Suzuki, T., Yasui, W., Yokozaki, H., Naka, K., Ishikawa, T., and Tahara, E. (1999). Expression of the E2F family in human gastrointestinal carcinomas. Int J Cancer 81, 535-538.
Sylvestre, Y., De Guire, V., Querido, E., Mukhopadhyay, U. K., Bourdeau, V., Major, F., Ferbeyre, G., and Chartrand, P. (2007). An E2F/miR-20a autoregulatory feedback loop. J Biol Chem 282, 2135-2143.
Takada, H., Imoto, I., Tsuda, H., Sonoda, I., Ichikura, T., Mochizuki, H., Okanoue, T., and Inazawa, J. (2005). Screening of DNA copy-number aberrations in gastric cancer cell lines by array-based comparative genomic hybridization. Cancer Sci 96, 100-110.
Tan, T. T., Degenhardt, K., Nelson, D. A., Beaudoin, B., Nieves-Neira, W., Bouillet, P., Villunger, A., Adams, J. M., and White, E. (2005). Key roles of BIM-driven apoptosis in epithelial tumors and rational chemotherapy. Cancer Cell 7, 227-238.
Thai, T. H., Calado, D. P., Casola, S., Ansel, K. M., Xiao, C., Xue, Y., Murphy, A., Frendewey, D., Valenzuela, D., Kutok, J. L., et al. (2007). Regulation of the germinal center response by microRNA-155. Science 316, 604-608.
Thomson, J. M., Newman, M., Parker, J. S., Morin-Kensicki, E. M., Wright, T., and Hammond, S. M. (2006). Extensive post-transcriptional regulation of microRNAs and its implications for cancer. Genes Dev 20, 2202-2207.
Uemura, N., Okamoto, S., Yamamoto, S., Matsumura, N., Yamaguchi, S., Yamakido, M., Taniyama, K., Sasaki, N., and Schlemper, R. J. (2001). Helicobacter pylori infection and the development of gastric cancer. N Engl J Med 345, 784-789.
van den Brink, G. R., and Offerhaus, G. J. (2007). The morphogenetic code and colon cancer development. Cancer Cell 11, 109-117.
Volinia, S., Calin, G. A., Liu, C.-G., Ambs, S., Cimmino, A., Petrocca, F., Visone, R., Iorio, M., Roldo, C., Ferracin, M., et al. (2006). A microRNA expression signature of human solid tumors defines cancer gene targets. Proc Natl Acad Sci 103, 2257-2261.
Voorhoeve, P. M., le Sage, C., Schrier, M., Gillis, A. J., Stoop, H., Nagel, R., Liu, Y. P., van Duijse, J., Drost, J., Griekspoor, A., et al. (2006). A genetic screen implicates miRNA-372 and miRNA-373 as oncogenes in testicular germ cell tumors. Cell 124, 1169-1181.
Weiss, M. M., Kuipers, E. J., Postma, C., Snijders, A. M., Pinkel, D., Meuwissen, S. G., Albertson, D., and Meijer, G. A. (2004). Genomic alterations in primary gastric adenocarcinomas correlate with clinicopathological characteristics and survival. Cell Oncol 26, 307-317.
Woods, K., Thomson, J. M., and Hammond, S. M. (2007). Direct regulation of an oncogenic micro-RNA cluster by E2F transcription factors. J Biol Chem 282, 2130-2134.
Yanaihara, N., Caplen, N., Bowman, E., Seike, M., Kumamoto, K., Yi, M., Stephens, R. M., Okamoto, A., Yokota, J., Tanaka, T., et al. (2006). Unique microRNA molecular profiles in lung cancer diagnosis and prognosis. Cancer Cell 9, 189-198.
Yano, T., Ito, K., Fukamachi, H., Chi, X. Z., Wee, H. J., Inoue, K., Ida, H., Bouillet, P., Strasser, A., Bae, S. C., and Ito, Y. (2006). The RUNX3 tumor suppressor upregulates Bim in gastric epithelial cells undergoing transforming growth factor beta-induced apoptosis. Mol Cell Biol 26, 4474-4488.

## Claims

1. A method of diagnosing whether a subject has gastric adenocarcinoma, comprising:
measuring the level of a biomarker in a test sample from the subject, wherein the biomarker is the miR-106b-25 cluster: miR-106b, miR-93 and miR-25 that are upregulated in the test sample, relative to the level of a corresponding biomarker in a control sample, being indicative of the subject having gastric adenocarcinoma.

2. Use of a biomarker of gastric adenocarcinoma, consisting of the miR-106b-25 cluster: miR-106b, miR-93 and miR-25, for diagnosing gastric adenocarcinoma.

## Patentansprüche

1. Verfahren zum Diagnostizieren, ob eine Person ein Adenokarzinom des Magens hat, wobei das Verfahren Folgendes umfasst:
Messen des Spiegels eines Biomarkers in einer Testprobe von der Person, wobei der Biomarker der miR-106b-25-Cluster ist: miR-106b, miR-93 und miR-25, die in der Testprobe hochreguliert werden, im Vergleich zu dem Spiegel eines entsprechenden Biomarkers in einer Kontrollprobe, was darauf hindeutet, dass die Person ein Adenokarzinom des Magens hat.

2. Verwendung eines Biomarkers eines Adenokarzinoms des Magens, der aus dem miR-106b-25-Cluster besteht: miR-106b, miR-93 und miR-25, zum Diagnostizieren eines Adenokarzinoms des Magens.

## Revendications

1. Procédé pour diagnostiquer si un sujet est atteint d'un adénocarcinome gastrique, consistant à :
mesurer le taux d'un biomarqueur dans un échantillon d'essai du sujet, dans lequel le biomarqueur est le groupe miR-106b-25 : miR-106b, miR-93 et miR-25 qui sont régulés positivement dans l'échantillon d'essai, par rapport au taux d'un biomarqueur correspondant dans un échantillon témoin, ce qui indique si le sujet est atteint d'un adénocarcinome gastrique.

2. Utilisation d'un biomarqueur d'adénocarcinome gastrique, constitué du groupe miR-106b-25 : miR-106b, miR-93 et miR-25 pour le diagnostic d'un adénocarcinome gastrique.
